# EUROPEAN PATENT SPECIFICATION

(11) **EP 0 758 243 B1**
(45) Date of publication and mention of the grant of the patent: **12.03.2003**
(21) Application number: 95918365.8
(22) Date of filing: 28.04.1995
(51) Int. Cl.: A61K 31/70, C07H 15/20, C07H 15/18, C07H 15/203

(54) **BINDING OF E-SELECTIN, P-SELECTIN OR L-SELECTIN TO SIALYL-LEWISx OR SIALYL-LEWISa**
BINDUNG VON E-SELECTIN, P-SELECTIN ODER L-SELECTIN AN SIALYL-LEWISx ODER AN SIALYL-LEWISa
FIXATION DE LA E-SELECTINE ET/OU DE LA P-SELECTINE SUR LA SIALYL-LEWIS-?x OU LA SIALYL-LEWIS-?a

(30) Priority: 29.04.1994 US 235293
(43) Date of publication of application: 19.02.1997
(73) Proprietor: TEXAS BIOTECHNOLOGY CORPORATION, Houston, TX 77030 (US)
(72) Inventor: KOGAN, Timothy P., Sugar Land, TX 77479 (US); DUPRE, Brian, Houston, TX 77030 (US); SCOTT, Ian L., Houston, TX 77035 (US); KELLER, Karin, Houston, TX 77067 (US); DAO, Huong, Houston, TX 77083 (US); BECK, Pamela J., Houston, TX 77081 (US)
(74) Representative: Modiano, Guido, Dr.-Ing.
(86) International application number: PCT/US95/05463
(87) International publication number: WO 95/029682

(56) References cited:
- WO-A-94/00477
- WO-A-95/29681
- US-A- 5 212 298
- US-A- 5 268 364
- US-A- 5 304 640
- ANGEW. CHEM. INT. ED. ENGL., vol. 32, no. 8, 1993, pages 1165-1167, XP002031717 JOACHIMI ET AL.: "Molecular self-organisation of amphotropic cyanobiphenyl Mesogens"
- J. MED. CHEM. (JMCMAR,00222623);95; VOL.38 (26); PP.4976-84, TEXAS BIOTECHNOLOGY CORPORATION;DEPARTMENTS OF MEDICINAL CHEMISTRY; HOUSTON; 77030; TX; USA (US), XP002031718 KOGAN T P ET AL: "Rational Design and Synthesis of Small Molecule, Non-oligosaccharide Selectin Inhibitors: (.alpha.-D-Mannopyranosyloxy)biphenyl-Subs tituted Carboxylic Acids"
- JOURNAL OF PHARMACEUTICAL SCIENCES, Volume 59, Number 4, issued April 1970, EL-DAKHAKHNY, "Some Coumarin Constituents of Prunus Mahaleb L. Fruit Kernels V", pages 551-553.
- JOURNAL OF NATURAL PRODUCTS, Volume 56, Number 11, issued November 1993, LUYENGI et al., "Linusitamarin, A New Phenylpropanoid Glucoside from Linum Usitatissimum", pages 2012-2015.

## Description

### Cross-Reference to Related Applications

The present application is a Continuation-In-Part Application of Application U.S Serial No. 08/235,293, filed April 29, 1994.

### Technical Field

This invention relates to compounds that inhibit the binding of E-selectin, P-selectin or L-selectin to sialyl-Lewis^{x} and sialyl-Lewis^{a} and to methods of inhibiting the binding of E-selectin, P-selectin or L-selectin to sialyl-Lewis^{x} or sialyl-Lewis^{a} using said compounds. This invention also relates to pharmaceutically active compositions comprising compounds that inhibit the binding of E, P or L-selectin to sialyl-Lewis^{x} or sialyl-Lewis^{a}.

### Background of the Invention

E-selectin, which has also been called ELAM-1 for endothelial leukocyte adhesion molecule-1 and LECAM-2 for lectin cell adhesion molecule, is a glycoprotein that is found on the surface of endothelial cells, the cells that line the interior wall of capillaries. E-selectin recognizes and binds to the carbohydrate sialyl-Lewis^{x} (sLe^{x}), which is present on the surface of certain white blood cells. E-selectin helps white blood cells recognize and adhere to the capillary wall in areas where the tissue surrounding the capillary has been infected or damaged. E-selectin is actually one of three selectins now known. The other two are L-selectin and P-selectin. P-selectin is expressed on inflamed endothelium and platelets, and has much structural similarity to E-selectin and can also recognize sialyl-Lewis^{X}. L-selectin is expressed on leukocytes and also has much structure similarity to P- and E-selectins. The structure of sialyl-Lewis^{X} and sialyl-Lewis^{a} (sLe^{a}) are shown in formulas Iₐ and I_{b} below:

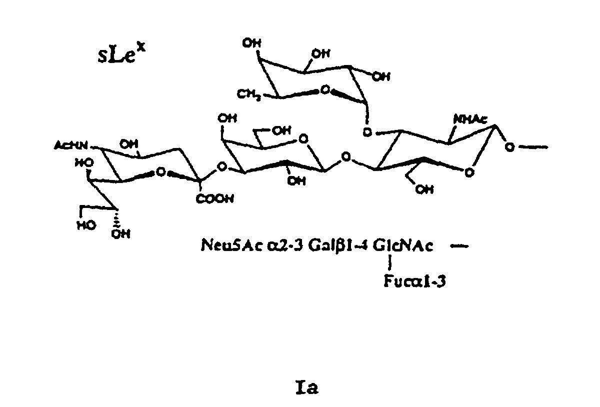

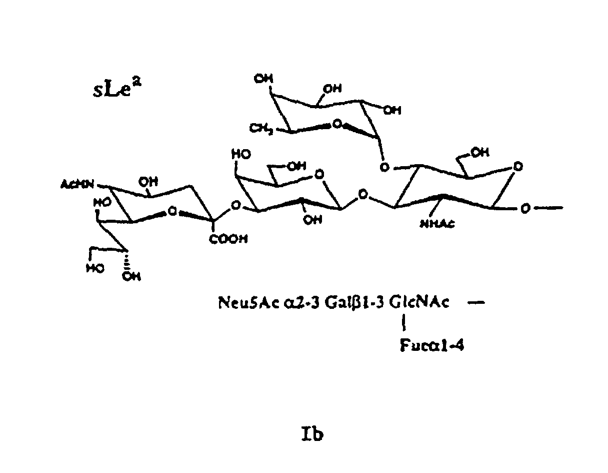

When a tissue has been invaded by a microorganism or has been damaged, white blood cells, also called leukocytes, play a major role in the inflammatory response. One of the most important aspects of the inflammatory response involves the cell adhesion event. Generally, white blood cells are found circulating through the bloodstream. However, when a tissue is infected or becomes damaged, the white blood cells must be able to recognize the invaded or damaged tissue and be able to bind to the wall of the capillary near the affected tissue and diffuse through the capillary into the affected tissue. E-selectin helps two particular types of white blood cells recognize the affected sites and bind to the capillary wall so that these white blood cells may diffuse into the affected. tissue.

There are three main types of white blood cells: granulocytes, monocytes and lymphocytes. Of these categories, E-selectin recognizes sLe^{x} presented as a glycoprotein or glycolipid on the surface of monocytes and neutrophils. Neutrophils are a subclass of granulocytes that phagocytose and destroy small organisms, especially bacteria. Monocytes, after leaving the bloodstream through the wall of a capillary, mature into macrophages that phagocytose and digest invading microorganisms, foreign bodies and senescent cells.

Monocytes and neutrophils are able to recognize the site where tissue has been damaged by binding to E-selectin, which is produced on the surface of the endothelial cells lining capillaries when the tissue surrounding a capillary has been infected or damaged. Typically, the production of E-selectins and P-selectins is increased when the tissue adjacent a capillary is affected. P-selectin is present constitutively in storage granules from which it can be rapidly mobilized to the cell surface after the endothelium has been activated. In contrast, E-selectin requires *de novo* RNA and protein synthesis, and peak expression does not occur until about 4-6 hours after activation, and declines to basal levels after about 24-48 hours. White blood cells recognize affected areas because sLe^{x} moieties present on the surface of the white blood cells bind to E-selectin and P-selectin. This binding slows the flow of white blood cells through the bloodstream, since it mediates the rolling of leukocytes along the activated endothelium prior to integrin mediated attachment and migration, and helps to localize white blood cells in areas of injury or infection.

While white blood cell migration to the site of injury helps fight infection and destroy foreign material, in many instances this migration can get out of control, with white blood cells flooding to the scene, causing widespread tissue damage. Compounds capable of blocking this process, therefore, may be beneficial as therapeutic agents. Thus, it would be useful to develop inhibitors that would prevent the binding of white blood cells to E-selectin or P-selectin. For example, some of the diseases that might be treated by the inhibition of selectin binding to sLe^{x} include, but are not limited to, ARDS, Crohn's disease, septic shock, traumatic shock, multi-organ failure, autoimmune diseases, asthma, inflammatory bowel disease, psoriasis, rheumatoid arthritis and reperfusion injury that occurs following heart attacks, strokes and organ transplants. In addition to being found on some white blood cells, sLe^{a}, a closely related regiochemical isomer of sLe^{x}, is found on various cancer cells, including lung and colon cancer cells. It has been suggested that cell adhesion involving sLe^{a} may be involved in the metastasis of certain cancers.

### Summary of the Invention

The present invention provides compounds having the structure of formula II below: 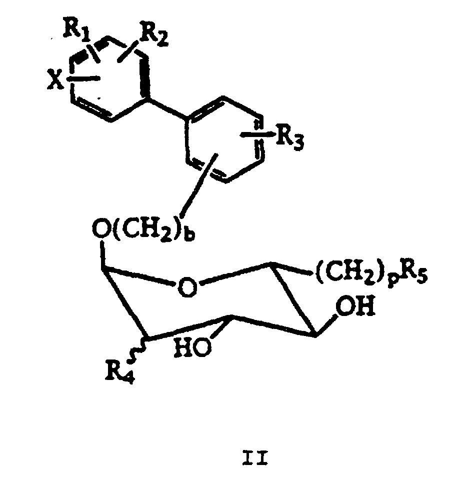 wherein X is selected from the group consisting -(CH₂)ₙCH₃ of -CN, -(CH₂)ₙCO₂H, -O(CH₂)ₘCO₂H, -(CH₂)ₙCOZ, -(CH₂)ₙZ, -CHCO₂H(CH₂)ₘCO₂H, -(CH₂)ₙO(CH₂)ₘCO₂H, -CONH(CH₂)ₘCO₂H, -CH(OZ)(CO₂H), -CH(Z)(CO₂H), -(CH₂)ₙSO₃H, -(CH₂)ₙPO₃D₁D₂, -NH(CH₂)ₘCO₂H, -CONH(CHR₆)CO₂H, (1-H-tetrazolyl-5-alkyl-), and -OH;

R₁ and R₂ are independently selected from the group consisting of hydrogen, alkyl, halogen, -OZ, -NO₂, -(CH₂)ₙCO₂H, -NH₂ and -NHZ;

R₃ is selected from the group consisting of hydrogen, halogen, alkyl, -OZ and -NHZ;

R₄ is selected from the group consisting of hydrogen, halogen, alkyl, hydroxyl, hydroxyl-O-sulfate and -OZ;

R₅ is selected from the group consisting of hydroxyl, -CN, -N₃, -NH₂, -NHNH₂, -NE₁E₂, -NHE₁, -NHCO(CH₂)ₙCO₂H, -S(CH₂)ₘCO₂H and -NHCHNHNH₂; and

R₆ is selected from the group consisting of hydrogen, alkyl, aralkyl, hydroxyalkyl, aminoalkyl, alkyl carboxylic acid and alkyl carboxamide;
wherein n is 0 to 6, m is 1 to 6, p is 0 to 6, b is 0 to 2, Z is alkyl, aryl or aralkyl, D₁ and D₂ are independently hydrogen or alkyl, E₁ is alkyl or -(CH₂)ₐCO₂H wherein a is 1 to 18, and E₂ is alkyl, and the pharmaceutically acceptable salts, esters, amides and prodrugs thereof.

More particularly, this invention provides compounds of the formula III: 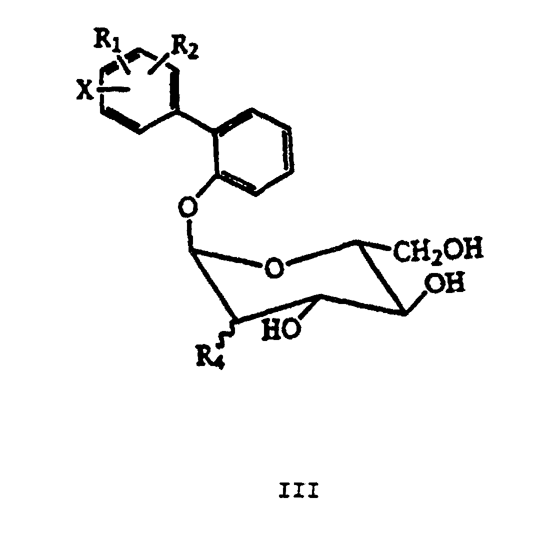 wherein X is -Q, -(CH₂)ₙQ, -O(CH₂)ₙQ, -(CH₂)ₙO(CH₂)ₘQ, -CONH(CH₂)ₙQ, -NH(CH₂)ₘQ, -O(CH₂)ₙO(CH₂)ₘQ, or -CONH(CHR₆)Q;

R₁ and R₂ are independently selected from the group consisting of hydrogen and -(CH₂)ₙQ;

R₄ is hydroxyl or hydrogen; R₆ is selected from the group consisting of hydrogen, alkyl, aralkyl, hydroxyalkyl, aminoalkyl, alkyl carboxylate, and alkyl carboxamide; Q is -CO₂H, n is 0 to 6, and m is 1 to 6, and the pharmaceutically acceptable salts, esters, amides and prodrugs thereof.

In a further embodiment, the present invention is directed to compounds of the formula IV: 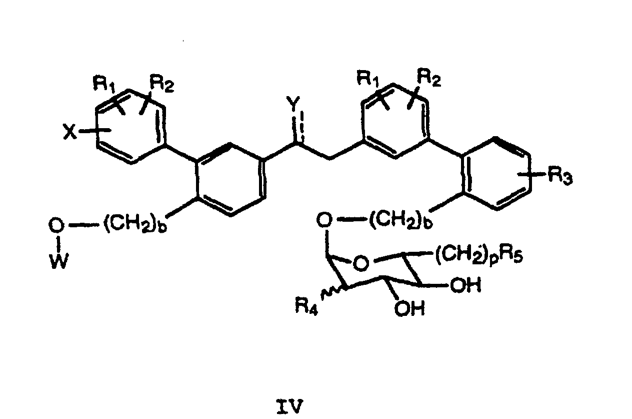 wherein W is hydrogen, alkyl or α-D-mannosyl and Y is selected from H/H, oxygen, H/hydroxyl, H/NH₂, H/NHE₁, H/NE₁E₂, NH, NE₁, oxime and O-alkyl oxime, and the pharmaceutically acceptable salts, esters, amides and prodrugs thereof.

The present invention also provides a method of inhibiting the binding of E-selectin or P-selectin to sLe^{x} or sLe^{a} comprising the step of administering to a patient an effective amount of a compound having the structure of formulae II, III or IV to inhibit the binding of E-, P- or L-selectin to sLe^{x} or sLe^{a}, and a pharmaceutically active composition comprising a compound of formula II, III or IV and a pharmaceutically acceptable carrier.

Also provided is a method for treating diseases such as ARDS, Crohn's disease, septic shock, traumatic shock, multi-organ failure, autoimmune diseases, asthma, inflammatory bowel disease, psoriasis, rheumatoid arthritis, reperfusion injury that occurs following and heart attacks, strokes and organ transplants, which comprises administering to an animal in need of such treatment a therapeutically effective amount of a compound having the formulae II, III or IV to reduce the symptoms of the disease.

### Detailed Description of the Preferred Embodiments

It has been found that compounds of formulae II, III and IV act to inhibit E-, P- or L-selectin binding to sLe^{x} or sLe^{a}.

The compounds of formula II comprise two major components: a mannopyranoside derived moiety (a carbohydrate) and a biphenyl moiety. With respect to the mannopyranoside moiety, D-mannopyranosides are preferred over L-mannopyranosides, and the absolute stereochemistry shown in formula II at the C₃ and C₄ positions of the mannose sugar is preferred.
However, epimeric stereochemistry is permitted at the C₂ position of the carbohydrate moiety (using glucopyranosides). The carbohydrate moiety will herein be called a mannopyranoside for the sake of simplicity. Also, the α-anomer is preferred over the β-anomer.

The mannopyranoside moiety is attached to the biphenyl moiety via an -O-(CH₂)_{b}- bridge where b is 0 to 2. Preferably, the mannopyranoside is attached to the ortho or meta positions on the phenyl ring.

In addition, the mannopyranoside moiety may be substituted. Particularly preferred are mannopyranosides having substituents at the C₂ and C₆ positions. For example, the mannose 6-position may be substituted with groups such as hydroxyl, -CN, -N₃, -NH₂, -NHNH₂, -NE₁E₂, -NHE₁, -NHCO(CH₂)ₙCO₂H, -S(CH₂)ₘCO₂H or -NHCHNHNH₂ wherein n is 0 to 6, m is 1 to 6, E₁ is alkyl or -(CH₂)₈CO₂H and E₂ is alkyl. Similarly, the C₂ position may be substituted with hydrogen, halogen, alkyl, hydroxyl,
hydroxyl-O-sulfate or alkoxy. It is preferred, however, that the alkyl group is a lower alkyl group. It is also recognized that the mannopyranoside moiety may have substituents at either the C₂ or C₆ positions or both.

The second component of the compounds of the present invention comprises a biphenyl moiety. The biphenyl moiety may be substituted at both phenyl groups or may be substituted at only one phenyl group. Moreover, each phenyl group may have more than one substituent.

The phenyl group that is not directly attached to the mannose moiety is substituted. Preferably, this phenyl group is substituted at the 3 or 4 position (meta or para) with at least one of the group consisting of -(CH₂)ₙCH₃, -CN, -(CH₂)ₙCO₂H, -O(CH₂)ₘCO₂H, -(CH₂)ₙO(CH₂)ₘCO₂H, -(CCH₂)ₙCOZ, -(CH₂)ₙZ, -CHCO₂H(CH₂)ₘCO₂H, -CONH(CH₂)ₘCO₂H, -CH(OZ)(CO₂H), -CH(Z)(CO₂H), -(CH₂)ₙSO₃H, -(CH₂)ₙPO₃D₁D₂, -NH(CH₂)ₘCO₂H, -CONH(CHR₆)CO₂H, (1-H-tetrazolyl-5-alkyl-) and -OH wherein n is 0 to 6, m is 1 to 6, Z is alkyl or aryl, D₁ and D₂ are independently hydrogen or alkyl, R₆ is selected from the group consisting of hydrogen, alkyl, aralkyl, hydroxyalkyl, aminoalkyl, alkyl carboxylic acid and alkyl carboxamide.

If the phenyl group not directly attached to the mannopyranoside moiety is substituted with more than one substituent, then one substituent is typically located at the 3 or 4 position and is preferably selected from the group consisting of -(CH₂)ₙCH₃, -CN, -(CH₂)ₙCO₂H, -O(CH₂)ₘCO₂H, -(CH₂)ₙO(CH₂)ₘCO₂H, -(CH₂)ₙCOZ, -(CCH₂)ₙZ, -CHCO₂H(CH₂)ₘCO₂H, -CONH(CH₂)ₘCO₂H, -CH(OZ) (CO₂H), -CH(Z) (CO₂H), - (CH₂)ₙSO₃H, -(CH₂)ₙPO₃D₁D₂, -NH(CH₂)ₘCO₂H, -CONH(CHR₆)CO₂H, and (1-H-tetrazolyl-5-alkyl-) wherein n is 0 to 6, m is 1 to 6, Z is alkyl or aryl, R₆ is selected from the group consisting of hydrogen, alkyl, aralkyl, hydroxyalkyl, aminoalkyl, alkyl carboxylic acid and alkyl carboxamide, and D₁ and D₂ are independently hydrogen or alkyl. Any other substituents are typically selected independently from the group consisting of hydrogen, halogen, alkyl, NO₂, CO₂H and OH.

It may also be desirable to have substitution on the phenyl ring directly attached to the mannopyranoside moiety. Substituents are preferably selected from the group consisting of hydrogen, halogen, alkyl, alkoxy, and alkylamino.

The most preferred compounds of the present invention have the formula III.

As used herein, the term "alkyl" shall mean a monovalent straight chain or branched chain group of 1 to 12 carbon atoms including, but not limited to, methyl, ethyl, n-propyl, isopropyl, n-butyl, sec-butyl, isobutyl, tert-butyl and the like.

The term "lower alkyl" shall mean any alkyl group having from one to six carbon atoms.

The term "halogen" shall mean any atom selected from the group consisting of chlorine, fluorine, bromine, and iodine.

The term "alkoxy" shall mean an alkyl group attached to a molecule through an oxygen atom including, but not limited to, methoxy, ethoxy, isopropoxy, n-butoxy, sec-butoxy, isobutoxy, tert-butoxy and the like.

The term "alkylamino" shall mean groups having the structure -NH-(alkyl), or -N-(alkyl)₂, including, for example, methylamino, ethylamino, isopropylamino and the like.

The term "aryl" shall mean unsubstituted or substituted carbocyclic aromatic groups including, but not limited to, phenyl, biphenyl, 1 or 2-naphthyl, fluorenyl, (1,2)-dihydronaphthyl, indenyl, indanyl, thienyl and the like. Substituents include other aryl groups or other substituents such as alkyl, alkoxy, alkyl carboxylic acid or mannose groups.

The term "aralkyl" (also called arylalkyl) shall mean a substituted or unsubstituted aryl group appended to an alkyl group including, but not limited to, benzyl, 1 and 2-naphthylmethyl, halobenzyl, alkoxybenyl, hydroxybenzyl, aminobenzyl, nitrobenzyl, guanidinobenzyl, fluorenylmethyl, phenylmethyl(benzyl), 1-phenylethyl, 2-phenylethyl, 1-naphthylethyl and the like.

The term "hydroxyalkyl" shall mean -OH appended to an alkyl group.

The term "aminoalkyl" shall mean a group having the structure -NRₓR_{y} appended to an alkyl group. The groups Rₓ and R_{y} are independently selected from hydrogen, alkyl and aryl.

The term "alkyl carboxylic acid shall mean a carboxyl group (-CO₂H) appended to an alkyl group.

The term "alkyl carboxamide" shall mean a group having the formula -CONRₓR_{y} appended to an alkyl group where Rₓ and R_{y} are as defined above under aminoalkyl.

The term "pharmaceutically acceptable salts, esters, amides and prodrugs" as used herein refers to those carboxylate salts, amino acid addition salts, esters, amides and prodrugs of the compounds of the present invention which are, within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of the compounds of the present invention. These salts can be prepared *in situ* during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, laurylsulphonate salts and the like. These may include cations based on the alkali and alkaline earth metals, such as sodium, lithium, potassium, calcium, magnesium and the like, as well as nontoxic ammonium, quaternary ammonium and amine Gations including, but not limited to, ammonium, tetramethylammonium, tetraethylammonium, methylamine, dimethylamine, trimethylamine, triethylamine, ethylamine, and the like. (See, for example S. M. Berge, et al., "Pharmaceutical Salts," **J. Pharm. Sci., 66:** 1-19 (1977), which is incorporated herein by reference.)

Examples of pharmaceutically acceptable, non-toxic esters of the compounds of this invention include C₁ to C₆ alkyl esters wherein the alkyl group is a straight or branched chain. Acceptable esters also include C₅ to C₇ cycloalkyl esters as well arylalkyl esters such as, but not limited to benzyl. C₁ to C₄ alkyl esters are preferred. Esters of the compounds of the present invention may be prepared according to conventional methods.

Examples of pharmaceutically acceptable, non-toxic amides of compounds of this invention include amides derived from ammonia, primary C₁ to C₆ alkyl amines and secondary C₁ to C₆ dialkyl amines wherein the alkyl groups are straight or branched chain. In the case of secondary amines the amine may also be in the from of a 5 or 6 membered heterocycle containing one nitrogen atom. Amides derived from ammonia, C₁ to C₃ alkyl primary amides and C₁ to C₂ dialkyl secondary amides are preferred. Amides of the compounds of the invention may be prepared according to conventional methods.

The term "prodrug" refers to compounds that are rapidly transformed *in vivo* to yield to the parent compound of the above formula, for example by hydrolysis in blood. A thorough discussion is provided in T. Higuchi and V. Stella, "Pro-drugs as Novel Delivery Systems", Vol 14 of the A.C.S. Symposium Series, and in *Bioreversible Carriers in Drug Design,* ed. Edward B. Roche, American Pharmaceutical Association and Pergamon Press, 1987, both of which are incorporated herein by reference.

The present invention also provides for pharmaceutically active compositions that contain the compounds of the present invention. It is also contemplated that pharmaceutically active compositions may contain a compound of the present invention and other compounds that inhibit or compete with E-, P- or L-selectin binding to sLe^{x} or sLe^{a}, including sLe^{x} and sLe^{a} themself.

Pharmaceutically active compositions of the present invention comprise a physiological carrier and a compound of formulae II, III or IV.

The pharmaceutical compositions of the present invention may include one or more of the compounds of formulae II, III or IV formulated together with one or more nontoxic, physiologically acceptable carriers, adjuvants or vehicles, which are collectively referred to herein as carriers, for parenteral injection, for oral administration in solid or liquid form, for rectal or topical administration and the like.

The compositions can be administered to humans and animals either orally, rectally, parentally (intravenously, intramuscularly, or subcutaneously), intracisternally, intravaginally, intraperitoneally, locally (powders, ointments or drops), or as buccal or nasal sprays.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyol, (propylene glycol, polyethylene glycol, glycerol and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispersing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

If desired, and for more effective distribution, the compounds can be incorporated into slow or timed release or targeted delivery systems such as polymer matrices, liposomes, and microspheres. They may be sterilized, for example, by filtration through a bacteria-retaining filter, or by incorporating sterilizing agents in the form of sterile water, or some other sterile injectable medium immediately before use.

Solid dosage forms for oral administration include capsules, tablets, pills, powders and granules. In such solid dosage forms, the active compound or a pro-drug ester is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alginates, gelatin, polyvinylpyrrolidone, sucrose and acacia, (c) humectants, as for example, glycerol,(d) diintegrating agents, as for example, agar-agar, calcium carbonate, potato or tapioca starch, alginic acid, certain complex silicates and sodium carbonate, (e) solution retarders, as for example, paraffin, (f) absorption accelerators, as for example, quaternary ammonium compounds, (g) wetting agents, as for example, cetyl alcohol and glycerol monostearate, (h) adsorbents, as for example, kaolin and bentonite, and (i) lubricants, as for example, talc, calcium stearate, magnesium stearate, solid polyethylene glycols, sodium lauryl sulfate or mixtures thereof. In the case of capsules, tablets and pills, the dosage forms may also comprise buffering agents.

Solid compositions of a similar type may also be employed as fillers in soft and hard-filled gelatin capsules using such excipients as lactose or milk sugar as well as high molecular weight polyethylene glycols and the like.

Solid dosage forms such as tablets, dragees, capsules, pills and granules can be prepared with coatings and shells, such as enteric coatings and others well known in the art. They may contain opacifying agents, and can also be of such composition that they release the active compound or compounds in a certain part of the intestinal tract in a delayed manner. Examples of embedding compositions that can be used are polymeric substances and waxes.

The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame seed oil, glycerol, tetrahydrofurfuryl alcohol, polyethylene glycols and fatty acid esters of sorbitan or mixtures of these substances, and the like.

Besides such inert diluents, the compositions can also include adjuvants, such as wetting agents, emulsifying and suspending agents, sweetening, flavoring and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth or mixtures of these substances and the like.

Compositions for rectal administrations are preferably suppositories, which can be prepared by mixing the compounds of the present invention with suitable nonirritating excipients or carriers such as cocoa butter, polyethylene glycol or a suppository wax, which are solid at ordinary temperatures but liquid at body temperature and therefore melt in the rectal or vaginal cavity and release the active component.

Dosage forms for topical administration of a compound of this invention include ointments, powders, sprays and inhalants.

The active component is admixed under sterile conditions with a physiologically acceptable carrier and any needed preservatives, buffers or propellants as may be required. Ophthalmic formulations, eye ointments, powders and solutions are also contemplated as being within the scope of this invention.

The compounds of this invention can also be administered in the form of liposomes. As is known in the art, liposomes are generally derived from phospholipids or other lipid substances. Liposomes are formed by mono or multilamellar hydrated liquid crystals that are dispersed in an aqueous medium. Any nontoxic, physiologically acceptable and metabolizable lipid capable of forming liposomes can be used. The present compositions in liposome form can contain, in addition to the selectin binding inhibitors of the present invention, stabilizers, preservatives, excipients and the like. The preferred lipids are the phospholipids and the phosphatidyl cholines (lecithins), both natural and synthetic. Methods to form liposomes are well known in the art.

Actual dosage levels of active ingredient in the compositions of the present invention may be varied so as to obtain an amount of active ingredient that is effective to obtain the desired therapeutic response for a particular composition and method of administration. The selected dosage levels, therefore, depends on the desired therapeutic effect, on the route of administration, on the desired duration of treatment and other factors.

The total daily dosage of the compounds of this invention administered to a host in single or divided doses may be in the range of from about 5 mg to about 250 mg per kilogram of body weight. Dosage unit compositions may contain such submultiples thereof as may be used to make up the daily dosage. It will be understood, however, that the specific dose level for any particular patient, whether human or other animal, will depend upon a variety of factors including the body weight, general health, sex, diet, time and route of administration, rates of absorption and excretion, combination with other drugs and the severity of the particular disease being treated.

In particular, the compounds of the present invention may be used to treat a variety of diseases relating to inflammation and cell-cell recognition and adhesion. For example, the compounds of the present invention may be administered to a patient to treat septic shock, chronic inflammatory diseases such as psoriasis and rheumatoid arthritis, and reperfusion tissue injury that occurs following heat attacks, strokes and organ transplants, traumatic shock, multi-organ failure, autoimmune diseases, asthma and inflammatory bowel disease. In each case, an effective amount of the compounds of the present invention is administered either alone or as part of a pharmaceutically active composition to a patient in need of such treatment. It is also recognized that a combination of the compounds may be administered to a patient in need of such administration. The compounds of the present invention may also be administered to treat other diseases that are associated with cell-cell adhesion. As the present compounds inhibit the binding of E-, P- or L-selectin with sLe^{x} or sLe^{a}, any disease that is related to this interaction may potentially be treated by the inhibition of this binding interaction.

In addition to being found on some white blood cells, sLe^{a} is found on various cancer cells, including lung and colon cancer cells. It has been suggested that cell adhesion involving sLe^{a} may be involved in the metastasis of certain cancers and that inhibitors of sLe^{a} binding might be useful in the treatment of some forms of cancer.

The compounds of the present invention may be synthesized according to the general synthetic scheme shown in scheme 1: 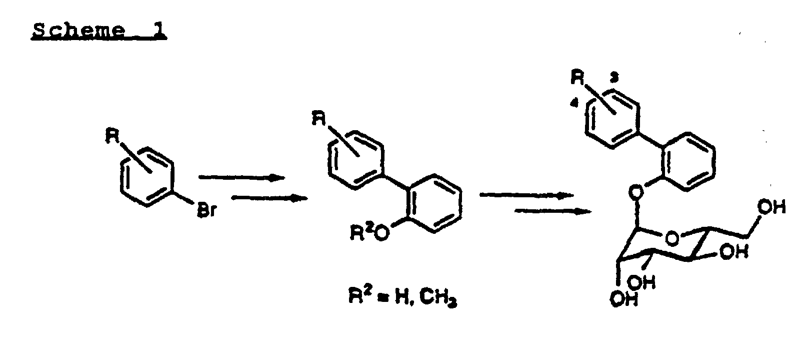 where R is selected from the group consisting of -(CH₂)ₙCO₂H, -O(CH₂)ₘCO₂H, -(CH₂)ₙO(CH₂) ₘCO₂H, -CONH(CH₂)ₘCO₂H, -CH(OZ)(CO₂H), -CH(Z)(CO₂H), -(CH₂)ₙSO₃H, -(CH₂)ₙPO₃D₁D₂, -NH(CH₂)ₘCO₂H, -CONH(CHR₆)CO₂H, and (1 H-tetrazolyl-5-alkyl-); wherein n is 0 to 6, m is 1 to 6, Z is alkyl, D₁ and D₂ are independently hydrogen or alkyl, and R₆ is selected from the group consisting of hydrogen, alkyl, aralkyl, hydroxyalkyl, aminoalkyl, alkyl carboxylic acid and alkyl carboxamide, and the pharmaceutically acceptable salts, esters, amides, and prodrugs thereof.

In this scheme, the desired biphenyl moiety is synthesized and subsequently reacted with the desired mannopyranoside moiety to form a compound of the present invention. Specific examples of the synthesis of the compounds of the invention are presented in the experimental section below wherein R is as defined above.

Other compounds may be synthesized according to the schemes set forth below where R is as defined above.

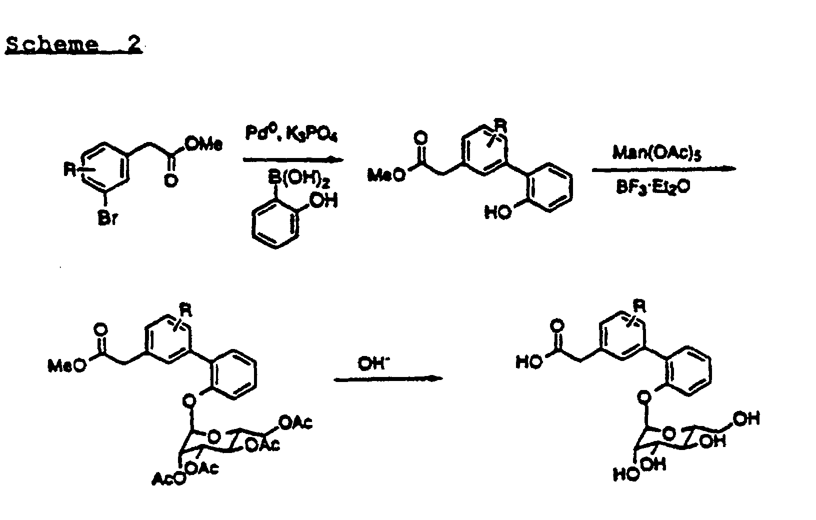

In this reaction scheme, a substituted phenylacetic ester is coupled with an aryl boronic acid in the presence of a palladium catalyst and base to give a biphenyl compound. The phenol functionality is reacted with a protected mannopyranoside in the presence of boron trifluoride etherate. The desired compound is obtained by treatment with base to hydrolyse the esters.

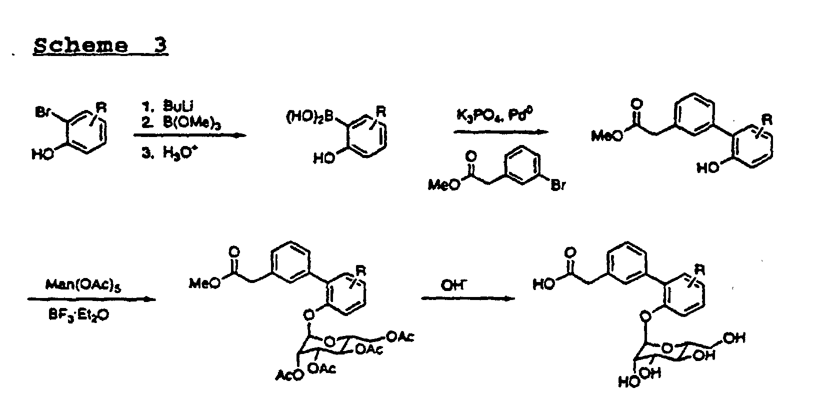

A substituted bromophenol is reacted first with butyl lithium, then with trimethoxy borate followed by acid hydrolysis to give a boronic acid. This compound is reacted with a substituted bromobenzene in a palladium(0) catalyzed coupling to give a biphenyl compound. The biphenyl compound is coupled with a protected mannopyranoside and the product is deprotected by base hydrolysis to form the desired compound.

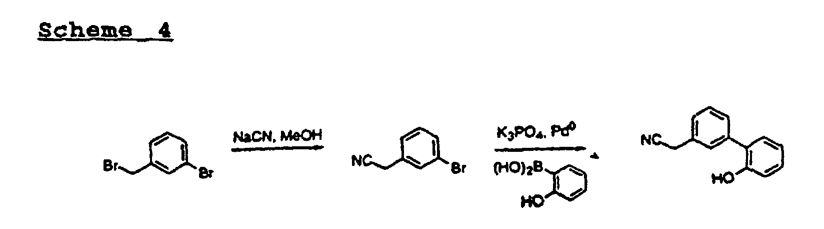

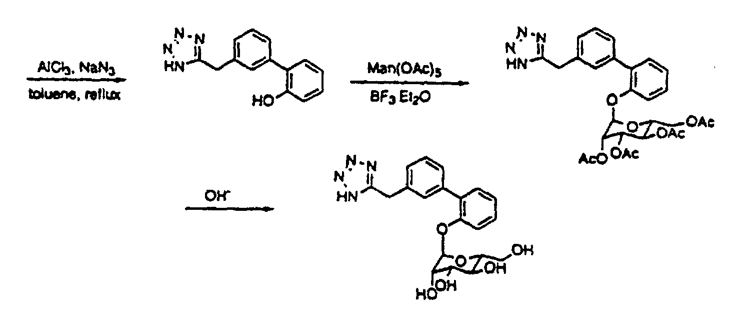

A bromobenzyl bromide is treated with sodium cyanide in refluxing methanol to give a bromobenzyl nitrile. This compound is coupled with a boronic acid in the presence of a palladium catalyst and base to give a substituted biphenyl compound. The nitrile functionality is converted to a tetrazole by treatment with sodium azide and aluminum chloride in refluxing toluene. The biphenyl tetrazole is coupled with a protected mannose derivative catalyzed by boron trifluoride etherate. Treatment with base deprotects the mannose and provides the desired compound.

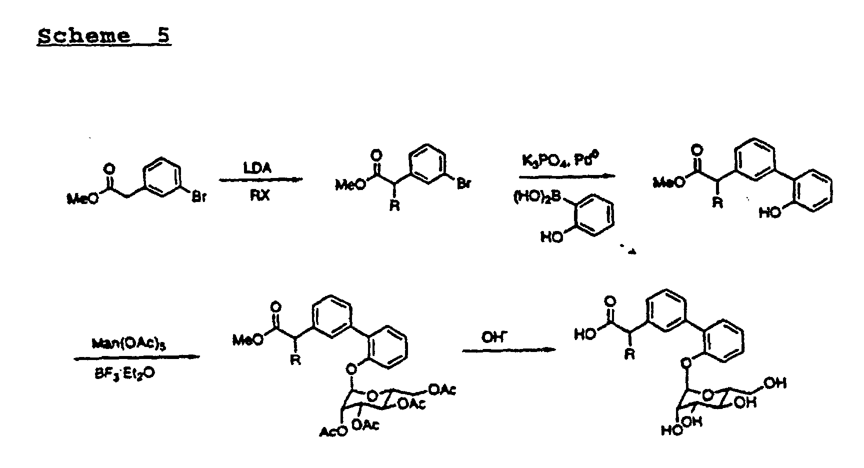

A bromophenyl acetate is treated with lithium diisopropylamide and an alkyl halide to give an α-alkyl bromophenyl acetate. This compound is coupled with a boronic acid to produce a biphenyl compound. The biphenyl compound is coupled with protected mannopyranoside using boron trifluoroetherate and the compound is deprotected by base hydrolysis to give the desired compound.

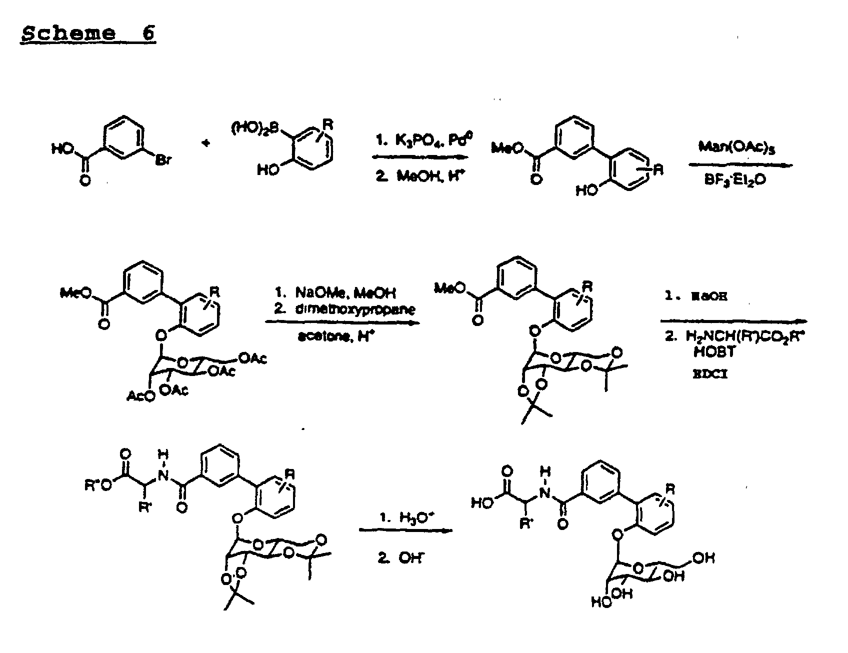

This scheme presents a general synthetic route to compounds having various amide functional groups. First, a phenolic boronic acid is reacted with a bromobenzoic acid to obtain a biphenyl using potassium phosphate and a catalytic amount of tetrakistriphenylphosphine palladium(0) in dimethylformamide and water. Next, the biphenyl moiety is esterified, then coupled with mannose pentaacetate. The acetate protecting groups are then converted to acetonide protecting groups using sodium methoxide in methanol followed by acetone and dimethoxypropane with a catalytic amount of p-toluenesulfonic acid. Base hydrolysis of the ester gives an acid that may be converted to various amide groups by reacting the acid with a particular amino ester, in the presence of standard coupling reagents. Then, the compound is deprotected to give the desired amide. 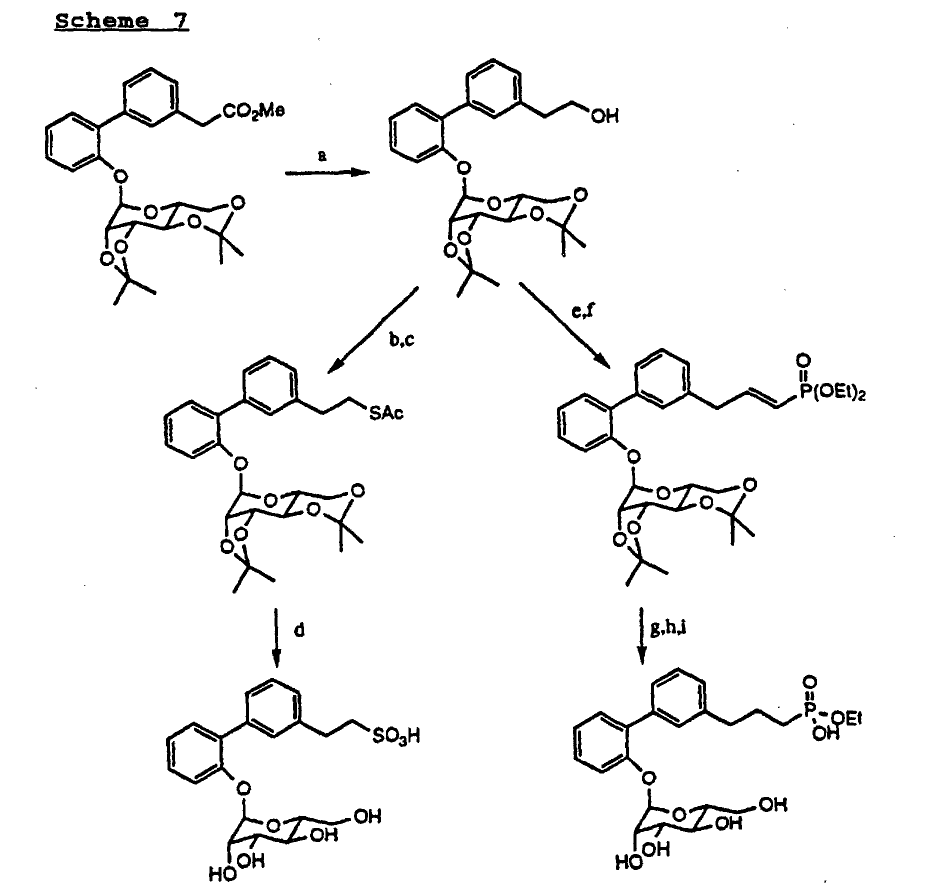 Reagents and conditions: a) LiBH₄, THF/toluene b) MsCl, Et₃N c) potassium thioacetate d) Oxone, H₂O/MeOH e) Dess-Martin periodinane, CH₂Cl₂ f) KCH(PO₃Et₂)₂, THF g) H₂, Pd/C, MeOH h) H⁺, H₂O i) NaOH, H₂O, Δ

A compound having a biphenyl moiety and an acetonide protected mannopyranoside moiety as synthesized in scheme 6 above may be reacted with lithium borohydride in tetrahydrofuran and toluene to produce a compound having a phenylethyl alcohol group on the biphenyl moiety. This may be reacted with mesyl chloride and triethylamine followed by potassium thioacetate to give a alkyl thioacetate; this thioacetate may be further treated with Oxone in water and methanol to give a sulfonic acid substituted biphenyl compound. Alternatively, the phenylethyl alcohol may be treated with the Dess-Martin reagent to afford an aldehyde, which may then be treated with tetraethylmethylene diphosphonate anion to produce an α,β-unsaturated phosphonate. Reduction with hydrogen and a palladium catalyst produces the corresponding diethyldiphosphonate, which may then be partially hydrolyzed with aqueous base to give an ethyl phosphonic acid.

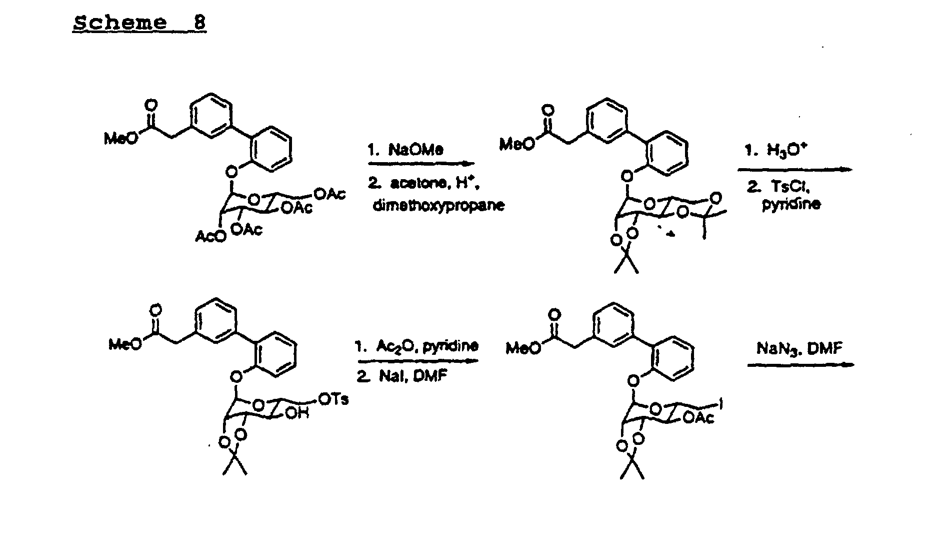

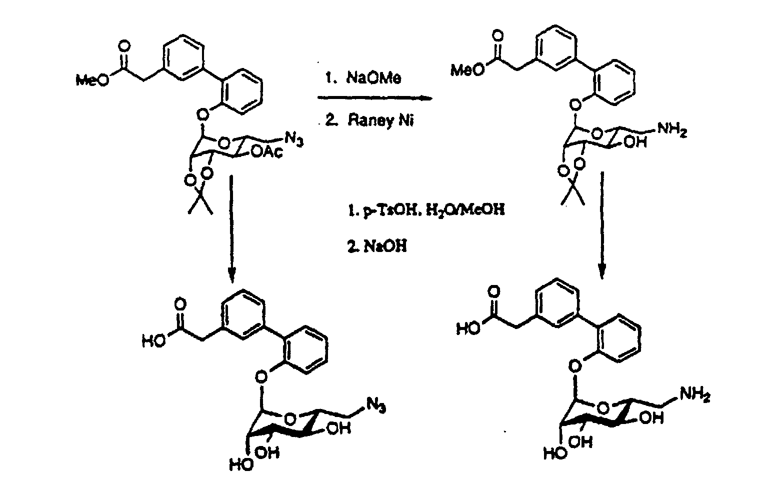

A substituted biphenyl compound having a protected mannose as prepared in Scheme 2 is treated first with sodium methoxide in methanol, then dimethoxypropane in acetone with catalytic acid to give a bisacetonide. Partial deprotection by mild acid hydrolysis, followed by reaction of the primary 6-hydroxyl group with tosyl chloride gives a hydroxy tosylate which is acetylated with acetic anhydride in pyridine. The tosylate group is replaced with an iodine using sodium iodide in dimethylformamide. Treatment of the iodide with sodium azide in DMF gives an azido compound which is either deprotected to give the 6-azido compound, or is reduced to an amine following removal of the acetate protecting group. Further removal of the remaining protecting groups by treatment with catalytic acid in methanol followed by hydrolysis gives the desired 6-amino compound.

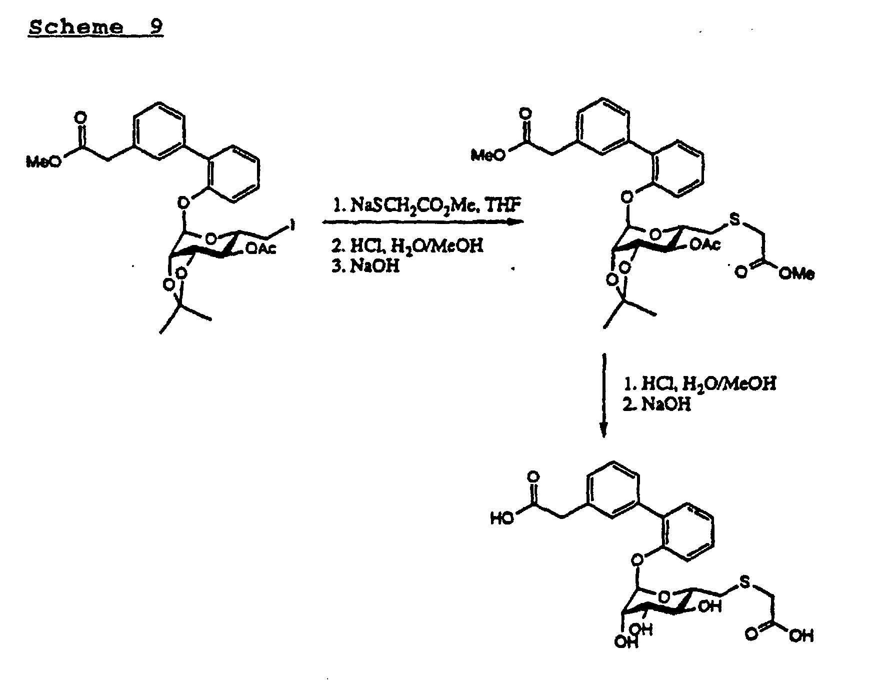

This scheme shows that the 6-iodomannopyranoside as synthesized in Scheme 8 can be displaced with a sulfur based nucleophile to generate the 6-mercapto-S-acetate ester, which can subsequently be deprotected by sequential mild acid then base hydrolysis to give the target compound.

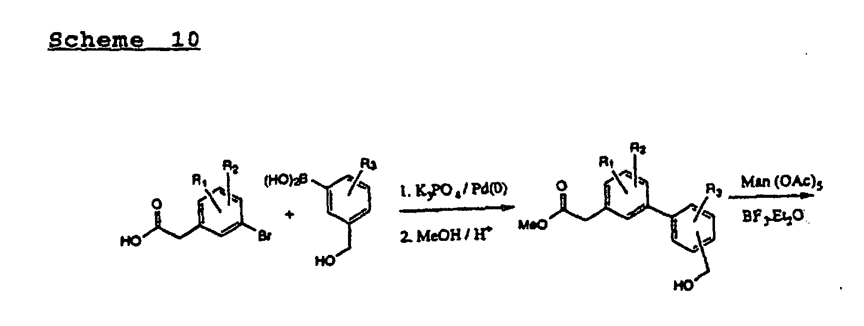

In this scheme, a substituted bromophenyl acetic acid is coupled with a substituted hydroxymethyl benzene boronic acid using a palladium (O) catalyst, and the product esterified. The resulting ester is glycosylated with a protected mannopyranoside in the presence of boron trifloride etherate. Hydrolysis of the protecting groups affords the desired compound.

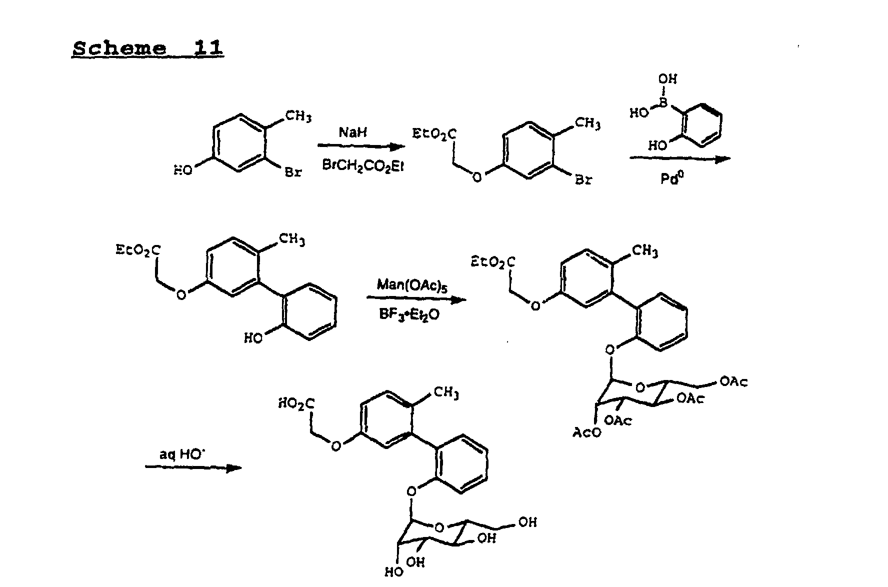

A substituted bromophenol is reacted with ethyl bromoacetate in the presence of sodium hydride to give a substituted bromophenyl ether which is coupled with a hydroxybenzene boronic acid using catalytic palladium (0) to give a substituted biphenyl. The phenol is reacted with a protected mannose unit using boron trifluoride etherate to give a protected mannopyranoside. Treatment with aqueous base gives the desired compound.

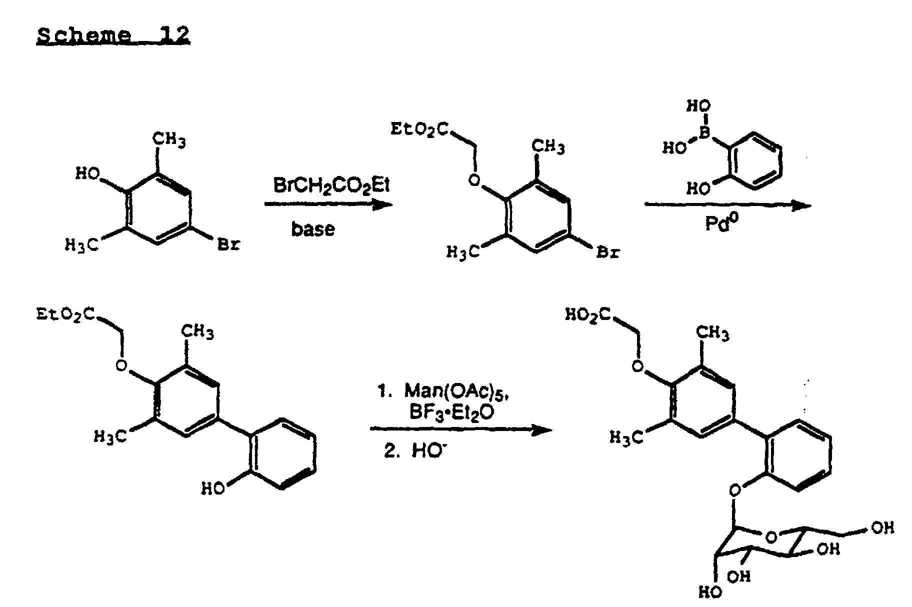

In this scheme, a substituted bromophenol is reacted with ethyl bromoacetate in the presence of a suitable base to give a bromophenyl ether. The halide is then reacted with a boronic acid in the presence of catalytic palladium (0) to produce a substituted biphenyl compound, which is coupled with a protected mannose unit in the presence of boron trifluoride etherate. The protective groups are then removed with aqueous hydroxide which produces the desired compound.

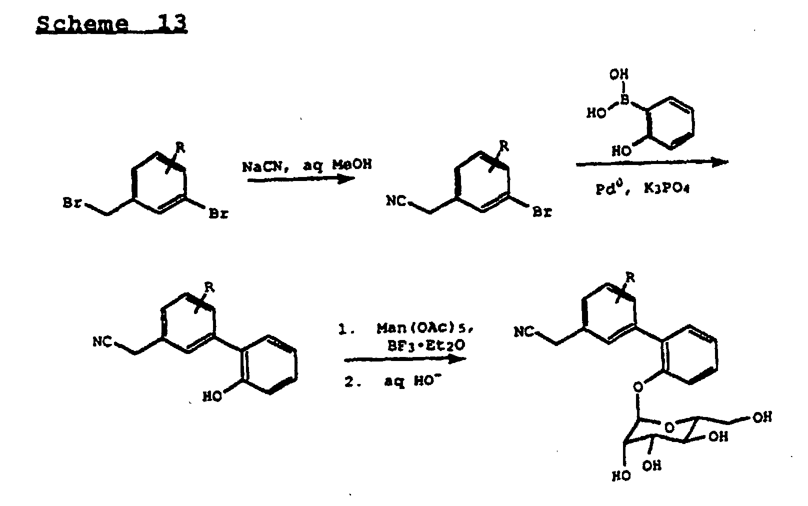

In this reaction scheme, a bromobenzyl bromide is reacted with sodium cyanide in methanol to give a cyanomethyl bromobenzene. The halide is then reacted with a boronic acid in the presence of catalytic tetrakistriphenylphosphine palladium (0) to produce a substituted biphenyl compound, which is coupled with a protected mannose unit in the presence of boron trifluoride etherate. The protective groups are then removed with aqueous hydroxide which produces the desired compound.

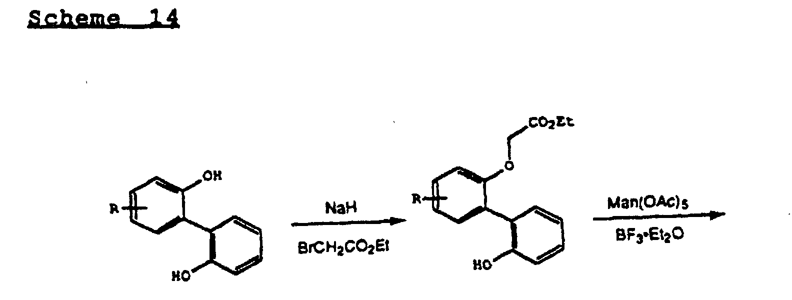

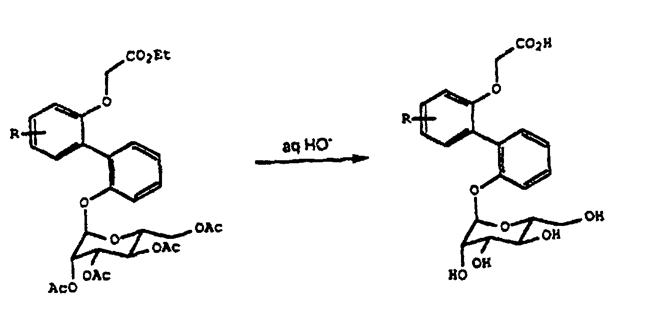

A substituted 2,2'-dihydroxybiphenyl is reacted with ethyl bromoacetate in the presence of sodium hydride to give a phenolic biphenyl ether. The phenol is reacted with a protected mannopyranoside using boron trifluoride etherate, and the protecting groups are then removed with aqueous base to give the desired compound.

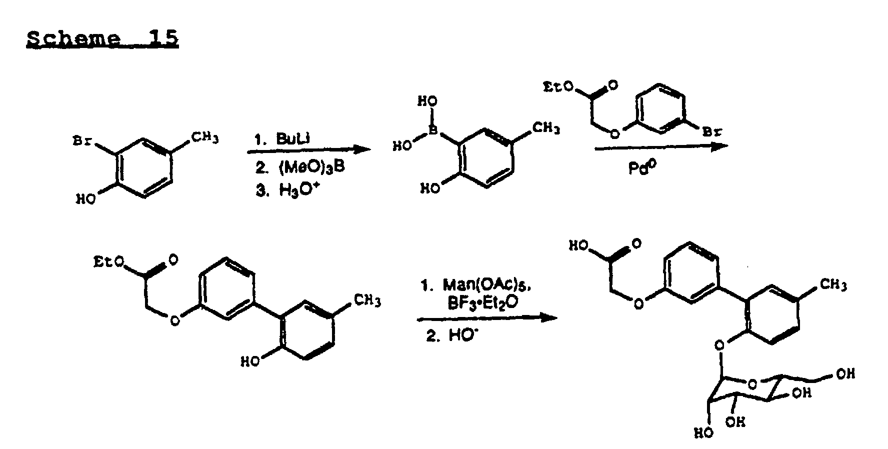

This scheme illustrates that a substituted bromophenol can be converted to a substituted benzene boronic acid by treatment with a suitable base followed by introduction of trimethyl borate then hydrolysis. The resulting boronic acid is then coupled with a bromophenyl ether in the presence of palladium (0) catalyst to give a substituted biphenyl phenol. The phenol is reacted with a protected mannose unit in the presence of boron trifluoride etherate, and the protective groups are then removed with aqueous base to give the desired product.

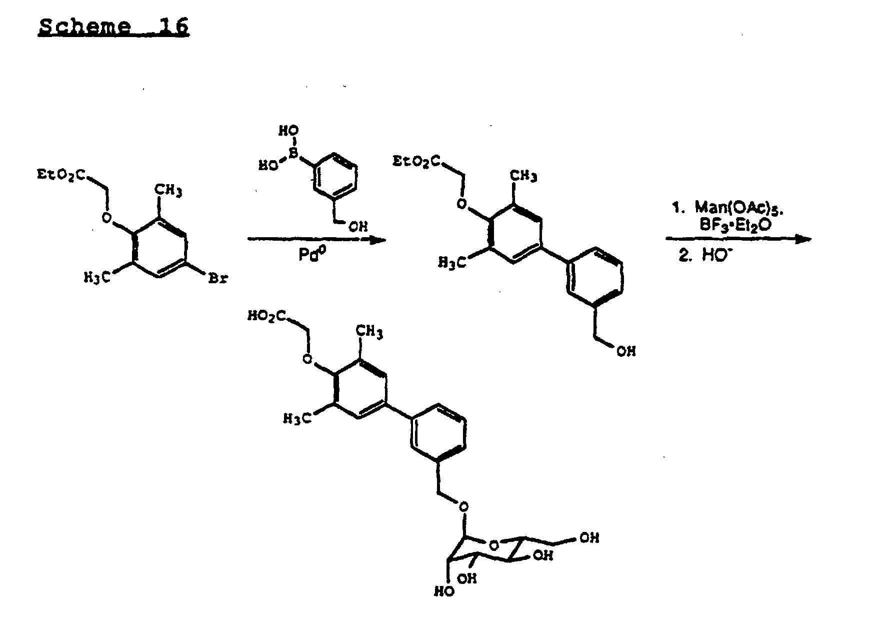

This scheme shows that a bromophenyl ether as prepared in scheme 2 can be reacted with a 3-hydroxymethylphenyl boronic acid in the presence of palladium (0) to give a substituted biphenyl compound. The hydroxy group is reacted with a protected mannose unit using boron trifluoride etherate as a coadditive. Treatment with aqueous base gives the desired compound. 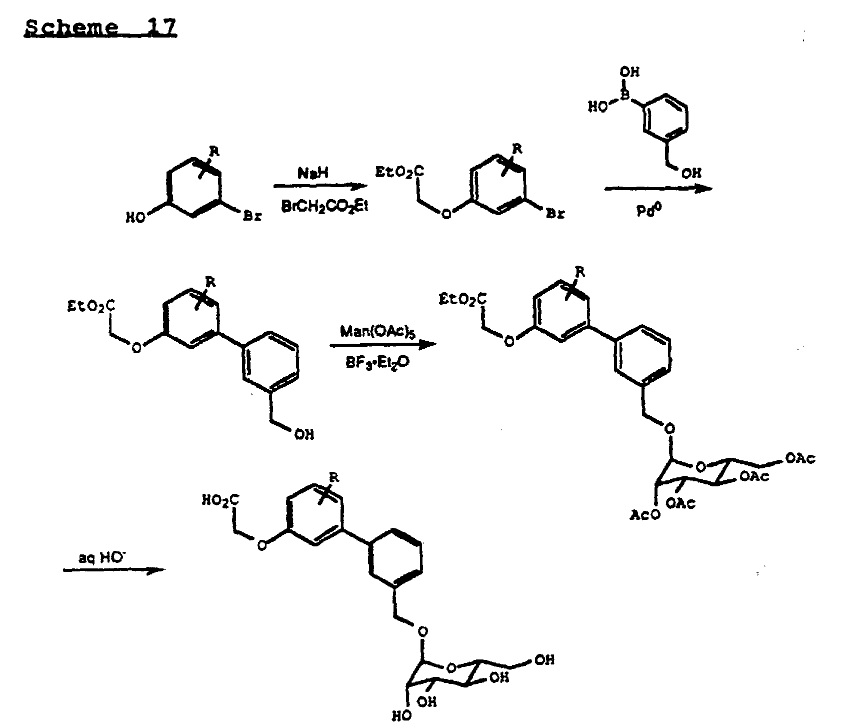 A substituted 3-bromophenol is reacted with ethyl bromoacetate in the presence of sodium hydride to give a substituted bromophenyl ether which is coupled with a 3-hydroxymethyl benzene boronic acid using catalytic palladium (0) to give a substituted biphenyl compound. The benzylic alcohol is reacted with a protected mannose unit using boron trifluoride etherate to give a protected mannopyranoside. Treatment with aqueous base, then gives the desired compound.

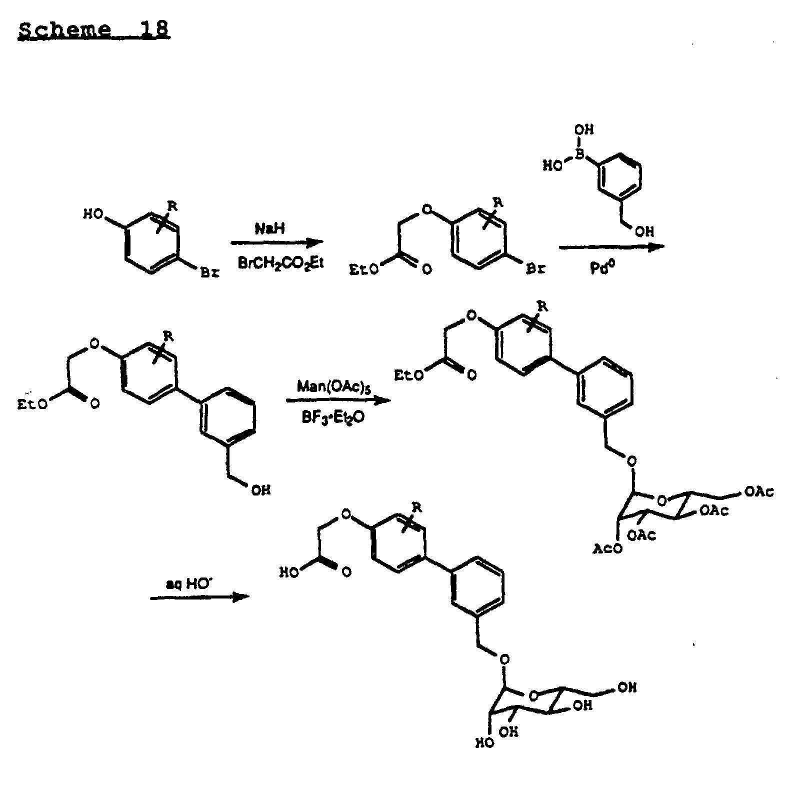

In this scheme, a substituted 4-bromophenol is reacted with ethyl bromoacetate in the presence of sodium hydride to give a bromophenyl ether. The halide is coupled with 3-hydroxymethyl benzene boronic acid using catalytic palladium (0) to give a substituted biphenyl compound. A protected mannose moiety is introduced, and the resulting glycoside is treated with aqueous base to give the desired compound.

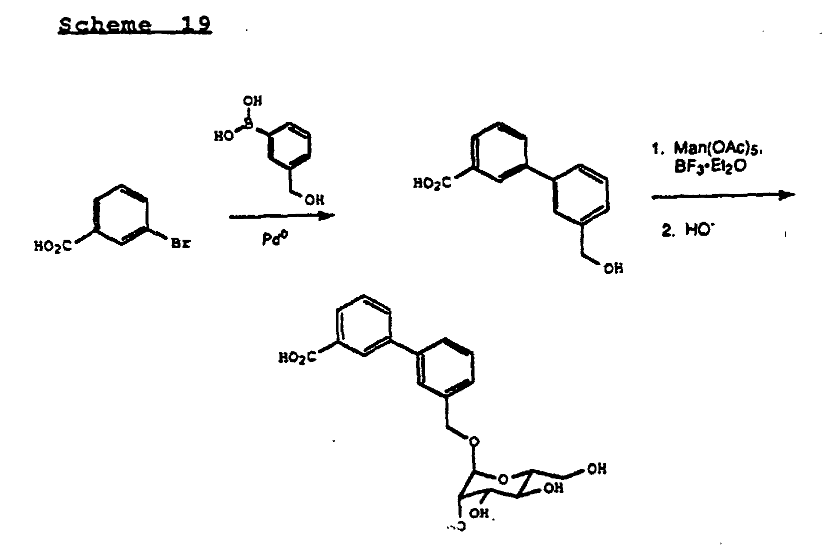

In this scheme, a bromo benzoic acid is coupled with a 3-hydroxymethyl phenyl boronic acid using palladium (0) as a catalyst. Reaction with a protected mannose unit, followed by base-induced deprotection gives the desired compound.

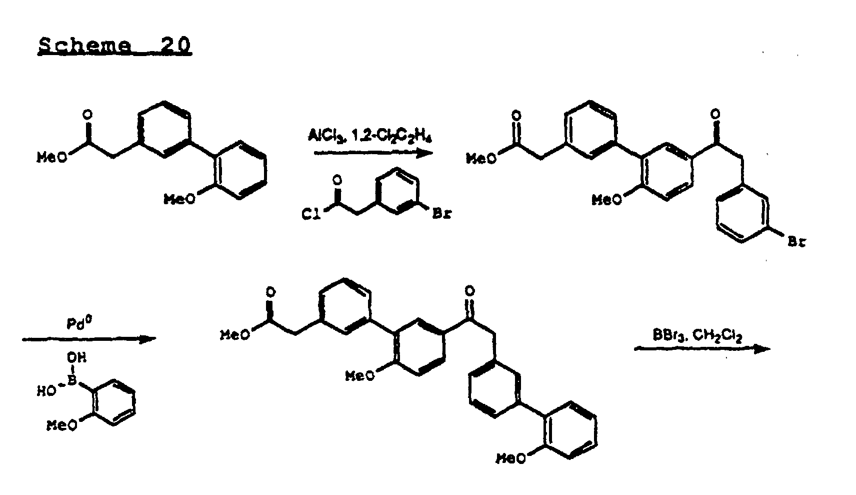

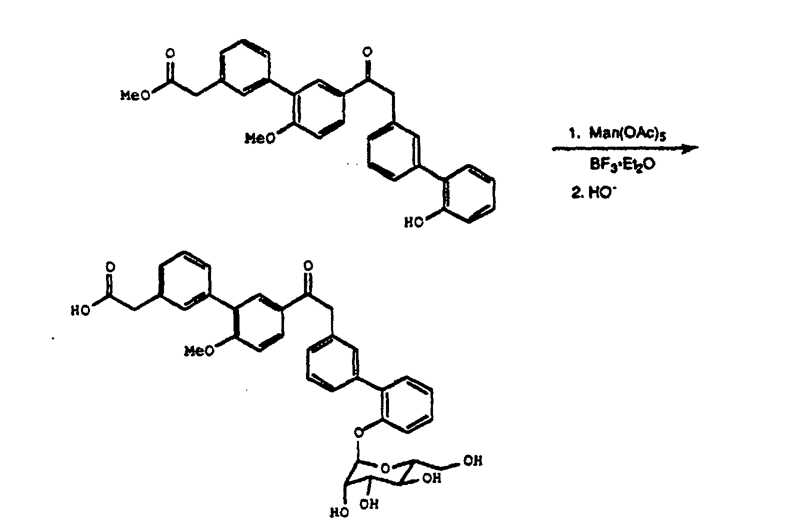

In this scheme, methyl-3-(2-methoxyphenyl)phenyl acetate as prepared in part B, Example 1 is reacted with a bromophenylacetic acid chloride using aluminum chloride in a suitable solvent to give a substituted acetophenone. Reaction with an aryl boronic acid in the presence of catalytic palladium (0) and aqueous sodium carbonate in toluene gives a substituted tetra-aryl compound. Treatment with boron tribromide in a halogenated solvent at low temperature gives a phenol which is reacted with a protected mannopyranoside using boron trifluoride etherate. Treatment with aqueous base gives the desired compound.

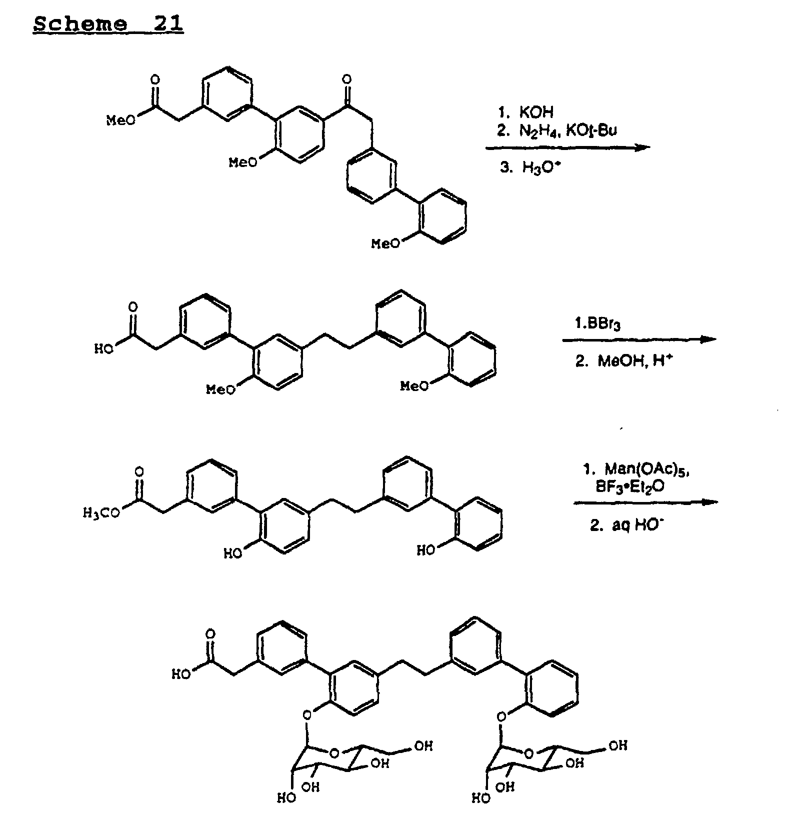

In this scheme, a tetra-aryl compound as synthesized in scheme 20 is treated with aqueous base to convert the ester functionality to a carboxylate salt, which is then treated with hydrazine and potassium *tert*-butoxide followed by acidification to give a disubstituted ethane moiety. The ether groups are removed using boron tribromide in a halogenated solvent, and the carboxylate is re-esterified to give a diphenolic ester. Reaction of the phenols with a protected mannose unit, followed by base-induced deprotection gives the desired compound.

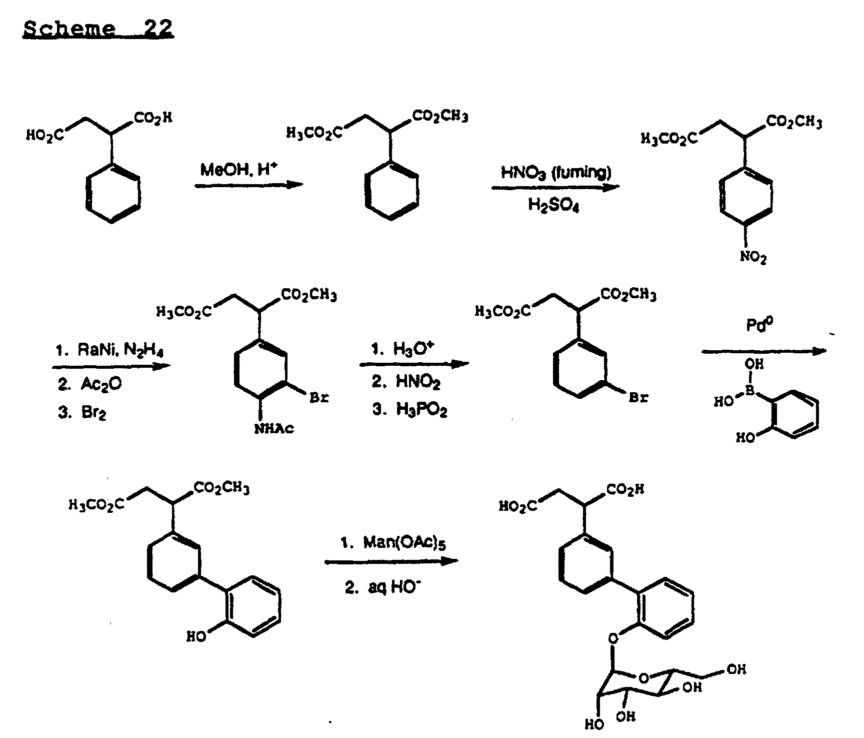

In this scheme, a phenyl succinic acid is esterified using an alcohol in the presence of catalytic acid to give a phenyl diester which is then treated with fuming nitric acid and sulfuric acid to give a nitro phenyl diester. The nitro group is reduced using Raney nickel and hydrazine, or other suitable reduction conditions known to those skilled in the art, and the resulting amino group is reacted with acetic anhydride to give an acetamide. Introduction of the halogen is accomplished using bromine in a suitable solvent, and the acetamide is then hydrolyzed using aqueous acid. The resultant amino group is removed by treatment with nitrous acid followed by hypophosphorous acid. The aryl halide is then reacted with a hydroxyphenyl boronic acid in the presence of catalytic palladium (0) to give a biphenyl phenol which is then reacted with a protected mannose unit. Treatment with aqueous base removes the protective groups and provides the desired compound.

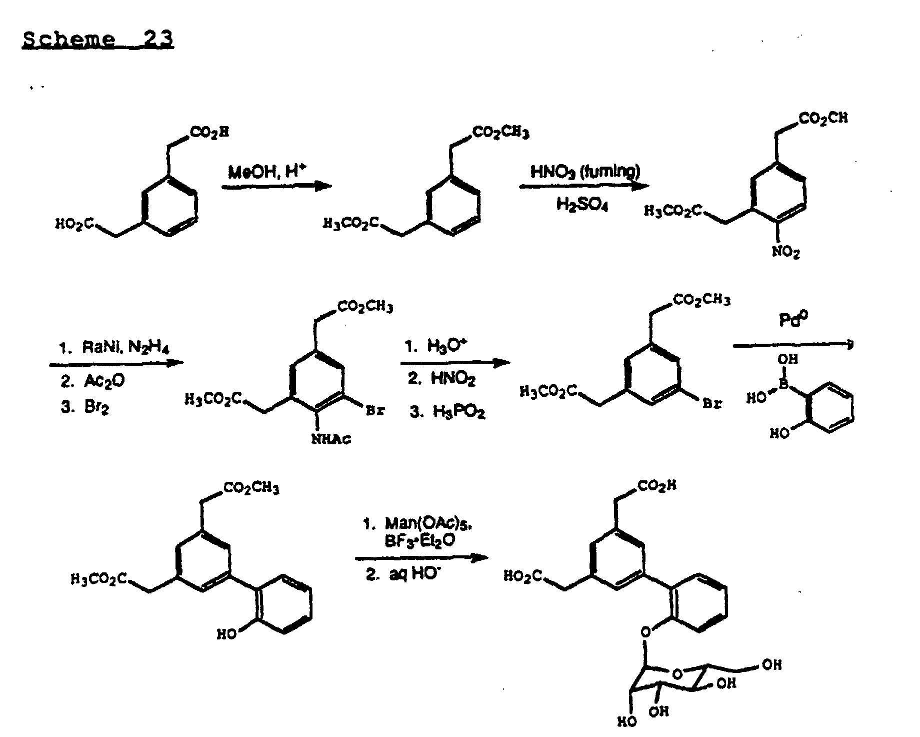

A phenyl diacetic acid is treated with an alcohol in the presence of catalytic acid to give a diester. The aromatic ring is then nitrated using fuming nitric acid and sulfuric acid to give a nitro phenyl diester, and the nitro group is reduced using Raney Nickel and hydrazine, or other suitable reduction conditions known to those skilled in the art. The resulting amino group is then reacted with acetic anhydride to give an acetamide. The halogen is introduced using bromine in a suitable suitable solvent, and the acetamide is then hydrolyzed using aqueous acid. introduced using bromine in a suitable solvent, and The resultant amino group is removed by treatment with nitrous acid followed by hypophosphorous acid, and the aryl halide is then reacted with a hydroxyphenyl boronic acid in the presence of catalytic palladium (0) to give a biphenyl phenol. Treatment with a protected mannose unit and boron trifluoride etherate gives a mannopyranoside, which is then treated with aqueous base to give the desired compound.

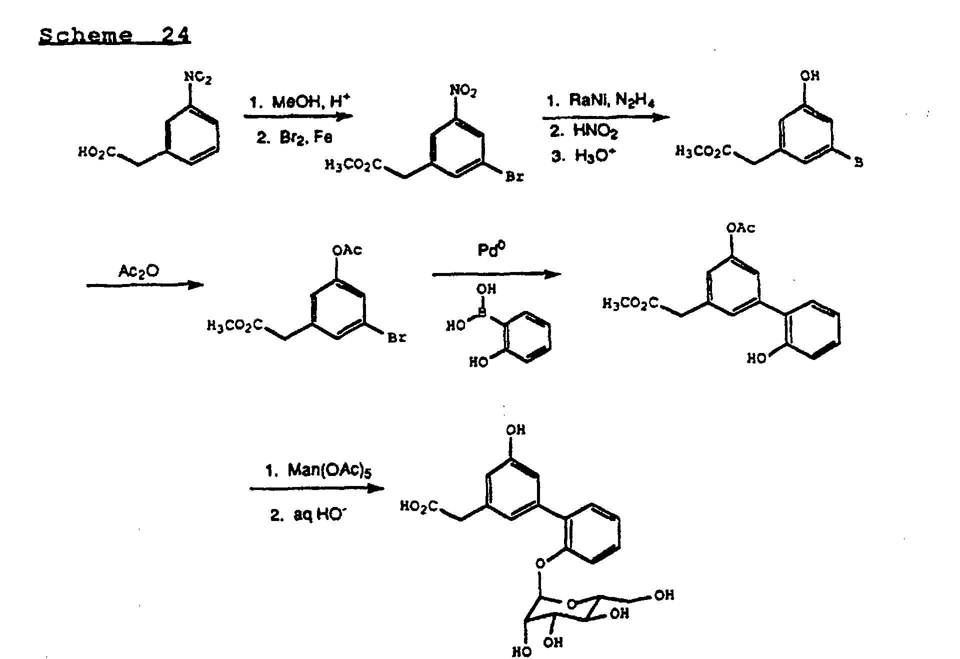

A nitrophenyl acetic acid is first converted to an ester using an alcohol and catalytic acid, then the aromatic ring is halogenated using bromine and iron in a suitable solvent. The nitro group is then reduced using hydrazine and Raney nickel. The resulting amino group is diazotized using sodium nitrite in aqueous sulfuric acid, and the diazonium salt is hydrolyzed to the phenol with aqueous acid. The phenol is protected using acetic anhydride and the aromatic halide is then reacted with a hydroxyphenyl boronic acid in the presence of catalytic palladium (0) to give a biphenyl phenol. Treatment with a protected mannose moiety followed by base-induced deprotection gives the desired compound.acetic anhydride to give an acetamide. The halogen is

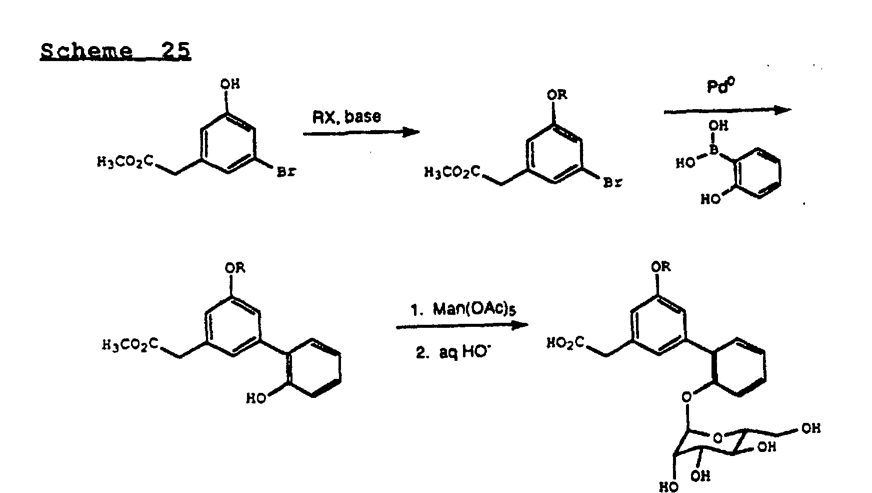

This scheme shows that the bromophenol as prepared in Scheme 24, can be alkylated using an alkyl or aryl halide and suitable base. Reaction of the resulting aryl haloether with a hydroxyphenyl boronic acid using catalytic palladium (0) gives a biphenyl phenol which is then reacted with a protected mannose unit. Treatment with aqueous base gives the desired compound.

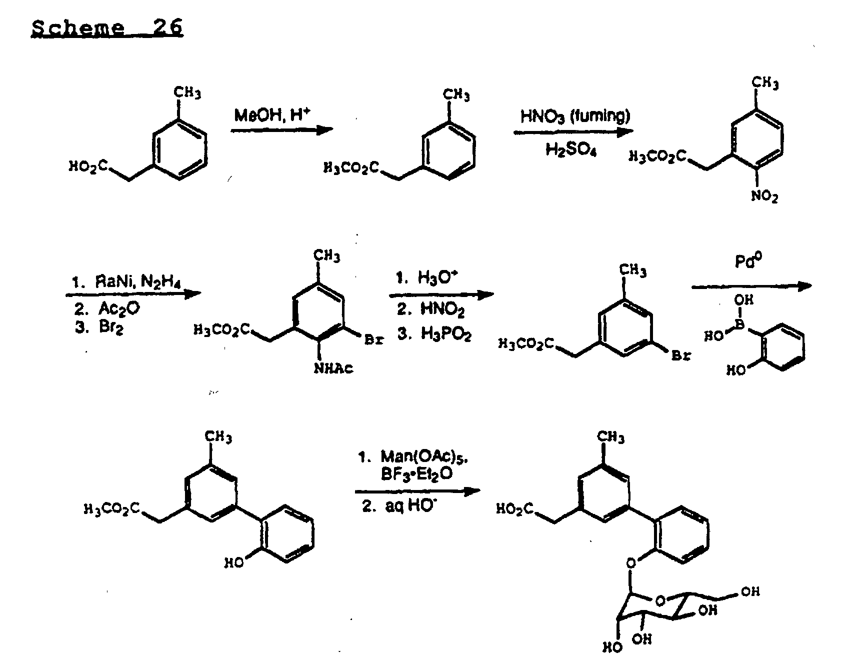

A 3-alkyl phenyl acetic acid is treated with an alcohol in the presence of catalytic acid to give an ester, which is then nitrated using fuming nitric acid and sulfuric acid. The nitro group is reduced using methods known to those skilled in the art, and the resulting amino group is acetylated using acetic anhydride. Treatment with bromine in a suitable solvent followed by hydrolysis of the acetamide gives a bromoaniline. The amino group is removed by a sequence of diazotization followed by reduction of the diazonium salt with hypophosphorous acid. The aromatic halide is coupled with a hydroxyphenyl boronic acid in the presence of catalytic palladium (0) to give a biphenyl phenol, which is coupled with mannose pentaacetate. The compound is then deprotected with aqueous base to give the desired compound.

The following examples further illustrate the invention and are not to be construed as limiting the specification or the claims in any way.

### EXAMPLES:

### EXAMPLE 1

### 3-(2-(α-D-Mannopyranosyloxy)phenyl)phenyl acetic acid

Part A: 3-Bromophenyl acetic acid (2.0 g, 9.3mmol) was dissolved in methanol (20 ml) in a 50 ml flask. Concentrated sulfuric acid (2 drops) was added, and the mixture was refluxed under nitrogen for ten hours then concentrated under reduced pressure. The residue was mixed with dichloromethane (20 ml) and saturated sodium bicarbonate solution (10 ml). The organic material was separated, dried (MgSO₄) and concentrated under reduced pressure. The residue was flushed through silica gel with hexane/ethyl acetate (3:1), and concentrated to provide 2.12 g (99%) of methyl (3-bromophenyl)acetate, which was used without further purification.

Part B: Anisole (2.16 g, 20.0 mmol) was dissolved in dry THF (50 ml) in a dry 100 ml flask flushed with nitrogen. The mixture was chilled in a dry ice/2-propanol bath, n-butyl lithium (10.9 ml of a 2.3 M solution in hexanes, 25 mmol) was added, then the cooling bath was exchanged for an ice-water bath. The reaction was stirred for an hour at 0°C, then trimethyl borate (2.3 ml, 20 mmol) was added and the mixture was stirred at room temperature overnight. The reaction mixture was treated with 2N aqueous HCl to pH 3 and mixed well for 30 minutes, then extracted with ether (3 X 15 ml). The organic materials were combined, dried (MgSO₄), then concentrated under reduced pressure which gave 2.88 g (95%) of 2-methoxybenzene boronic acid as a clear oil which was used in the next step without further purification.

Methyl (3-bromophenyl)acetate (2.06 g, 9.0 mmol), tetrakis(triphenylphosphine)palladium (0) (115 mg), sodium carbonate (2.61 g, 25 mmol in 2ml water) and toluene (10 ml) were degassed under nitrogen in a 25 ml flask fitted with a reflux condenser. 2-Methoxybenzene boronic acid (1.5 g, 9.87 mmol) in toluene (1 ml) was added and the mixture was heated at reflux overnight, then mixed with 1:1 saturated sodium chloride/ethyl acetate (15 ml). The organic materials were separated, dried (MgSO₄), then concentrated under reduced pressure which gave 2.81 g of methyl (3-(2-methoxyphenyl)phenyl) acetate.

Part C: In a dry 250 ml flask, methyl 3-(2-methoxyphenyl)phenyl acetate (2.0 g, 7.8 mmol) was dissolved in dichloromethane (100 ml) under nitrogen, and chilled in a dry-ice/2-propanol bath. Boron tribromide (2.2 ml, 24 mmol) was added slowly drop-wise and the mixture was kept at -10°C for 14 hours, then mixed with ice-water (100 ml). The organic material was separated, washed with saturated sodium bicarbonate solution (50 ml), water (50 ml), saturated sodium chloride (60 ml), then dried (MgSO₄) and concentrated under reduced pressure. The residue was purified by flash chromatography (SiO₂, 3:1 hexane/ethyl acetate) which gave 1.25 g (66% from methyl (3-bromophenyl) acetate) of methyl 3-(2-hydroxyphenyl)phenyl acetate as a clear oil.

Part D: Methyl 3-(2-hydroxyphenyl)phenyl acetate (1.28 g, 5.28 mmol) was dissolved in 1,2-dichloroethane (20 ml) in a dry 50 ml flask. α-D-Mannose pentaacetate (2.08 g, 5.34 mmol) was added in one portion, then borontrifluoride etherate (2.32 ml, 18.5 mmol) was added slowly. The mixture was stirred under nitrogen overnight at room temperature, then mixed with water (50 ml). The organic material was separated and the aqueous portion was extracted with dichloromethane (3 X 5 ml). The extracts were combined with the original organic fraction, dried (MgSO₄), then concentrated under reduced pressure. The residue was purified by flash chromatography (SiO₂, gradient elution hexane to 3:1 hexane/ethyl acetate) which provided 2.74 g (91%) of methyl 3-(2-(2,3,4,6-tetra-O-acetyl-α-D-mannopyranosyloxy)phenyl)phenyl acetate contaminated with a small amount of unreacted α-D-mannose pentaacetate which co-eluted with the product.

Part E: Methyl 3-(2-(2,3,4,6-tetra-O-acetyl-α-D-mannopyranosyloxy)phenyl)phenyl acetate (2.74 g, 4.78 mmol) was dissolved in acetonitrile ( 25 ml) in a 50 ml flask, and treated with a solution of lithium hydroxide monohydrate (1.1 g, 26.3 mmol) in water (10 ml) and the mixture was stirred at room temperature overnight then acidified to pH 2 with concentrated hydrochloric acid. The mixture was concentrated under reduced pressure and the residue was purified by HPLC (reverse phase, gradient elution 5-50% acetonitrile in water (0.1% trifluoroacetic acid), monitored at 254 nm) which gave 0.87 g (47%) of 3-(2-(α-D-mannopyranosyloxy)phenyl)phenyl acetic acid as a white solid, m.p. 85-86°C.
¹H NMR: (300MHz, DMSO-d6) 7.02-7.40 (comp,8H), 5.31 (s, 1H), 3.25-4.00 (comp, 12H) ppm.
IR (KBr): 3408, 1791, 1713, 1478, 1223, 1171, 1019, 979, 755 cm⁻¹.
Analysis: Calc for C₂₀H₂₂O₈_1.5 [H₂O]: 57.55% C, 5.76% H.
Found 57.33% C, 5.59% H.

### EXAMPLE 2

### 4-(2-(α-D-Mannopyranosyloxy)phenyl)phenyl acetic acid

Part A: Operating as in Part A of EXAMPLE 1, but employing 4-bromophenyl acetic acid gave methyl 4-bromophenyl acetate in 85% yield.

Part B: 2-Bromophenol (10.0 g, 57.8 mmol) was dissolved in dry THF (100 ml) in a dry 250ml flask flushed with nitrogen. The mixture was chilled in a dry ice/2-propanol bath, n-butyl lithium (51 ml of a 2.5 M solution in hexanes, 127.2 mmol) was added, then the cooling bath was exchanged for an ice-water bath. The reaction was stirred for an hour at 0°C, then trimethyl borate (6.9 ml, 60.7 mmol) was added to the slurry which became homogeneous after a few minutes. The mixture was stirred at room temperature overnight, then treated with 2N aqueous HCl to pH 3, mixed well for 30 minutes and extracted with ether (3 X 25 ml). The organic materials were combined, dried (MgSO₄), then concentrated under reduced pressure which gave 7.6 g (91%) of 2-hydroxybenzene boronic acid as a white solid, m.p. 156-158°C.

Methyl (4-bromophenyl)acetate (2.79 g, 12.2 mmol), tetrakis(triphenylphosphine)palladium (0) (170 mg), potassium phosphate (9.71 g, 45.75 mmol) and dimethoxyethane (50 ml) were degassed under nitrogen in a 100 ml flask fitted with a reflux condenser. 2-Hydroxybenzene boronic acid (2.52 g, 18.3 mmol) in dimethoxyethane (5 ml) was added and the mixture was heated at reflux overnight then mixed with 1:1 saturated sodium chloride/ethyl acetate (25 ml). The organic materials were separated, dried (MgSO₄), then concentrated under reduced pressure. The residue was purified by flash chromatography (SiO₂, gradient hexane to 3:1 hexane/ethyl acetate) which provided 2.21 g (75%) of methyl (4-(2-hydroxyphenyl)phenyl)acetate.

Part C: Operating as in Part D in EXAMPLE 1, but employing methyl (4-(2-hydroxyphenyl)phenyl)acetate gave methyl (4-(2-(2,3,4,6-tetra-O-acetyl-α-D-mannopyranosyloxy)phenyl)phenyl acetate in 86% yield.

Part D: Operating as in Part E in EXAMPLE 1, but employing methyl (4-(2-(2,3,4,6-tetra-O-acetyl-α-D-mannopyranosyloxy)phenyl)phenyl acetate gave 4-(2-(α-D-mannopyranosyloxy)phenyl)phenyl acetic acid as a hygroscopic white solid.
¹H NMR: (300MHz, DMSO-d6) 7.02-7.50 (comp, 8H), 5.30 (s, 1H), 3.30-3.75 (comp, 12H) ppm.
IR (KBr) : 3404, 1788, 1712, 1486, 1218, 1170, 1018, 752 cm⁻¹.
m.p.: 65-68°C.
Analysis: Calc for C₂₀H₂₂O₈• [C₂HF₃O₂]: 52.38% C, 4.59% H.
Found: 52.02% C, 4.52% H.

### EXAMPLE 3

### 3-(2-(α-D-Mannopyranosyloxy)phenyl)benzoic acid, lithium salt

Part A: Operating as in Part A in EXAMPLE 1, but employing 3-bromobenzoic acid gave methyl 3-bromobenzoate in 95% yield.

Part B: Operating as in Part B in EXAMPLE 1, but employing methyl 3-bromobenzoate gave methyl 3-(2-methoxyphenyl)benzoate in 64% yield, m.p.: 92-93°C.

Part C: Operating as in Part C in EXAMPLE 1, but employing methyl 3-(2-methoxyphenyl)benzoate gave methyl 3-(2-hydroxyphenyl)benzoate in 84% yield.

Part D: Operating as in Part D in EXAMPLE 1, but employing methyl 3-(2-hydroxyphenyl)benzoate gave methyl 3-(2-(2,3,4,6-tetra-O-acetyl-α-D-mannopyranosyloxy)phenyl)benzoate in 85% yield.

Part E: In a dry 50 ml flask under nitrogen, methyl 3-(2-(2,3,4,6-tetra-O-acetyl-α-D-mannopyranosyloxy)phenyl)benzoate (2.18 g, 3.9 mmol) was dissolved in methanol (20 ml) and treated in one portion with sodium methoxide (250 mg) and the mixture was stirred at room temperature overnight, then concentrated under reduced pressure. The residue was purified by flash chromatography (SiO₂, 7:3 methylene chloride/methanol) which gave methyl 3-(2-(α-D-mannopyranosyloxy)phenyl)benzoate in 84% yield.

Part F: Methyl 3-(2-(α-D-mannopyranosyloxy)phenyl)benzoate (0.662 g, 1.7 mmol) was dissolved in acetonitrile (15 ml). An aqueous solution of lithium hydroxide monohydrate (0.12 g, 2.55 mmol in 1 ml water) was added and the mixture was stirred at room temperature for 8 hours. The mixture was then diluted with additional acetonitrile (approximately 10 ml) and the lithium salt of 3-(2-(α-D-mannopyranosyloxyphenyl)benzoic acid precipitated. The solids were collected and dried which gave 0.614 g (94%) of product, m.p. 109-115°C.
¹H NMR: (300MHz, DMSO-d6) 8.11 (s, 1H), 7.79 (d, J=7Hz, 1H), 7.20-7.40 (comp, SH), 7.06 (t, J=7 Hz, 1H), 5.43 (s, 1H), 5.25 (br s, 1H), 5.04 (br s, 1H), 4.70 (br s, 1H), 4.55 (br s, 1H), 3.25-3.70 (comp, 6H) ppm.
IR (KBr): 3384, 1560, 1405, 1389, 1111, 1057, 1020, 757cm⁻¹.
Analysis: Calc for C₁₉H₁₉O₈Li•[H₂O]•1.8[LiOH]: 51.4% C, 5.18% H.
Found: 51.62% C, 4.81% H.

### EXAMPLE 4

### 4-(2-(α-D-Mannopyranosyloxy)phenyl)benzoic acid

Part A: Operating as in Part A in EXAMPLE 1, but employing 4-bromobenzoic acid gave methyl 4-bromobenzoate in 90% yield, m.p. 66-68°C.

Part B: Operating as in Part B in EXAMPLE 1, but employing methyl 4-bromobenzoate gave methyl 4-(2-methoxyphenyl)benzoate in 51% yield.

Part C: Operating as in Part C in EXAMPLE 1, but employing methyl 4-(2-methoxyphenyl)benzoate gave methyl 4-(2-hydroxyphenyl)benzoate in 71% yield.

Part D: Operating as in Part D in EXAMPLE 1, but employing methyl 4-(2-hydroxyphenyl)benzoate gave methyl 4-(2-(2,3,4,6-tetra-O-acetyl-α-D-mannopyranosyloxyphenyl)benzoate in 95% yield.

Part E: Operating as in Part E in EXAMPLE 1, but employing methyl 4-(2-(2,3,4,6-tetra-O-acetyl-α-D-mannopyranosyloxy)phenyl)benzoate gave 4-(2-(α-D-mannopyranosyloxy)phenyl)benzoic acid in 71% yield, m.p. 248-249°C.
¹H NMR: (300MHz, DMSO-d6) 7.98 (d, J=8Hz, 2H), 7.61 (d, J=8Hz, 2H), 7.30-7.40 (comp, 3H), 7.05-7.16 (comp, 1H), 5.36 (s, 1H), 4.90-5.05 (br s, 1H), 4.76-4.90 (br s, 1H), 4.65-4.76 (br s, 1H), 4.40-4.58 (br s, 1H), 3.25-3.70 (comp, 6H) ppm.
IR (KBr) : 3511, 3398, 2929, 1683, 1614, 1485, 1419, 1314, 1259, 1107, 1013, 986, 746 cm⁻¹.
Analysis: Calc for C₁₉H₂₀O₈•0.25 [H₂O]: 59.92% C, 5.43% H.
Found: 59.80% C, 5.25% H.

### EXAMPLE 5

### 3-(2-(α-D-Mannopyranosyloxy)phenyl)phenyloxyacetic acid

Part A: 3-Bromophenol (2.84 g, 16.4 mmol) was dissolved in dimethylformamide (50 ml) in a dry 100 ml flask under nitrogen. Sodium hydride (0.7 g of a 60% suspension in mineral oil, washed with hexane, 16.7 mmol) was added in portions and the mixture was stirred for one hour at room temperature. Ethyl bromoacetate (1.85 ml, 16.7 mmol) was added drop-wise and the reaction was stirred overnight at room temperature. Approximately two-thirds of the solvent was removed under reduced pressure and the residue was mixed with water (150 ml) and extracted with methylene chloride (3 X 20 ml). The extracts were combined, washed with water (50 ml), saturated sodium chloride solution (50 ml), then dried (MgSO₄). The solution was filtered and concentrated which gave 4.18 g (98%) of ethyl 3-bromophenyloxyacetate.

Part B: Operating as in Part B in EXAMPLE 2, but employing ethyl 3-bromophenyloxyacetate gave ethyl 3-(2-hydroxyphenyl)phenyloxyacetate in 52% yield.

Part C: Operating as in Part D in EXAMPLE 1, but employing ethyl 3-(2-hydroxyphenyl)phenyloxyacetate gave ethyl 3-(2-(2,3,4,6-tetra-O-acetyl-α-D-mannopyranosyloxy)phenyl)phenyloxyacetate in 69% yield.

Part D: Operating as in Part E in EXAMPLE 1, but employing ethyl 3-(2-(2,3,4,6-tetra-O-acetyl-α-D-mannopyranosyloxy)phenyl)phenyloxyacetate gave 3-(2-(α-D-mannopyranosyloxy)phenyl)phenyloxyacetic acid in 82% yield, m.p. 58-60°C.
¹H NMR: (300MHz, DMSO-d6) 7.25-7.38 (comp, 4H), 7.06-7.15 (comp, 2H), 6.97 (s, 1H), 6.90. (mult, 1H), 5.34 (mult, 1H), 4.70 (s, 2H), 3.80-4.40 (br s, 4H), 3.30-3.75 (comp, 6H) ppm.
IR (KBr): 3404, 2943, 1788, 1737, 1478, 1424, 1220, 1173, 1068, 1016, 979, 755, 696 cm⁻¹.
Analysis: Calc for C₂₀H₂₂O₉•[H₂O]•1.1[C₂HF₃O₂]: 48.50% C, 4.60% H.
Found: 48.70% C, 4.21% H.

### EXAMPLE 6

### 4-(2-(α-D-Mannopyranosyloxy)phenyl)phenyloxyacetic acid

Part A: Operating as in Part A in EXAMPLE 5, but employing 4-bromophenol gave ethyl 4-bromophenyloxyacetate in 98% yield.

Part B: Operating as in Part B in EXAMPLE 2, but employing ethyl 4-bromophenyloxyacetate gave ethyl 4-(2-hydroxyphenyl)phenyloxyacetate in 41% yield.

Part C: Operating as in Part D in EXAMPLE 1, but employing ethyl 4-(2-hydroxyphenyl)phenyloxyacetate gave ethyl 4-(2-(2,3,4,6-tetra-O-acetyl-α-D-manno-pyranosyloxy)phenyl)phenyloxyacetate in 80% yield.

Part D: Operating as in Part E in EXAMPLE 1, but employing ethyl 4-(2-(2,3,4,6-tetra-O-acetyl-α-D-mannopyranosyloxy)phenyl)phenyloxyacetate gave 4-(2-(a-D-mannopyranosyloxy)phenyl)phenyloxyacetic acid in 69% yield, m.p.: 145-146°C.
¹H NMR: (300MHz, DMSO-d6) 7.42 (d, J=BHz, 2H), 7.23-7.35 (comp, 3H), 7.07 (t, J=7Hz, 1H), 6.95 (d, J=8Hz, 2H), 5.30 (s, 1H), 4.71 (s, 2H), 3.30-3.80 (comp, 10H) ppm.
IR (KBr): 3418, 2930, 1739, 1521, 1486, 1240, 1219, 1110, 1068, 1013, 834, 756 cm⁻¹.
Analysis: Calc for C₂₀H₂₂O₉•1.5[H₂O]: 55.43% C, 5.81% H.
Found: 55.81% C, 5.54% H.

### EXAMPLE 7

### 3-(2-(α-D-Mannopyranosyloxy)phenyl)benzyloxyacetic acid

Part A: Operating as in Part A in EXAMPLE 5, but employing 3-bromobenzylalcohol gave ethyl 3-bromobenzyloxyacetate in 40% yield.

Part B: Operating as in Part B in EXAMPLE 2, but employing ethyl 3-bromobenzyloxyacetate gave ethyl 3-(2-hydroxyphenyl)benzyloxyacetate in 34% yield.

Part C: Operating as in Part D in EXAMPLE 1, but employing ethyl 3-(2-hydroxyphenyl)benzyloxyacetate gave ethyl 3-(2-(2,3,4,6-tetra-O-acetyl-α-D-mannopyranosyloxy)phenyl)benzyloxyacetate in 74% yield.

Part D: Operating as in Part E in EXAMPLE 1, but employing ethyl 3-(2-(2,3,4,6-tetra-O-acetyl-α-D-mannopyranosyloxy)phenyl)benzyloxyacetate gave 3-(2-(α-D-mannopyranosyloxy)phenyl)benzyloxyacetic acid in 30% yield, m.p. 77-78°C.
¹H NMR: (300MHz, DMSO-d6) 7.20-7.51 (comp, 7H), 7.15 (mult, 1H), 5.32 (s, 1H), 4.11 (s, 2H), 3.25-3.75 (comp, 6H), 3.47 (s, 2H) ppm.
IR (KBr): 3417, 2938, 1787, 1734, 1220, 1172, 1112, 1013, 977, 754 cm⁻¹.
MS (FAB) : 443.2 (m + Na)⁺
Analysis: Calc for C₂₁H₂₉O₉•[C₂HF₃O₂]•0.5[H₂O]: 50.83% C, 4.82% H.
Found: 50.59% C, 4.74% H.

### EXAMPLE 8

### N-(4-(2-(α-D-Mannopyranosyloxy)phenyl)benzoyl)glycine

Part A: Acetone (5.6ml) and dimethoxypropane (5.6 ml) were added to 4-(2-(α-D-mannopyranosyloxy)phenyl)benzoic acid (0.54 g, 1.43 mmol) to form a heterogeneous mixture. A catalytic amount of p-toluenesulfonic acid monohydrate was introduced and the reaction was stirred at room temperature for 45 minutes, at which point a clear, homogeneous solution was obtained. The solvent was removed in vacuo and the yellow oily residue taken up in ethyl acetate, washed with saturated sodium bicarbonate then saturated sodium chloride, dried (MgSO₄) and concentrated in vacuo to afford 4-(2-(2,3:4,6-di-O-isopropylidene)α-D-manno pyranosyloxy)phenyl)benzoic acid (0.70g).

Part B: A solution of crude 4-(2-(2,3:4,6-di-O-isopropylidene)α-D-mannopyranosyloxy)phenyl)benzoic acid (0.70 g) in dry dichloromethane (4 ml) was added to a slurry of glycine ethyl ester hydrochloride (0.20 g, 1.44 mmol) and triethylamine (0.40 ml, 2.88 mmol) in dry dichloromethane (3 ml). N-Hydroxysuccinimide 0.16 g, 1.44 mmol) and N,N'-dicyclohexylcarbodiimide (0.32 g, 1.55 mmol) were added and the reaction mixture stirred under nitrogen at room temperature for 4 hours. Precipitated dicyclohexylurea was filtered away and the filtrate diluted with dichloromethane. The resulting solution was washed successively with water, 1 N HCl, saturated sodium bicarbonate and brine, then dried (MgSO₄) and concentrated under reduced pressure to afford 0.69g (89% for two steps) of N-(4-(2-(2,3:4,6-di-O-isopropylidene)α-D-mannopyranosyloxy)phenyl)benzoyl)glycine ethyl ester.

Part C: N-(4-(2-(2,3:4,6-di-O-isopropylidene)
α-D-mannopyranosyloxy)phenyl)benzoyl) glycine ethyl ester (0.69 g, 1.28 mmol) was dissolved in tetrahydrofuran (2.5 ml). An equal volume of 1 N HCl was added and the reaction mixture stirred overnight. 2 N sodium hydroxide (2 ml) was added and the reaction stirred for another 8 hours. The solution was then re-acidified to pH 4.5 with 1 N HCl and the product isolated by preparative reverse-phase HPLC on a Dynamax 300 Å 5 micron (21 mm ID x 25 cm) C₁₈ column. A gradient of 5-50% solvent B was run over 20 minutes at a flow rate of 10ml/min, where solvent A was composed of 5% acetonitrile/water with 0.1% TFA and solvent B was composed of 95% acetonitrile/water with 0.1% TFA. The effluent was monitored at 254 nm. Pure fractions were combined and lyophilized to yield 0.33g of N-(4-(2-(α-D-mannopyranosyloxy)phenyl)benzoyl)glycine.
¹H NMR: (300MHz, D₂O) 7.85 (d, J=7.8, 2H), 7.61 (d, J=7.8, 2H), 7.41 (m, 2H), 7.32 (d, J=9.0, 1H), 7.20 (t, J=6.9, 7.8, 1H), 5.48 (s, 1H), 4.11 (s, 2H) 3.94 (s, 1H), 3.60 (br m, 4H), 3.28 (br m, 1H).
IR (KBr): 3404, 2938, 1734, 1637, 1544, 1220, 1107, 1066 cm⁻¹
m.p.: 127-129°C
Analysis: Calc for C₂₁H₂₃NO₉•1/5[CF₃CO₂H]; 56.34% C, 5.13% H, 3.07% N.
Found: 56.36% C, 4.93% H, 2.98% N.

### EXAMPLE 9

### N-(4-(2-(α-D-Mannopyranosyloxy)phenyl)benzoyl)-D-phenylalanine

Part A: 4-(2-(2,3:4,6-Di-O-isopropylidene-a-D-mannopyranosyloxy)phenyl)benzoic acid (0.25g, 0.55 mmol) and D-phenylalanine methyl ester hydrochloride (0.13g, 0.60 mmol) were slurried under dry dichloromethane (2 ml). N-methylmorpholine (0.13 ml, 1.18 mmol), hydroxybenzatriazole hydrate (74 mg, 0.55 mmol) and 1-(3-dimethylaminopropyl)-3-ethylcarbodiimide hydrochloride (0.26 g, 1.35 mmol) were added and the reaction was stirred at room temperature for two hours. Ethyl acetate was added and the solution was washed with water, 1 N HCl, saturated sodium bicarbonate solution, brine, dried (MgSO₄) and concentrated *in vacuo*. The residue was purified by silica gel chromatography (2:1 hexane:ethyl acetate) to afford N-(4-(2-(2,3:4,6-di-O-isopropylidene-α-D-mannopyranosyloxy)phenyl)benzoyl)-D-phenylalanine methyl ester (0.26 g, 76%) as a white foam.

Part B: Operating in a manner analogous to Part C of EXAMPLE 8, but employing an HPLC gradient of 20-80% solvent B in 20 minutes gave N-(4-(2-(α-D-mannopyranosyloxy)phenyl)benzoyl)-D-phenylalanine in 46% yield; m.p. = 116-119°C; IR (KBr): 3424, 2972, 1738, 1642, 1539, 1361, 1215, 1109, 1070, 1013 cm⁻¹; ¹H NMR (300 MHz, CD₃OD): ∂ 7.77 (d, 2H, J = 8.4), 7.54 (d, 2H, J = 8.4), 7.29 (m, 9H), 7.10 (t, 1H), 5.41 (s, 1H), 3.80 (br s, 1H), 3.67 (m, 4H), 3.49 (br m, 1H), 3.35 (m, 1H), 3.12 ppm (dd, 2H, J = 13.8, 9.6); MS (CI): *m*/*z* = 524, 362, 163; analysis: calc. for C₂₈H₂₉NO₉, 64.2% C, 5.6% H, 2.7% N; found: 64.2% C, 5.6% H, 2.4% N.

### EXAMPLE 10

### 3-(2-(6-Azido-6-deoxy-α-D-mannopyranosyloxy)phenyl) phenylacetic acid

Part A: Methyl 3-(2-(2,3,4,6-tetra-O-acetyl-α-D-mannopyranosyloxy)phenyl)phenylacetate (3.96 g, 6.9 mmol, from Part E, EXAMPLE 2) was dissolved in methanol (50 ml), sodium methoxide (100 mg) was added and the solution stirred at room temperature for two hours. The reaction mixture was neutralized with Dowex-50W ion exchange resin (H⁺ form), filtered and concentrated *in vacuo*. Chromatography (silica, 9:1 CHCl₃:methanol) gave methyl 3-(2-(α-D-mannopyranosyloxy)phenyl)phenylacetate (2.8 g, quantitative yield (≈100%)).

Part B: Methyl 3-(2-(α-D-mannopyranosyloxy)phenyl)phenylacetate (1.86 g, 4.6 mmol) was dissolved in 2,2-dimethoxypropane (30 ml) and acetone (30 ml). *p*-Toluenesulfonic acid (100 mg) was added and the solution stirred at room temperature overnight. The reaction mixture was poured into saturated sodium bicarbonate and the product extracted with ethyl acetate. The combined extracts were dried (MgSO₄) and concentrated *in vacuo*. Purification by chromatography (silica, eluent 6:1 hexane:ethyl acetate) gave methyl 3-(2-(2,3:4,6-di-O-isopropylidene-α-D-mannopyranosyloxy)phenyl) phenylacetate (1.87 g, 84%).

Part C: Methyl 3-(2-(2,3:4,6-di-O-isopropylidene-α-D-mannopyranosyloxy)phenyl)phenylacetate (1.53 g, 3.2 mmol) was dissolved in methanol (50 ml), *p*-toluenesulfonic acid (100 mg) added and stirred at room temperature until t.l.c. (eluent 9:1 CHCl₃:methanol) showed optimum conversion to the monoacetonide. The reaction was quenched by the addition of a small volume of saturated sodium bicarbonate and then concentrated under reduced pressure. The residue was partitioned between ethyl acetate and water and the aqueous layer extracted with ethyl acetate. The combined organic layers were washed once with saturated sodium chloride, dried over magnesium sulfate and concentrated under reduced pressure. Chromatography (silica, eluent 9:1 CHCl₃:methanol) gave methyl 3-(2-(2,3-O-isopropylidene-α-D-mannopyranosyloxy)phenyl)phenylacetate (0.92 g, 55%).

Part D: Methyl 3-(2-(2,3-O-isopropylidene-α-D-mannopyranosyloxy)phenyl)phenylacetate (6.37 g, 14.3 mmol) was dissolved in pyridine (100 ml) and the solution cooled to 0°C. *p*-Toluenesulfonyl chloride (5.5 g, 28.9 mmol) was added followed by 4-dimethylaminopyridine (100 mg) and the solution stirred at room temperature overnight. After cooling to 0°C, acetic anhydride (5 ml, 53 mmol) was added and the solution stirred at room temperature for 24 hours. The reaction mixture was diluted with ethyl acetate and washed with dilute hydrochloric acid to remove the pyridine. The organic layer was then washed with dilute sodium bicarbonate and saturated sodium chloride, dried (MgSO₄) and concentrated *in vacuo.* This gave methyl 3-(2-(4-O-acetyl-2,3-O-isopropylidene-6-O-*p*-toluenesulfonyl-α-D-mannopyranosyloxy)phenyl)phenylacetate (9.19 g, quantitative yield ( 100%)).

Part E: Methyl 3-(2-(4-O-acetyl-2,3-O-isopropylidene-6-O-p-toluenesulfonyl-α-D-mannopyranosyloxy)phenyl)phenylacetate (9.19 g, 14.4 mmol) was dissolved in dimethylformamide (100 ml), sodium iodide (4.3 g, 29 mmol) was added and the mixture heated at 110°C for 6 hours. After cooling to room temperature most of the DMF was removed *in vacuo* and the residue partitioned between ethyl acetate and water. The organic layer was washed with water, dilute sodium thiosulfate solution, water, saturated sodium chloride, dried (MgSO₄) and concentrated *in vacuo*. Chromatography (silica, eluent 2:1 hexane:ethyl acetate) gave methyl 3-(2-(4-O-acetyl-6-iodo-6-deoxy-2,3-O-isopropylidene-α-D-mannopyranosyloxy)phenyl)phenylacetate (6.42 g, 75%).

Part F: Methyl 3-(2-(4-O-acetyl-6-azido-6-deoxy-2,3-O-isopropylidene-α-D-mannopyranosyloxy)phenyl)phenylacetate was prepared from methyl 3-(2-(4-O-acetyl-6-iodo-6-deoxy-2,3-O-isopropylidene-α-D-mannopyranosyloxy)phenyl)phenylacetate and sodium azide in 93% yield using an analogous procedure to Part E, of this Example, with sodium azide being used in place of sodium iodide.

Part G: Methyl 3-(2-(4-O-acetyl-6-azido-6-deoxy-2,3-O-isopropylidene-α-D-mannopyranosyloxy)phenyl)phenylacetate (0.208 g, 0.41 mmol) was dissolved in methanol (6 ml) and water (2 ml). Concentrated hydrochloric acid (3 drops) was added and the solution stirred at room temperature for two days. The solution was made basic with dilute sodium hydroxide solution and stirred at room temperature for one hour. After neutralizing with dilute hydrochloric acid the solution was concentrated *in vacuo.* The residue was shaken with methanol and the white solid removed by centrifugation. The solution was concentrated under reduced pressure and the residue taken up in 5% acetonitrile/water with 0.1% in trifluoroacetic acid. After adjusting the pH to 3.5 using dilute hydrochloric acid the product was purified by preparative reverse-phase HPLC on a Dynamax 300Å 5 micron C18 column (21.4 x 250 mm) at a flow rate of 10 ml/min. An elution gradient of 20-80% solvent B over 30 minutes was used, with solvent B composed of 95% acetonitrile/water with 0.1% trifluoroacetic acid and solvent A composed of 5% acetonitrile/water with 0.1% trifluoroacetic acid. The effluent was monitored at 254 nm, and pure fractions were combined and lyophilized to yield methyl 3-(2-(6-azido-6-deoxy-a-D-mannopyranosyloxy)phenyl)phenylacetic acid, (44 mg, 26%); ¹H NMR (300 MHz, D₂O / DMSO-d₆) 3.3-3.4 (m, 3 H, OH), 3.55 (dd, *J*= 9.6, 8.4, 1 H, CH₂N₃), 3.62 (dd, *J* = 9.6, 2.7, 1 H, CH₂N₃), 3.73 (s, 2 H, CH₂CO₂H), 3.96 (m, 1 H), 4.6-5.0 (m , 3 H), 5.46 (s, 1 H), 7.14-7.50 (m, 8 H, arom.). IR (KBr: cm⁻¹): 3421, 2101, 1717; Mass Spectrum m/e (CI: CH₄) 229, 183 (100%). Anal. Calcd. for C₂₀H₂₁N₃O₇•0.3 [CF₃CO₂H]: C, 55.9; H, 4.9 N, 9.6; Found: C, 56.1; H, 4.6; N, 9.5%.

### ExAMPLE 11

### 3-(2-(6-Amino-6-deoxy-α-D-mannopyranosyloxy)phenyl)phenylacetic acid, hydrochloride

Part A: Methyl 3-(2-(4-O-acetyl-6-azido-6-deoxy-2,3-O-isopropylidene-α-D-mannopyranosyloxy)phenyl)phenylacetate (1.26 g, 2.5 mmol, from part F, EXAMPLE 10) was stirred with sodium methoxide (100 mg) in methanol (15 ml) at room temperature overnight. The solution was neutralized with Dowex-50W ion exchange resin (H⁺ form), filtered and concentrated *in vacuo*. Chromatography (silica, eluent 3:1 hexane:ethyl acetate) gave methyl 3-(2-(6-azido-6-deoxy-2,3-O-isopropylidene-α-D-mannopyranosyloxy)phenyl)phenylacetate (1.02 g, 88%).

Part B: Methyl 3-(2-(6-azido-6-deoxy-2,3-O-isopropylidene-α-D-mannopyranosyloxy)phenyl)phenylacetate (0.79 g, 1.7 mmol) was dissolved in methanol (20 ml), Raney nickel (0.56 g) was added and the mixture stirred at room temperature for two hours. The mixture was filtered and concentrated *in vacuo*. Chromatography (silica, eluent 9:1 CHCl₃ /methanol) gave methyl 3-(2-(6-amino-6-deoxy-2,3-O-isopropylidene-α-D-mannopyranosyloxy)phenyl)phenylacetate 0.52 g (69%).

Part C: Methyl 3-(2-(6-amino-6-deoxy-2,3-O-isopropylidene-α-D-mannopyranosyloxy)phenyl)phenylacetate (77 mg, 0.17 mmol) was dissolved in methanol (5 ml), *p*-toluenesulfonic acid (40 mg, 0.21 mmol) was added and the solution stirred at room temperature overnight. Aqueous sodium hydroxide (2 M, 0.4 ml) was added and the solution stirred for 5 minutes before acidifying with dilute hydrochloric acid. The product was purified by preparative reverse-phase HPLC on a Dynamax 300Å 5 micron C18 column (21.4 x 250 mm) at a flow rate of 10 ml/min. An elution gradient of 10-40% solvent B over 25 minutes was used, with solvent B composed of 95% acetonitrile/water with 0.1% trifluoroacetic acid and solvent A composed of 5% acetonitrile/water with 0.1% trifluoroacetic acid. The effluent was monitored at 254 nm, and pure fractions were combined and concentrated *in vacuo*. Water (5 ml) and dilute hydrochloric acid (0.2 ml) were added and the mixture re-concentrated. This procedure was repeated once more with hydrochloric acid and once with water. The residue was then dissolved in water (5 ml) and lyophilized to yield 3-(2-(6-amino-6-deoxy-α-D-mannopyranosyloxy)phenyl)phenylacetic acid, hydrochloride (50.3 mg, 74%).

### EXAMPLE 12

### N-(3-(2-(α-D-Mannopyranosyloxy)phenyl)benzoyl)-L-glutamic acid

Part A: 3-(2-(2,3:4,6-Di-O-isopropylidene-α-D-mannopyranosyloxy)phenyl)benzoic acid can be prepared in a manner analogous to Part A of EXAMPLE 8.

Part B: Operating in a manner analogous to Part A of EXAMPLE 9, but employing L-glutamic acid dimethyl ester hydrochloride and 3-(2-(2,3:4,6-di-O-isopropylidene-α-D-mannopyranosyloxy)phenyl)benzoic acid gave N-(3-(2-(2,3:4,6-di-O-isopropylidene-α-D-mannopyranosyloxy)phenyl)benzoyl)-L-glutamic acid dimethyl ester in 89% yield.

Part C: Operating in a manner analogous to Part C of EXAMPLE 8, but employing an HPLC gradient of 10-60% solvent B in 20 minutes gave N-(3-(2-(a-D-mannopyranosyloxy)phenyl)benzoyl)-L-glutamic acid in 19% yield; m.p. = 109-112°C; IR (KBr): 3390, 2938, 1717, 1635, 1539, 1416, 1217, 1100, 1059, 1011 cm⁻¹; ¹H NMR (300 MHz, D₂O): ∂ 7.82 (s, 1H), 7.73 (d, 1H, J = 7.8), 7.65 (d, 1H, J = 7.5), 7.53 (t, 1H J = 7.5), 7.37 (d, 2H, J = 7.8), 7.29 (d, 1H, J = 8.1), 7.17 (t, 1H, J = 7.2), 5.45 (s, 1H), 4.58 (q, 1H, J = 4.8), 3.92 (s, 1H), 3.57 (m, 6H), 3.19 (br m, 1H), 2.52 (t, 2H, J = 7.2), 2.27 (m, 1H), 2.11 ppm (m, 1H); Mass Spectrum m/e (CI:CH₄) 344, 163; analysis: calc. for C₂₄H₂₇NO₁₁•1/3[C₂F₃HO₂], 54.5% C, 5.1% H, 2.6% N; found: 54.4% C, 4.8% H, 2.6% N.

### EXAMPLE 13

### 3-(2-(6-(Carboxymethylthio)-6-deoxy-α-D-mannopyranosyloxy)phenyl)phenylacetic acid

Part A: Sodium hydride (60% wt. disp., 0.14 g, 3.4 mmol) was washed with hexane, tetrahydrofuran (5 ml) was added and the mixture degassed. Methyl thioglycolate (0.4 ml, 4.5 mmol) was added, followed by a solution of methyl 3-(2-(4-O-acetyl-6-deoxy-6-iodo-2,3-O-isopropylidene-α-D-mannopyranosyloxy)phenyl)phenylacetate (0.83 g, 1.4 mmol, Part E, EXAMPLE 10) in THF (10 ml). The solution was degassed again and then stirred at room temperature for 72 hours. The reaction was quenched with water then partitioned between ethyl acetate and water. The organic layer was washed with dilute sodium thiosulphate, water and saturated sodium chloride. After drying (MgSO₄), the solution was concentrated *in vacuo.* Chromatography (silica, eluent 2:1 hexane:ethyl acetate) gave methyl 3-(2-(4-O-acetyl-6-(carbomethoxymethylthio)-5-deoxy-2,3-O-isopropylidene)-α-D-mannopyranosyloxy)phenyl)phenylacetate (0.7 g, 87%).

Part B: Methyl 3-(2-(4-O-acetyl-6-(carbomethoxymethylthio)-6-deoxy-2,3-O-isopropylidene-α-D-mannopyranosyloxy)phenyl)phenylacetate (0.56 g, 1 mmol) was dissolved in methanol (30 ml), dilute hydrochloric acid (2 ml) was added and stirred at room temperature overnight, then warmed to reflux for 30 minutes. After cooling to room temperature, sodium hydroxide (2 M, 5 ml) was added and the solution stirred for 10 minutes. The solution was neutilized using dilute hydrochloric acid and then concentrated in vacuo. The residue was shaken with methanol and the white solid removed by centrifugation. The solution was concentrated *in vacuo* and the residue taken up in 5% acetonitrile/water with 0.1% trifluoroacetic acid. After adjusting the pH to 3.5 using dilute hydrochloric acid the product was purified by preparative reverse-phase HPLC on a Dynamax 300Å 5 micron C18 column (21.4 x 250 mm) at a flow rate of 10 ml/min. An elution gradient of 20-80% solvent B over 20 minutes was used, with solvent B composed of 95% acetonitrile/water with 0.1% trifluoroacetic acid and solvent A composed of 5% acetonitrile/water with 0.1% trifluoroacetic acid. The effluent was monitored at 254 nm, and pure fractions were combined and lyophilized to yield 3-(2-(6-(carboxymethylthio)-6-deoxy-α-D-mannopyranosyloxy)phenyl)phenylacetic acid (0.40g, 83%); ¹H NMR (300 MHz, D₂O): δ 2.60 (dd, *J* = 14.1, 8.4, 1 H, 6-H), 2.84 (dd, *J* = 14.1, 1.8, 1 H, 6'-H), 3.04 and 3.30 (both d, *J* = 14.4, 1 H, SCH₂CO₂H), 3.3-3.6 (m, 3 H), 3.70 (s, 2 H, CH₂CO₂H), 3.94 (m, 1 H), 4.6-5.0 (m , 3 H), 5.48 (s, 1 H, 1-H), 7.14-7.50 (m, 8 H, arom.); IR (KBr: cm⁻¹): 3396, 1710; Mass spectrum *m*/*e* (CI: CH₄) 465 (1%), 393, 229, 183 (100%). Anal. calcd for C₂₂H₂₄O₉S•0.8 [H₂O]: C, 55.2; H, 5.4. Found: C, 55.25; H, 5.25%.

### EXAMPLE 14

### 2-(3-(2-(α-D-Mannopyranosyloxy)phenyl)phenyl)ethanesulfonic acid

Part A: Methyl 3-(2-(2,3:4,6-di-O-isopropylidene-α-D-mannopyranosyloxy)phenyl)phenylacetate (1.28 g, 2.6 mmol, from Part B, EXAMPLE 10) was dissolved in ether (50 ml) and cooled to 0°C. Lithium aluminum hydride (1 M in THF, 50 ml, 5 mmol) was added dropwise and quenched by careful addition of water, followed by ice cold dilute sulfuric acid. The organic layer was washed with water, then saturated sodium bicarbonate solution, dried (MgSO₄) and concentrated *in vacuo.* to give 2-(3-(2-(2,3:4,6-di-O-isopropylidene-α-D-mannopyranosyloxy)phenyl)phenyl)ethanol (1.13 g, 94%).

Part B: 2-(3-(2-(2,3:4,6-Di-O-isopropylidene-α-D-mannopyranosyloxy)phenyl)phenyl)ethanol (0.73 g, 1.6 mmol) was dissolved in dichloromethane (50 ml) and cooled to 0°C. Triethylamine (0.33 ml, 2.4 mmol) was added, followed by methanesulfonyl chloride (0.15 ml, 1.9 mmol). After five minutes at 0°C the reaction mixture was diluted with dichloromethane and washed with dilute hydrochloric acid, water and saturated sodium bicarbonate solution, dried (MgSO₄) and concentrated *in vacuo* to give 2-(3-(2-(2,3:4,6-di-O-isopropylidene-α-D-mannopyranosyloxy)phenyl)phenyl)ethanol 1-O-methanesulfonate (0.77 g, 90%).

Part C: 2-(3-(2-(2,3:4,6-Di-O-isopropylidene-a-D-mannopyranosyloxy)phenyl)phenyl)ethanol 1-O-methanesulfonate (0.22 g, 0.4 mmol) was dissolved in ethanol (5 ml), potassium thioacetate (0.1 g, 0.88 mmol) added and the mixture heated at 80 °C for 30 minutes. After cooling to room temperature the reaction mixture was partitioned between ethyl acetate and water. The organic layer was washed twice with water, dried (MgSO₄) and concentrated *in vacuo*. Chromatography (silica, eluent 2:1 hexane:ethyl acetate) gave 2-mercapto-(3-(2-(2,3:4,6-di-O-isopropylidene-α-D-mannopyranosyloxy)phenyl)phenyl)ethane S-acetate (0.17 g, 80%).

Part D: 2-Mercapto-(3-(2-(2,3:4,6-di-O-isopropylidene-a-D-mannopyranosyloxy)phenyl)phenyl) ethane S-acetate (0.15g, 0.29 mmol) was dissolved in methanol (1 ml). A solution of Oxone (approx. 1 meq/ml in methanol/water, 1.5 ml) was added dropwise at room temperature over the course of 90 minutes. After stirring for 7 days another portion of Oxone solution (1 ml) was added over the course of 60 minutes and stirring was continued for another 3 days. The product was isolated by preparative reverse-phase HPLC on a Dynamax 300Å 5 micron C18 column (21 x 250 mm). A gradient of 0-70% solvent B was run over 20 minutes at a flow rate of 10 ml/min, where solvent B was composed of 95% acetonitrile/water with 0.1% trifluoroacetic acid and solvent A was composed of 5% acetonitrile/water with 0.1% trifluoroacetic acid. The effluent was monitored at 254 nm, and pure fractions were combined and lyophilized to give 2-(3-(2-α-D-mannopyranosyloxyphenyl)phenyl)ethanesulfonic acid (50.8 mg, 38%); ¹H NMR (300 MHz, D₂O): δ 7.36 (m, 8H), 7.17 (t, 1H, J = 7.5), 5.44 (s, 1H), 3.93 (s, 1H), 3.61 (m, 1H), 3.57 (m, 4H), 3.25 (br m, 1H), 3.10 ppm (m, 4H); Mass Spectrum m/e (CI:CH₄) 279, 163; anal. calcd for C₂₀H₂₄SO_{9•}2(H₂O], 50.4% C, 5.9% H; found: 50.4% C, 6.0% H.

### EXAMPLE 15

### N-(4-(2-(α-D-Mannopyranosyloxy)phenyl)benzoyl)-L-glutamic acid

Part A: Operating in a manner analogous to Part A of EXAMPLE 9, but employing L-glutamic acid dimethyl ester hydrochloride gave N-(4-(2-(2,3:4,6-di-O-isopropylidene)-α-D-mannopyranosyloxyphenyl)benzoyl)-L-glutamic acid dimethyl ester in 89% yield.

Part B: Operating in a manner analogous to Part C of EXAMPLE 8, but employing an HPLC gradient of 10-60% solvent B in 20 minutes gave N-(4-(2-(α-D-mannopyranosyloxy)phenyl)benzoyl)-L-glutamic acid in 43% yield; m.p. = 118-121°C; IR (KBr): 3390, 2938, 1717, 1635, 1539, 1477, 1217, 1107, 1059, 1011 cm⁻¹; ¹H NMR (300 MHz, CD₃OD): δ 7.80 (d, 2H, J = 8.7), 7.55 (d, 2H, J = 8.4), 7.35 (m, 2H), 7.30 (d, 1H, J = 8.1), 7.18 (t, 1H, J = 7.5), 5.46 (s, 1H), 4.58 (dd, 1H, J = 9.9, 5.7), 3.93 (s, 1H), 3.60 (m, 5H), 3.24 (br m, 1H), 2.53 (t, 2H, J = 7.2), 2.27 (m, 1H), 2.12 ppm (m, 1H); Mass Spectrum m/e (CI:CH₄) 344, 163; analysis: calc. for C₂₄H₂₇NO₁₁6,34•1/4[C₂F₃HO₂], 54.0% C, 5.1% H, 2.6% N; found: 53.7% C, 5.1% H, 2.4% N.

### EXAMPLE 16

### N-(4-(2-(α-D-Mannopyranosyloxy)phenyl)benzoyl)-β-alanine

Part A: Operating in a manner analogous to Part A of EXAMPLE 9, but employing β-alanine ethyl ester hydrochloride gave N-(4-(2-(2,3:4,6-di-O-isopropylidene)-α-D-mannopyranosyloxyphenyl)benzoyl)-β-alanine ethyl ester in 58% yield.

Part B: Operating in a manner analogous to Part C of EXAMPLE 8, but employing an HPLC gradient of 0-50% solvent B in 20 minutes gave N-(4-(2-(α-D-mannopyranosyloxy)phenyl)benzoyl)-β-alanine in 53% yield; m.p. = 101-104°C; IR (KBr): 3397, 2938, 1717, 1635, 1539, 1484, 1217, 1107, 1066, 1011 cm⁻¹; ¹H NMR (300 MHz, D₂O) : δ 7.75 (d, 2H, J = 8.1), 7.54 (d, 2H, J = 7.8), 7.35 (m, 3H), 7.19 (t, 1H, J = 7.5), 5.47 (s, 1H), 3.93 (s, 1H), 3.64 (m, 7H), 3.26 (br m, 1H), 2.69 ppm (t, 2H, J = 6.4); Mass Spectrum m/e (CI:CH₄) 448, 286, 163; analysis: calc. for C₂₂H₂₅NO₉•1/3[C₂F₃HO₂], 56.1% C, 5.3% H, 2.9% N; found: 56.1% C, 5.1% H, 3.0% N.

### EXAMPLE 17

### 3-(3-(α-D-Mannopyranosyloxymethyl)phenyl)phenylacetic acid

Part A: 3-Bromobenzyl alcohol (2.0 g, 10.7 mmol) was dissolved in dry THF (50 ml) in a dry 100 ml flask flushed with nitrogen. The mixture was chilled in a dry ice/acetone bath. n-Butyl lithium (11 ml of a 2.13 M solution in hexane, 23.5 mmol) was added. The reaction was warmed to room temperature for 1 hour, then cooled in an ice water bath. Trimethyl borate (1.3 ml, 11.2 mmol) was added and the mixture was stirred at room temperature overnight, then treated with 2 N aqueous HCl to pH 2, and stirred for 3 hours. Brine (15 ml) was added, and the mixture was extracted with ethyl acetate (3 x 15 ml). The organic materials were combined, dried (MgSO₄) then concentrated under reduced pressure which gave of 3-hydroxymethylbenzeneboronic acid (98%) as a clear oil.

3-Hydroxymethylbenzeneboronic acid (1.8 g, 11.8 mmol), 3-bromophenylacetic acid (2.55 g, 11.8 mmol), tribasic potassium phosphate, (7.54 g, 35.5 mmol), DMF (55ml), and water (20 ml) were degassed under nitrogen in a 250 ml flask fitted with a reflux condenser. Bis[triphenylphosphine]palladium(II) chloride (0.17 g, 0.24 mmol) was added. The mixture was degassed under nitrogen, and heated at 90°C overnight, then acidified with 2 N HCl, mixed with brine (15 ml), and extracted with methylene chloride (3 x 15 ml). The organic materials were combined, dried (MgSO₄), then concentrated under reduced pressure which gave 3.0 g of 3-(3-hydroxymethylphenyl)phenyl acetic acid.

3-(3-Hydroxymethylphenyl)phenyl acetic acid (3.0 g, 12.4 mmol), methanol (50 ml) and concentrated sulfuric acid (10 drops) were heated at reflux overnight in a 100 ml flask, quenched with saturated sodium bicarbonate solution, diluted with water (10 ml), extracted with methylene chloride (3 x 15 ml), washed with brine (1 x 15 ml), dried (MgSO₄), and concentrated under reduced pressure. The residue was purified by flash chromatography (SiO₂, 5:1/hexane:ethyl acetate) which gave 0.90 g (30% from 3-bromophenyl acetic acid) of methyl 3-(3-hydroxymethylphenyl)phenyl acetate.

Part B: Methyl 3-(3-hydroxymethylphenyl)phenyl acetate (0.87 g, 3.4 mmol) was dissolved in 1,2-dichloroethane (17 ml) in a dry 50 ml flask. D-Mannose pentaacetate (1.66 g, 4.24 mmol) was added in one portion, then borontrifluoride etherate (1.46 ml, 11.9 mmol) was added slowly. The mixture was stirred under nitrogen overnight at room temperature, then mixed with H₂O (50 ml). The organic material was separated and the aqueous portion was extracted with methylene chloride (3 x 10 ml). The extracts were combined with the original organic fraction, dried (MgSO₄), then concentrated under reduced pressure. The residue was purified by flash chromatography (SiO₂, gradient elution hexane:ethyl acetate/3:1) which provided 1.50 g (75%) of methyl 3-(3-(2,3,4,6-tetra-O-acetyl-α-D-mannopyranosylmethyl)phenyl)phenyl acetate contaminated with a small amount of unreacted D-mannose pentaacetate which co-eluted with the product.

Part C: Methyl 3-(3-(2,3,4,6-tetra-O-acetyl-α-D-mannopyranosylmethyl)phenyl)phenyl acetate. (1.0 g, 1.7 mmol was dissolved in acetonitrile (10 ml) in a 50 ml flask, and treated with a solution of lithium hydroxide monohydrate (0.72 g, 17.0 mmol) in water (8 ml). The mixture was stirred at room temperature overnight then acidified to pH 3.5 with concentrated hydrochloric acid. The mixture was concentrated under reduced pressure and the residue was purified by HPLC (reverse phase C18, gradient elution 10-70% acetonitrile in water (0.1% trifluoroacetic acid), monitored at 254 nm) which gave 3-(3-(α-D-mannopyranosyloxymethyl) phenyl)phenylacetic acid (0.50 g, 73%); mp 74 -75°C; ¹H NMR: (300 MHz, DMSO-d₆) 7.20 - 7.60 (comp, 8H), 4.73 (d, 2H, J = 12), 4.71 (s, 1H), 4.50 (d, 2H, J = 12), 3.25 - 3.75 (comp,11H plus water) ppm; IR (KBr): 3394, 2934, 1715, 1366, 1220, 1130, 1060 cm⁻¹; analysis: calculated for C₂₁H₂₄O₈•0.15(C₂HF₃O₂]: 60.69 C, 5.77 H; found: 60.60 C, 5.99% H.

Part D: 3-(3-(α-D-mannopyranosyloxymethyl)phenyl)phenylacetic acid (0.05 g, 0.12 mmol), methanol (10 ml), and concentrated sulfuric acid (2 drops) were heated reflux for 1 hour in a 25 ml flask, then quenched with saturated sodium bicarbonate solution, diluted with water (5 ml), extracted with methylene chloride (3 x 5 ml), washed with brine (10 ml), dried (MgSO₄), concentrated under reduced pressure. The residue was purified by flash chromatography (SiO₂, 5:1/methylene chloride:methanol) which gave methyl 3-(3-(α-D-mannopyranosyloxymethyl)phenyl)phenylacetate (0.04 g, 77%); ¹H NMR: (300 MHz, DMSO-d₆) 7.2-7.6 (comp, 8H), 4.70 (s, 1H), 4.49-4.80 (comp, 7H), 3.75 (s, 2H), 3.62 (s, 3H), 3.30-3.50 (comp, 5H) ppm; IR: 3383, 1738, 1135, 1066 cm⁻¹.

### EXAMPLE 18

### Ethyl 3-(3-(2-α-D-mannopyranosyloxyphenyl)phenyl)propylphosphonate

Part A: A solution of 2-(3-(2-(2,3:4,6-di-0-isopropylidine)-α-D-mannopyranosyloxyphenyl)phenyl)ethanol (1.12 g, 2.46 mmol) in dichloromethane (15 ml) was added slowly to a suspension of Dess-Martin periodinane (4.95 g, 11.7 mmol) in dry dichloromethane (5 ml), and the reaction was stirred at room temperature overnight. The mixture was then diluted with ether and filtered; the filtrate was washed twice with saturated sodium bicarbonate solution and once with brine, then dried with magnesium sulfate. The solvent was removed in vacuo and the residue was purified by silica gel chromatography (4:1 hexane:ethyl acetate) to afford 2-(2-(2,3:4,6-di-O-isopropylidine)-α-D-mannopyranosyloxyphenyl)phenylethanal (0.63 g, 57%).

Part B: Tetraethylmethylenediphosphonate (0.37 g, 1.28 mmol) was dissolved in dry tetrahydrofuran (3.6 ml) under a blanket of nitrogen and the solution was cooled to -78°C. A 0.5 M solution of potassium hexamethyldisilazide in toluene (2.56 ml, 1.28 mmol) was added dropwise, and the reaction was stirred for 10 minutes. A solution of 2-(2-(2,3:4,6-di-O-isopropylidine)-α-D-mannopyranosyloxyphenyl)phenylethanal (0.58 g , 1.28 mmol) in tetrahydrofuran (3.8 ml) was then added, and the reaction was allowed to warm to room temperature as it stirred overnight. Water was then added and the mixture was extracted with ethyl acetate. The extracts were washed with water, 1 N HCl, saturated sodium bicarbonate solution, brine, then dried over magnesium sulfate. The solvent was removed in vacuo and the residue was purified by silica gel chromatography (1:2 hexane:ethyl acetate) to afford diethyl 3-(3-(2-α-D-mannopyranosyloxyphenyl)phenyl)prop-1-enylphosphonate (0.46 g, 63%).

Part C: Diethyl 3-(3-(2-(2,3:4,6-di-O-isopropylidine)-α-D-mannopyranosyloxyphenyl)phenyl)prop-1-enylphosphonate (0.46 g, 0.80 mmol) was dissolved in ethanol (25 ml) and hydrogenated (40 psi H₂, 10% Pd/C) for 3 hours. The suspension was filtered through celite and the filtrate concentrated under reduced pressure to afford diethyl 3-(3-(2-(2,3:4,6-si-O-ispeopylisine)-α-D-mannopyranosyloxyphenyl)phenyl)propylphosphonate (0.46 g, quantitative).

Part D: Diethyl 3-(3-(2-(2,3:4,6-di-O-isopropylidine-α-D-mannopyranosyloxyphenyl)phenyl)propylphosphonate (0.23 g, 0.40 mmol) was dissolved in methanol (2 ml). 2 N Hydrochloric acid (0.5 ml) was added and the reaction was stirred overnight. The solution was brought to pH 10 by the addition of 2 N NaOH and allowed to stir at room temperature for 3 hours. The reaction was heated at 60°C for 18 hours, then at 80°C for 116 hours. The solution was then cooled and acidified with 1 N HCl, and the product was isolated by preparative reverse-phase HPLC on a Dynamax 300Å 5 micron C18 column (21.4 x 250 mm) at a flow rate of 10 ml / min. An elution gradient of 0-50% solvent B over 20 minutes was used, with solvent B composed of 95% acetonitrile / water, 0.1% trifluoroacetic acid and solvent A composed of 5% acetonitrile / water, 0.1% trifluoroacetic acid. The effluent was monitored at 254 nm, and pure fractions were combined and lyophilized to yield (76.4 mg, 40%) of ethyl 3-(3-(2-α-D-mannopyranosyloxyphenyl)phenyl)propylphosphonate as a white solid, M.P.: 67-70°C; IR (KBr) : 3404, 2931, 1478, 1454, 1423, 1222, 1108, 1043, 1008, 976, 797, 752, 706 cm⁻¹; NMR (300 MHz, D₂O) : d 7.25 (m, 8 H), 5.41 (s, 1 H), 3.88 (m, 3 H), 3.61 (m, 4 H), 3.26 (m, 1 H), 2.64 (m, 2 H), 1.78 (m, 2 H), 1.66 (m, 2 H), 1.16 (t, 3 H, *J =* 6.9).

### EXAMPLE 19

### 3-(2-α-D-Mannopyranosyloxyphenyl)phenylacetonitrile

Part A: In a 50 ml flask, 5.19 g (20.77 mmol) 3-bromobenzyl bromide was dissolved in methanol (25 ml) and treated with a solution of sodium cyanide (1.29 g, 26.3 mmol) in water (5 ml) and the mixture was heated at reflux for 3 hours then concentrated under reduced pressure. The residue was dissolved in methylene chloride (20 ml) and washed with water (2 X 25 ml), saturated sodium chloride (25 ml), dried (MgSO₄) then concentrated under reduced pressure which provided 4.01 g (98%) of 3-cyanomethyl bromobenzene as a clear oil.

The crude nitrite in DME (10 ml) was added to a mixture of 2-hydroxybenzene boronic acid (2.9 g, 21 mmol), potassium phosphate tribasic (13.4 g, 63 mmol) and bis[triphenylphosphine]palladium (II) chloride (0.3 g, 0.42 mmol) in DME (90 ml) and the mixture was degassed under nitrogen, then heated at reflux for 3 hours under nitrogen. The reaction was mixed with water (150 ml) and the aqueous was brought to pH 4 with 2N HCl, then the mixture was extracted with ethyl acetate (3 X 15 mL). The organic materials were combined, dried (MgSO₄) then concentrated under reduced pressure which provided 3.8 g (89%) of 2-(3-cyanomethylphenyl)phenol. An analytical sample was obtained by recrystallization from ethyl acetate, which gave a white solid m.p.: 113-114°C.

Part B: 2-(3-Cyanomethylphenyl)phenol (0.5 g, 2.39 mmol) was dissolved in 1,2-dichloroethane (10 ml) then α-D-mannose pentaacetate (1.4 g, 3.6 mmol) and borontrifluoride etherate (1.05 ml, 8.4 mmol) were added, and the mixture was stirred at room temperature overnight then mixed with water (20 ml). The organic material was separated, washed with saturated sodium chloride (15 ml), dried (MgSO₄) then concentrated under reduced pressure. The residue was purified by flash chromatography (SiO₂, 3:1 hexane /ethyl acetate) which gave 1.38 g (105%) of the desired product contaminated with a small amount of unreacted mannose pentaacetate which coeluted with the product.

Part C: 3-(2-(2,3,4,6-Tetra-O-acetyl)-α-D-mannopyranosyloxyphenyl)phenylacetonitrile (0.82 g, 1.52 mmol) was dissolved in THF (10 ml) then a solution of lithium hydroxide monohydrate (0.5 g, 12.0 mmol) in water (2 ml) was added and the mixture was stirred at room temperature overnight then concentrated under reduced pressure. The residue was acidified to pH 4 with 2N HCl and purified by reverse-phase HPLC (C₁₈, gradient elution 0 to 50% acetonitrile in water (0.1% TFA), monitored at 254 nm) which gave 202 mg (37%) of 3-(2-α-D-mannopyranosyloxyphenyl)phenylacetonitrile as a white solid, m.p.: 70-72°C. ¹H NMR (300 MHz, DMSO-d₆): 7.10-7.50 (comp, 8H), 5.38 (s, 1H), 4.02 (s, 2H), 3.25-3.90 (camp). IR (KBr): 3424, 2934, 2258, 1477, 1425, 1220, 1109, 1009, 975, 756. MS (CI): m/e 372 (M⁺ + 1), 228, 115. Analysis: Calculated for C₁₉H₂₁NO₆•0.3 TFA: 61.00%C, 5.29%H, 3.45%N. Found: 61.03%C, 5.53%H, 3.49%N.

### EXAMPLE 20

### 2-(2'-α-D-Mannopyranosyloxyphenyl)phenoxyacetic acid

Part A: 2,2'-Dihydroxybiphenyl (1.0 g, 5.37 mmol) was dissolved in THF (5 ml) in a dry 50 ml flask flushed with nitrogen. Sodium hydride (0.24 g of a 60% suspension in mineral oil, 5.91 mmol) was added in one portion and the mixture was stirred at room temperature for 30 minutes. Ethyl bromoacetate (0.61 ml, 5.5 mmol) was added and the mixture was stirred overnight at room temperature, then warmed at reflux for 30 minutes, cooled then quenched with saturated ammonium chloride. The mixture was extracted with ether, and the organic materials were combined, dried (MgSO₄) then concentrated under reduced pressure. The residue was purified by flash chromatography (SiO₂, gradient elution: hexane to 3:1 hexane / ethyl acetate) which gave 0.89 g (61%) of ethyl 2-(2'-hydroxyphenyl)phenoxyacetate.

Part B: Ethyl 2-(2'-hydroxyphenyl)phenoxyacetate (0.75 g, 2.75 mmol) was dissolved in 1,2-dichloroethane (10 ml) then α-D-mannose pentaacetate (2.15 g, 5.51 mmol) and borontrifluoride etherate (1.73 ml, 13.8 mmol) were added, and the mixture was stirred at room temperature overnight then mixed with water (20 ml). The organic material was separated, washed with saturated sodium chloride (15 ml), dried (MgSO₄) then concentrated under reduced pressure. The residue was purified by flash chromatography (SiO₂, 3:1 hexane /ethyl acetate) which gave 1.11 g (64%) of the desired product contaminated with a small amount of unreacted mannose pentaacetate which coeluted with the product.

Part C: Ethyl-2-(2'-(2,3,4,6-tetra-O-acetyl)-α-D-mannopyranosyl- oxyphenyl)phenoxyacetate (1.1 g, 1.84 mmol) was dissolved in THF (10 ml) then a solution of lithium hydroxide monohydrate (0.53 g, 12.6 mmol) in water (3 ml) was added and the mixture was stirred at room temperature overnight then concentrated under reduced pressure. The residue was acidified to pH 4 with 2N HCl and purified by reverse-phase HPLC (C₁₈, gradient elution 0 to 50% acetonitrile in water (0.1% TFA), monitored at 254 nm) which gave 202 mg (37%) of 2-(2'-α-D-mannopyranosyloxyphenyl) phenoxyacetic acid as a white solid, m.p.: 93-96°C°C. ¹H NMR (300 MHz, DMSO-d₆): 6.85-7.35 (comp, 8H), 5.25 is, 1H), 4.60 (s, 2H), 3.25-4.20 (comp, 11H). IR (KBr): 3417, 2934, 1735, 1481, 1443, 1215, 1107, 1067, 978, 755. MS (CI): m/e 407 (M⁺ + 1), 245, 199, 115. Analysis: Calculated for C₂₀H₂₂O₉•0.3 TFA, 0.3 H₂O: 55.70%C, 5.18%H. Found: 55.80%C, 5.47%H.

### EXAMPLE 21

### 3-(2-α-D-Mannopyranosyloxyphenyl)-5-methyl-phenoxyacetic acid

Part A: 3-Bromo-4-methylphenol (1.4 g, 7.5 mmol) was dissolved in acetone (15 ml) in a dry 25 ml flask. Ethyl bromoacetate (0.96 ml, 8.66 mmol), then potassium carbonate (1.03 g, 7.5 mmol) and potassium iodide (25 mg) were added and the reaction mixture was stirred at reflux for 1 hour, then cooled and concentrated. The residue was mixed with water (15 ml) and ethyl acetate (10 ml). The organic material was separated, then dried (MgSO₄) and concentrated under reduced pressure. The residue was dissolved in hexane and filtered through silica gel and concentrated to give 2.1 g (100%) of ethyl 3-bromo-4-methylphenoxyacetate which was used without further purification.

Part B: Ethyl 3-bromo-4-methylphenoxyacetate (0.5 g, 1.83 mmol), tetrakis(triphenylphosphine)palladium (0) (17 mg), potassium phosphate (0.55 g, 2.6 mmol) and dimethylformamide (8 ml) were degassed under nitrogen in a 25 ml flask fitted with a reflux condenser. 2-Hydroxybenzene boronic acid (0.3 g, 2.0 mmol) in dimethylformamide (1 ml) was added and the mixture was heated at reflux overnight then mixed with water (25 ml) and extracted with methylene chloride (3 X 3 ml). The organic materials were separated, dried (MgSO₄) then concentrated under reduced pressure. The residue was purified by flash chromatography (SiO₂, gradient hexane to 3:1 hexane / ethyl acetate) which provided 130 mg (25%) of ethyl 3-(2-hydroxyphenyl)-4-methylphenoxy acetate.

Part C: Ethyl 3-(2-hydroxyphenyl)-4-methylphenoxy acetate (130 mg, 0.454 mmol) was dissolved in 1,2-dichloroethane (2 ml) in a dry 10 ml flask. α-D-Mannose pentaacetate (0.4 g, 0.9 mmol) was added in one portion, then borontrifluoride etherate (0.3 ml, 2.3 mmol) was added slowly. The mixture was stirred under nitrogen overnight at room temperature then mixed with water (15 ml). The organic material was separated and the aqueous portion was extracted with dichloromethane (3 X 1 ml). The extracts were combined with the original organic fraction, dried (MgSO₄) then concentrated under reduced pressure. The residue was purified by flash chromatography (SiO₂, gradient elution hexane to 3 : 1 hexane /ethyl acetate) which provided 0.42 g (>100 %) of ethyl-3-(2-(2,3,4,6-tetra-O-acetyl)-α-D-mannopyranosyloxyphenyl)-4-methylphenoxy acetate contaminated with a small amount of unreacted α-D-mannose pentaacetate which coeluted with the product.

Part D: Ethyl-3-(2-(2,3,4,6-tetra-O-acetyl)α-D-mannopyranosyl oxyphenyl)-4-methylphenoxy acetate (2.74 g, 4.78 mM) was dissolved in acetonitrile (10 ml), and treated with a solution of lithium hydroxide monohydrate (0.25 g, 5.95 mmol) in water (2 ml) and the mixture was stirred at room temperature overnight then acidified to pH 2 with concentrated hydrochloric acid. The mixture was concentrated under reduced pressure and the residue was purified by HPLC (reverse-phase, gradient elution 5-50% acetonitrile in water, monitored at 254 nm) which gave 101 mg g (53% from ethyl-3-(2-hydroxyphenyl)-4-methylphenoxy acetate) of 3-(2-α-D-mannopyranosyloxyphenyl)-4-methylphenoxy acetic acid, m.p. 87-89°C. ¹H NMR (300 MHz, DMSO-d₆): 7.32 (s, 2H), 7.05-7.18 (comp, 3H), 6.80 (comp, 1H), 6.60 (br s, 1H), 5.31 (br s, 1H), 5.11(br s, 1H), 4.9 (br s, 1H), 4.60 (s, 2H), 4.59 (br s, 1H), 3.10-3.72 (comp, 7H), 2.00 (s, 3H). IR (KBr): 3424, 2931, 1735, 1984, 1219, 1193, 1068, 1010, 976, 757. MS (CI): m/e 421 (M⁺ + 1), 259, 241, 213, 163, 145, 127, 115. Analysis: Calculated for C₂₁H₂₄O₉•0.3 TFA: 57.07%C, 5.39%H. Found: 56.90%C, 5.57%H.

### EXAMPLE 22

### 3-(2-Methoxy-5-(3-(2-α-D-mannopyranosyloxyphenyl)-2-phenylacetophenone))phenylacetic acid

Part A: Methyl 3-(2-methoxyphenyl)phenyl acetate (0.82 g, 3.2 mmol) and 3-bromophenylacetic acid chloride (0.77 g, 3.3 mmol) were dissolved in dichloroethane (11 ml) and chilled in an ice bath. Aluminum chloride (0.88 g, 6.6 mmol) was added in one portion and the mixture was warmed at 50°C for 15 minutes then mixed with ice water (20 ml). The organic materials were separated, dried (MgSO₄), then concentrated under reduced pressure which gave 1.68 g of methyl 3-(2-methoxy-5-(3-bromophenyl-2-phenylacetophenone))phenyl acetate as a yellow oil which was used without further purification. The crude halide was mixed with 2-methoxyphenylboronic acid (0.54 g, 3.55 mmol) in toluene (20 ml), then tetrakis(triphenylphosphine)palladium (0) (120 mg, 3 mol%) and aqueous sodium carbonate (6 ml of a 2N solution) were added and the mixture was degassed under nitrogen. The mixture was then heated at reflux for 14 hours then mixed with water (100 ml), and the mixture was extracted with ethyl acetate (3 X 8 ml). The organic materials were combined, washed with saturated sodium chloride (15 ml), dried (MgSO₄) then concentrated under reduced pressure. The residue was purified by flash chromatography (SiO₂, gradient elution hexane to 3:1 hexane/ethyl acetate) which gave 1.57 g (77%) of methyl 3-(2-methoxy-5-(3-(2-methoxyphenyl)-2-phenylacetophenone))phenylacetate as a clear oil.

Part B: Methyl 3-(2-methoxy-5-(3-(2-methoxyphenyl)-2-phenylacetophenone))phenylacetate (0.68 g, 1.42 mmol) was dissolved in dichloromethane (5 ml) under nitrogen, and chilled in a dry-ice / 2-propanol bath. Boron tribromide (0.8 ml, 8.5 mmol) was added slowly drop-wise and the mixture was kept at 0°C for 1 hour, then mixed with ice-water (25 ml). The organic material was separated, washed with saturated sodium bicarbonate solution (10 ml), water (10 ml), saturated sodium chloride (10 ml) then dried (MgSO₄) and concentrated under reduced pressure which gave 0.73 g of methyl 3-(2-methoxy-5-(3-(2-hydroxyphenyl)-2-phenylacetophenone))phenyl acetate as a clear oil. The crude phenol was dissolved in dichloroethane (10 ml) and α-D-mannose pentaacetate (1.8 g, 4.5 mmol) was added in one portion, then boron trifluoride etherate (1.0 ml, 7.5 mmol) was added slowly. The mixture was stirred under nitrogen overnight at room temperature then mixed with water (25 ml). The organic material was separated and the aqueous portion was extracted with dichloromethane (3 X 2 ml). The extracts were combined with the original organic fraction, dried (MgSO₄) then concentrated under reduced pressure. The residue was purified by flash chromatography (SiO₂, gradient elution hexane to 3 : 1 hexane / ethyl acetate) which provided 0.8 g (73%) of methyl 3-(2-methoxy-5-(3-(2-(2,3,4,6-tetra-O-acetyl)-α-D-mannopyranosyloxyphenyl)-2-phenylacetophenone))phenyl acetate contaminated with a small amount of unreacted α-D-mannose pentaacetate which coeluted with the product.

Part C: Methyl 3-(2-methoxy-5-(3-(2-(2,3,9,6-tetra-O-acetyl)-α-D-mannopyranosyloxy phenyl)-2-phenylacetophenone))phenyl acetate (0.8 g, 1.0 mmol) was dissolved in tetrahydrofuran (6 ml), and treated with a solution of lithium hydroxide monohydrate (0.25g, 6.0 mmol) in water (3 ml). The mixture was stirred at room temperature overnight then acidified to pH 2 with concentrated hydrochloric acid and concentrated under reduced pressure. The residue was purified by HPLC (reverse-phase, gradient elution 5-50% acetonitrile in water, monitored at 254 nm) which gave 134 mg (22%) of 3-(2-methoxy-5-(3-(2-α-D-mannopyranosyloxyphenyl)-2-phenylacetophenone))phenyl acetic acid as a white solid, m.p. 122-125°C. ¹H NMR (400 MHz, DMSO-d₆): 8.13 (dd, *J =* 8.8, 2.2 Hz, 2H), 7.95 (d, *J* = 2.2 Hz, 1H), 7.22-7.43 (Comp, 12H), 7.09 (mult, 1H), 5.34 (d, *J* = 1.5 Hz, 1H), 4.44 (dd, *J* = 19, 15.8 Hz, 1H), 4.16 (br s, 4H), 3.85, (s, 3H), 3.70 (comp, 1H), 3.62 (s, 2H), 3.34-3.55 (comp, 3H), 2.07 (s, 2H). IR (KBr): 3415, 1713, 1669, 1597, 1269, 1217, 1135, 1015. MS (CI): m/e 453 (M⁺ minus C₆H₁₁O₅).

### EXAMPLE 23

### 3-(2-α-D-Mannopyranosyloxyphenyl-5-(2-(3-(2-α-D-mannopyranosyloxyphenyl)phenyl)ethyl)phenylacetic acid

Part A: Methyl 3-(2-methoxy-5-(3-(2-methoxyphenyl)-2-phenylacetophenone))phenyl acetate (2.25 g, 4.68 mmol) was dissolved in DMSO (15 ml) and the solution was treated with aqueous potassium hydroxide (2.5 ml of a 2 N solution) then stirred at 60°C under nitrogen for an hour. The mixture was cooled to room temperature and treated with hydrazine hydrate (0.4 ml, 11.7 mmol), then heated at 60°C for an additional hour. Potassium tert-butoxide (1.31 g, 11.7 mmol) was added and the temperature was increased to 100°C. After 18 hours the mixture was cooled, mixed with water (50 ml) and acidified to pH 4 with 2N HCl. The mixture was saturated with sodium chloride and extracted with THF/ethyl acetate (1:1) and the extracts were combined, dried (MgSO₄) then concentrated under reduced pressure which gave 2.29 g of a dark oil. The crude product was dissolved in dichloromethane (25 ml) and cooled in a dry-ice / 2-propanol bath. Boron tribromide (2.4 ml, 25 mmol) was added dropwise and the mixture was stirred at -78°C for 2 hours, warmed to 0°C for 2 hours then recooled to -78°C for quench with water. The mixture was concentrated under reduced pressure and the residue was partitioned between THF and saturated sodium chloride. The organic material was separated, dried (MgSO₄) then concentrated. The residue (2.63 g) was dissolved in methanol (30 ml) and 5 drops of concentrated sulfuric acid were added and the mixture was refluxed overnight then concentrated. The residue was purified by flash chromatography (SiO₂, gradient elution hexane to 3:1 hexane/ethyl acetate) which gave 0.89 g (43%) of methyl 3-(2-hydroxyphenyl-5-(2-(3-(2-hydroxyphenyl)phenyl) ethyl))phenyl acetate as a clear oil.

Part B: Methyl 3- (2-hydroxyphenyl-5-(2-(3-(2-hydroxyphenyl) phenyl)ethyl))phenyl acetate (0.69 g, 1.57 mmol) was dissolved in dichloroethane (10 ml) and α-D-mannose pentaacetate (1.8 g, 4.5 mmol) was added in one portion, then boron trifluoride etherate (2.3 ml, 18.8 mmol) was added slowly. The mixture was stirred under nitrogen overnight at room temperature then mixed with water (50 ml). The organic material was separated and the aqueous portion was extracted with dichloromethane (3 X 2 ml). The extracts were combined with the original organic fraction, dried (MgSO₄) then concentrated under reduced pressure. The residue was purified by flash chromatography (SiO₂, gradient elution hexane to 3 : 1 hexane / ethyl acetate) which provided 1.38 g (66%) of methyl 3-(2-(2,3,4,6-tetra-O-acetyl)-α-D-mannopyranosyl oxyphenyl-5-(2-(3-(2-(2,3,4,6-tetra-O-acetyl)-α-D-mannopyranosyl oxyphenyl)phenyl)ethyl))phenyl acetate as a foam. The product was dissolved in acetonitrile (5 ml) and treated with a solution of lithium hydroxide hydrate (0.24 g, 5.6 mmol in 5 ml water) and stirred at room temperature overnight then acidified to pH 3 with concentrated HCl and concentrated under reduced pressure. The residue was purified by reverse-phase HPLC (C₁₈, gradient elution 0 to 50% acetonitrile in water (0.1% TFA), monitored at 254 nm) which gave 142 mg (18%) of 3-(2-α-D-mannopyranosyloxyphenyl-5-(2-(3-(2-α-D-mannopyranosyloxyphenyl)phenyl)ethyl))phenyl acetic acid as a white solid, m.p.: 129-134°C. ¹H NMR (400 MHz, DMSO-d₆): 7.10-7.40 (comp, 15H), 5.33 (d, *J* = 1.7 Hz, 1H), 5.27 (d, *J* = 1.7 Hz, 1H), 3.30-3.70 (comp, 14H, plus H₂O), 2.92 (2, 4H). IR (KBr): 3410, 2935, 1710, 1478, 1222, 1113, 1064, 1011, 977. MS (CI): m/e 425 (M⁺ minus C₁₂H₂₀O₁₀). Analysis: calculated for C₄₀H₄₄O₁₄• 2.2 H₂O: 60.94% C, 6.19% H. Found: 60.70% C, 5.84% H.

### EXAMPLE 24

### 2,6-Dimethyl-4-(2-α-D-mannopyranosyloxyphenyl)phenoxy acetic acid

Part A: 4-Bromo-2,6-dimethylphenol (8.0 g, 39.8 mM) was dissolved in toluene (80 ml) in a 250 ml flask. Potassium bis (trimethylsilyl) amide (80 ml, 39.8 mM) was added followed by tris (2-(2-methoxyethoxy)ethyl) amine (1.3 ml, 4.0 mM), and stirred under nitrogen for 45 minutes. Ethyl bromoacetate (5 ml, 43.8 mM) was added, and stirred at room temperature overnight. The reaction mixture was mixed with saturated ammonium chloride solution (30 ml), and extracted with ethyl acetate (3 x 50 ml). The organic materials were combined, dried (MgSO₄), and concentrated under reduced pressure. The residue was flushed through silica gel with hexane / ethyl acetate (10 : 1), and concentrated which provided 11.3 g (99%) of ethyl 4-bromo-2,6-dimethylphenoxyacetate. ¹H NMR (400MHz,CDCl₃): 7.13 (s, 2H), 4.35 (s, 2H), 4.26-4.31 (q, 2H), 2.25 (s, 6H), 1.23-1.33 (t, 3H) ppm. IR (NaCl) : 2987, 1749, 1470, 1287, 1192 cm⁻¹.

Part B: 2-Hydroxybenzene boronic acid (2.0 g, 14.5 mM), ethyl 4-bromo-2,6-dimethylphenoxyacetate (4.2 g, 14.5 mM), potassium phosphate, tribasic (7.7 g, 36.3 mM), and DME (30 ml) were degassed under nitrogen in a 100 ml flask fitted with a reflux condenser. Bis (triphenylphosphine) palladium (II) chloride (0.20 g, 0.3 mM) was added. The mixture was degassed under nitrogen, and heated at 80°C overnight, then mixed with brine (20 ml), and extracted with ethyl acetate (3 x 20 ml). The organic materials were combined, dried (MgSO₄), then concentrated under reduced pressure. The residue was purified by flash chromatography (SiO₂, 10 : 1 / hexane : ethyl acetate) which gave 1.08 g (23%) of ethyl 2,6-dimethyl-4-(2-hydroxyphenyl)phenoxyacetate. ¹H NMR (500 MHz, CDCl₃): 7.21-7.29 (comp, 6H), 4.48 (s, 2H), 4.32-4.37 (q, 2H), 2.38 (s, 6H), 1.35-1.39 (t, 3H) ppm. IR (NaCl) : 3438, 1738, 1484, 1443, 1203, 1176, 1080 cm⁻¹.

Part C: Ethyl 2,6-Dimethyl-4-(2-hydroxyphenyl)phenoxyacetate (1.08 g, 3.6 mM) was dissolved in 1,2-dichloroethan (10 ml) in a dry 50 ml flask. α-D-Mannose pentaacetate (1.75 g, 4.5 mM) was added in one portion, then borontrifluoride etherate (1.3 ml, 10.8 mM) was added slowly. The mixture was stirred under nitrogen overnight at room temperature, then mixed with water (30 ml). The organic material was separated and the aqueous portion was extracted with dichloromethane (3 x 10 ml). The extracts were combined with the original organic fraction, dried (MgSO₄), then concentrated under reduced pressure. The residue was purified by flash chromatography (SiO₂, 10 : 1 / hexane : ethyl acetate) which provided 2.4 g (95%) of ethyl 2,6-dimethyl-4-(2-(2,3,4,6-tetra-O-acetyl)-α-D-annopyranosyloxyphenyl)phenoxyacetate contaminated with a small amount of unreacted α-D-mannose pentaacetate which coeluted with the product. ¹H NMR (500 MHz, CDCl₃): 7.11-7.35 (comp, 6H), 5.42 (s, 1H), 4.47 (s, 2H), 4.29-4.34 (comp, 2H), 2.36 (s, 6H), 2.17-2.18 (comp, 6H), 1.97-2.14 (7H), 1.32-1.35 (comp, 3H) ppm. IR (NaCl) : 2972, 1752, 1471, 1436, 1375, 1210, 1176, 1142, 1080, 1032, 970 cm⁻¹.

Part D: ethyl 2,6-Dimethyl-9-(2-(2,3,4,6-tetra-O-acetyl)-α-D-mannopyranosyloxyphenyl)phenoxyacetate (2.4 g, 3.8 mM) was dissolved in acetonitrile (10 ml) in a 50 ml flask, and treated with a solution of lithium hydroxide monohydrate (1.5 g, 38.0 mM) in water (20 ml), and the mixture was stirred at room temperature overnight. Acetonitrile was evaporated under reduced pressure. The residue was acidified to pH 2 with concentrated hydrochloric acid, then purified by HPLC (reverse-phase, gradient elution 10-60% acetonitrile in water, monitored at 254 nm) which gave 0.88 g (53%) of 2,6-dimethyl-4-(2-α-D-mannopyranosyloxyphenyl)phenoxy acetic acid, m.p. 95-96°C. ¹H NMR (300 MHz, DMSO-d₆): 7.06-7.31 (comp, 6H), 5.29 (s, 1H), 4.40 (s, 2H), 3.28-3.68 (comp, 10H plus water), 2.25 (s, 6H) ppm.
IR (KBr): 3431, 2931, 1738, 1477, 1217, 1176, 1107, 1066, 1011 cm⁻¹.
Analysis: calc for C₂₂H₂₆O₉·0.27 TFA: 58.19% C, 5.69% H. Found: 58.15% C, 5.98% H.

### EXAMPLE 25

### 2,6-Dimethyl-4-(3-α-D-mannopyranosyloxymethylphenyl)phenoxyacetic acid

Part A: Operating as in Part B in EXAMPLE 21, but employing 3-hydroxymethyl benzene boronic acid gave ethyl 2,6-dimethyl-4-(3-hydroxymethylphenyl)phenoxy acetate in 30% yield.

Part B: Operating as in Part C in EXAMPLE 21, but employing 2,6-dimethyl-4-(3-hydroxymethylphenyl)phenoxyethylacetate gave ethyl 2, 6-dimethyl-4-(3-(2,3,4,6-tetra-O-acetyl)-α-D-mannopyranosyloxybenzyl)phenoxyacetate in 118% yield. The desired product was contaminated with a small amount of unreacted α-D-mannose pentaacetate which coeluted with the product.

Part C: Operating as in Part D in EXAMPLE 21, but employing ethyl 2,6-dimethyl-4-(3-(2,3,9,6-tetra-O-acetyl)-α-D-mannopyranosyloxymethylphenyl)phenoxyacetate gave 2,6-dimethyl-4-(3-α-D-mannopyranosyloxymethylphenyl)phenoxy acetic acid in 25% yield, m.p. 131-132°C. ¹H NMR (300 MHz, DMSO-d₆): 7.29-7.55 (comp, 6H), 4.50-4.76 (comp, 7H), 4.40 (s, 1H), 3.40-3.65 (comp, 7H), 2.30 (s, 6H) ppm. IR (KBr) : 3397, 2924, 1752, 1710, 1477, 1443, 1203, 1135, 1066 cm⁻¹. Analysis: calc for C₂₃H₂₈O₉·0.3 TFA: 58.73% C, 5.91% H. Found: 58.66% C, 6.22% H.

### EXAMPLE 26

### 3-(3-α-D-mannopyranosyloxybenzyl)phenoxyacetic acid

Part A: 3-Bromophenol (9.0 g, 52.0 mM) was dissolved in DMF (105 ml) in a 250 ml flask. Sodium hydride (2.3 g, 57.2 mM, 60% dispersion in mineral oil) was added and stirred under nitrogen for an hour. Ethyl bromoacetate (6.35 ml, 57.2 mM) was added, and stirred at room temperature overnight. Water (800 ml) was added slowly with stirring in an ice bath. Precipitate formed was filtered which provided 11.9 g (88%) of ethyl 3-bromophenoxyacetate.

Part B: Ethyl 3-bromophenoxyacetate (1.7 g, 6.6 mM), bis (triphenylphosphine) palladium (II) chloride (0.09g, 0.13 mM), potassium phosphate, tribasic (4.2 g, 19.7 mM) and DME (30ml) were degassed under nitrogen in a 100 ml flask fitted with a reflux condenser. 3-Hydroxymethyl benzene boronic acid (0.8 g, 5.3 mM) in DME (1 ml) was added and the mixture was heated at 80°C overnight, then mixed with brine (20 ml), and extracted with ethyl acetate (3 x 20 ml). The organic materials were combined, dried (MgSO₄), then concentrated under reduced pressure. The residue was purified by flash chromatography (SiO₂, 10 : 1 / hexane : ethyl acetate) which gave 0.28 g (19%) ethyl of ethyl 3-(3-hydroxymethylphenyl) phenoxyacetate.

Part C: Operating as in Part C in EXAMPLE 1, but employing ethyl 3-(3-hydroxymethyl phenyl) phenoxyacetate gave ethyl 3-(3-(2,3,4,6-tetra-O-acetyl)-α-D-mannopyranosyl-oxybenzyl)phenoxyacetate in 45% yield.

Part D: Operating as in Part D in EXAMPLE 1, but employing ethyl 3-(3-(2,3,4,6-tetra-O-acetyl)-α-D-mannopyranosyloxybenzyl)phenoxyacetate gave ethyl 3-(3-α-D-manno-pyranosyloxybenzyl)phenoxyacetic acid in 35% yield, m.p. 73-75°C. ¹H NMR (300 MHz, DMSO-d₆): 6.90-7.60 (comp, 8H), 4.72 (comp, 4H), 4.51 (d, 1H), 3.50-3.66 (comp, 10H plus water) ppm. IR (KBr): 3431, 2931, 1738, 1608, 1423, 1210, 1066 cm⁻¹. Analysis: calc for C₂₁H₂₄O₉·0.25 TFA: 57.52% C, 5.44% H. Found: 57.29% C, 5.58% H.

### EXAMPLE 27

### 4-(3-α-D-mannopyranosyloxybenzyl)phenoxyacetic acid

Part A: Operating as in Part A in EXAMPLE 26, but employing 4-bromophenol gave ethyl 4-bromophenoxy acetate in 100% yield.

Part B: Operating as in Part B in EXAMPLE 26, but emploing ethyl 4-bromophenoxyacetate gave ethyl 4-(3-hydroxymethyl phenyl)phenoxyacetate in 26% yield.

Part C: Operating as in Part C in EXAMPLE 21, but employing 4-(3-hydroxymethyl phenyl) phenoxyethyl acetate gave 4-(3-(2,3,4,6-tetra-O-acetyl)-α-D-mannopyranosyloxybenzyl) phenoxyacetic acid in 33% yield.

Part D: Operating as in Part D in EXAMPLE 21, but employing 4-(3-(2,3,4,6-tetra-O-acetyl)-α-D-mannopyranosyloxybenzyl)phenoxyacetic acid gave 4-(3-α-D-manno-pyranosyloxybenzyl)phenoxyacetic acid in 57% yield, m.p. 83-85°C. ¹H NMR (300 MHz, DMSO-d₆): 6.98-7.60 (comp, 8H), 4.46-4.77 (comp, 8H), 3.37-4.2 (comp, 7H). IR (KBr): 3424,2931, 1738, 1608, 1512, 1936, 1224, 1073 cm⁻¹.
Analysis: calc for C₂₁H₂₄O₉·0.2 TFA: 57.99% C, 5.50% H. Found: 57.98% C, 5.72% H.

### EXAMPLE 28

### 3-(3-α-D-mannopyranosyloxybenzyl)benzoicacid

Part A: 3-Hydroxymethyl benzene boronic acid (2.0 g, 13.2 mM), 3-bromobenzoic acid (2.1 g, 10.5 mM), potassium phosphate, tribasic (8.3 g, 39.5 mM), DMF (26 ml) and water (20 ml) were degassed under nitrogen in a 100 ml flask fitted with a reflux condenser, Bis (triphenylphosphine)palladium (II) chloride (0.18 g, 0.26 mM) was added. The mixture was degassed under nitrogen, and heated at 90°C overnight, then acidified with 2N HCl, mixed with brine (15 ml), and extracted with methylene chloride (3 x 15 ml). The organic materials were combined, dried (MgSO₄), then concentrated under reduced pressure which gave 1.57 g (52%) of 3-(3-hydroxymethyl phenyl)benzoic acid.

Part B: Operating as in Part C in EXAMPLE 21, but employing 3-(3-hydroxymethyl phenyl) benzoic acid gave 3-(3-(2,3,4,6-tetra-O-acetyl)-α-D-mannopyranosyloxybenzyl) benzoic acid in 53% yield. Part C: Operating as in Part D in EXAMPLE 21, but employing 3-(3-(2,3,4,6-tetra-O-acetyl)-α-D-mannopyranosyloxybenzyl)benzoic acid gave 3-(3-α-D-mannopyranosyl-oxybenzyl)benzoic acid in 29% yield, m.p. 90-91°C. ¹H NMR (300 MHz, DMSO-d₆): 7.38-8.18 (comp, 8H), 4.72-4.77 (comp, 2H), 4.51-4.55 (d, 1H), 3.40-3.71 (comp, 10H plus water) ppm. IR (KBr): 3424, 2931, 1704, 1409, 1244, 1128, 1059 cm⁻¹. Analysis: calc for C₂₀H₂₂O₈·0.24 TFA: 58.88% C, 5.37% H. Found: 58.85% C, 5.66% H.

### EXAMPLE 29

### 3-(2-α-D-Mannopyranosyloxy-5-methylphenyl)benzoxyacetic acid

Part A: 2-Bromo-4-methylphenol (2.60 ml, 21.51 mmol) was dissolved in anhydrous diethyl ether (20 ml) and the solution was cooled to -78°C. A solution of n-butyllithium in hexane (19.0 ml, 47.5 mmol) was added slowly and the reaction was stirred for 10 minutes at -78°C, then for 1 hour at room temperature. The flask was cooled to 0°C, then trimethyl borate (2.44 ml, 10.73 mmol) was added, followed by anhydrous THF (10 ml). The reaction was stirred at room temperature for 2 hours, then quenched carefully with water. The aqueous layer was acidified, and stirring was continued for 30 minutes. The mixture was extracted with ether and the organic phase was washed with saturated sodium chloride solution, then dried with magnesium sulfate and concentrated under reduced pressure. The resulting brown residue was triturated with hexane and filtered to yield 2-hydroxy-5-methylbenzeneboronic acid (1.35g, 8.9 mmol, 41%) as a white solid.

Part B: 2-Hydroxy-5-methylbenzeneboronic acid (1.17g, 7.70 mmol) and ethyl 3-bromophenoxyacetate (1.99g, 7.68 mmol) were dissolved in dimethoxyethane (30 ml). The solution was degassed thoroughly, then (30 ml). The solution was degassed thoroughly, then a catalytic amount of bis(triphenylphosphine) palladium(II) chloride was added. The reaction mixture was again degassed and then refluxed overnight under nitrogen. Ethyl acetate and dilute HCl were added and the organic phase was washed with water then saturated sodium chloride solution, and dried with magnesium sulfate then concentrated *in vacuo*. The resulting residue was chromatographed on silica gel (3:1 hexane:ethyl acetate) to afford ethyl 3-(2-hydroxy-5-methylphenyl) phenoxyacetate (1.25g, 4.37 mmol, 57%) as a yellow oil.

Part C: Ethyl 3-(2-hydroxy-5-methylphenyl)benzoxyacetate (0.30g, 1.05 mmol) and α-D-mannose pentaacetate (0.45g, 1.15 mmol) were dissolved in anhydrous 1,2-dichloroethane (4ml). Boron trifluoride diethyl etherate (0.52ml, 4.21 mmol) was added and the mixture was stirred at room temperature overnight. The reaction was quenched carefully with water, then extracted with dichloromethane. The organic phase was dried with sodium sulfate and concentrated *in vacuo*. The residue was purified by silica gel chromatography (2:1 hexane:ethyl acetate) to yield ethyl 3-(2-(2,3,4,6-tetraacetyl-α-D-mannopyranosyloxy) -4-methylphenyl)phenoxyacetate (0.40g, 0.65 mmol, 62%) as a colorless glass.

Part D: Ethyl 3-(2-(2,3,4,6-tetraacetyl-α-D-mannopyranosyloxy)-4-methylphenyl)phenoxyacetate (0.39g, 0.63 mmol) was dissolved in methanol (3 ml), and 2 N sodium hydroxide solution (1.70 ml) was added. The reaction was stirred for 30 minutes, then the solution was acidified with 1 N hydrochloric acid, concentrated *in vacuo*, and microfiltered. The product was isolated by preparative reverse-phase HPLC on a Dynamax 300A 5 micron C₁₈ column (21 x 250 mm). A gradient of 10-70% solvent B was run over 20 minutes at a flow rate of 10 ml/min, where solvent B was composed of 95% acetonitrile/0.1% TFA and solvent A was composed of 5% acetonitrile/0.1% TFA. The effluent was monitored at 254 nm, and pure fractions were combined and lyophilized to give 173.3 mg (60%) of 3-(2-α-D-mannopyranosyloxy-4-methylphenyl)phenoxyacetic acid as a white solid, m.p.: 83-84°C. NMR (d₆-DMSO): d 7.31 (t, 1H, *J =* 8.0), 7.21 (d, 1H, *J =* 8.1), 7.10 (m, 3H), 6.95 (s, 1H), 6.87 (d, 2H, *J =* 8.1), 5.22 (s, 1H), 4.70 (s, 2H), 3.66 (s, 1H), 3.57 (d, 1H, *J =* 11.4), 3.44 (m, 3H), 3.33 (m, 1H), 2.29 (s, 3H). IR (KBr): 3417, 2931, 1731, 1215, 1177, 1066, 1011 cm⁻¹. MS (CI): *m*/*z* = 259. Analysis: calc. for , C₂₁H₂₄O₉·1/4 CF3CO2H, 57.5% C, 5.4 % H; found: 57.7 % C, 5.5 % H.

## Claims

1. A compound having the formula: 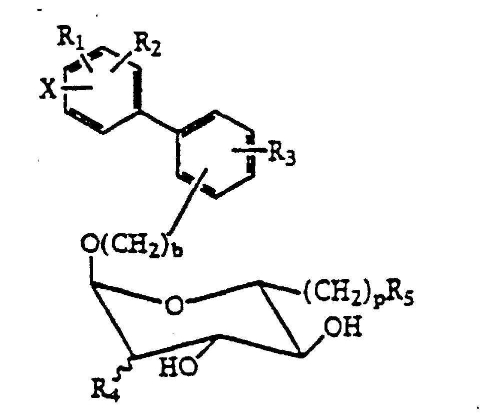 wherein X is selected from the group consisting of -(CH2)nCH3, -CN, --(CH2)n CO2 H, --O(CH2)m CO2 H, -(CH2)nCOZ, (CH2)nZ, -CHCO2H(CH2)mCO2H, --(CH2)n O(CH2)m CO2 H, --CONH(CH2)m CO2 H, --CH(OZ)(CO2 H), --CH(Z)(CO2 H), --(CH2)n SO3 H, --(CH2)n PO3 D1 D2, --NH(CH2)m CO2 H, --CONH(CHR6)CO2 H, (1-H-tetrazolyl-5-straight or branched chain C1-C12 alkyl-), and --OH;
R1 and R2 are independently selected from the group consisting of hydrogen, straight or branched chain C1-C12 alkyl , halogen, --(CH2)n CO2 H, --OZ, --NO2, --NH2, -NHZ;
R3 is selected from the group consisting of hydrogen, halogen, straight or branched chain C1-C12 alkyl--OZ and --NHZ;
R4 is selected from the group consisting of hydrogen, halogen, straight or branched chain C1-C12 alkyl, hydroxyl, hydroxyl-O-sulfate and --OZ;
R5 is selected from the group consisting of hydroxyl, --CN, --N3, --NH2, --NHNH2, --NE1 E2. --NHE1, --NHCO(CH2)n CO2 H, --S(CH2)m CO2 H and --NHCHNHNH2 ; and
R6 is selected from the group consisting of hydrogen, straight or branched chain C1-C12 alkyl. optionally substituted aryl-(straight or branched chain C1-C12 alkyl) by groups selected from straight or branched chain C1-C12 alkyl, (straight or branched chain C1-C12 alkyl)-oxy, straight or branched chain C1-C12 alkyl carboxyclic acid or mannose groups; hydroxy-(straight or branched chain C1-C12 alkyl), RₓR_{y}N- (straight or branched chain C1-C12 alkyl), straight or branched chain C1-C12 alkyl carboxylic acid and RₓR_{y}NOC-(straight or branched chain C1-C12 alkyl), Rₓ and R_{y} are independently selected from the group consisting of hydrogen, straight or branched chain C1-C12 alkyl, or aryl which is optionally substituted by groups selected from straight or branched chain C1-C12 alkyl, (straight or branched chain C1-C12 alkyl)-oxy, straight or branched chain C1-C12 alkyl carboxyclic acid or mannose groups;
wherein n is 0 to 6, m is 1 to 6, p is 0 to 6, b is 0 to 2, Z is straight or branched chain C1-C12 alkyl, optionally substituted aryl by groups selected from straight or branched chain C1-C12 alkyl, (straight or branched chain C1-C12 alkyl)-oxy, straight or branched chain C1-C12 alkyl carboxyclic acid or mannose groups; optionally substituted aryl-(straight or branched chain C1-C12 alkyl) by groups selected from straight or branched chain C1-C12 alkyl, (straight or branched chain C1-C12 alkyl)-oxy, straight or branched chain C1-C12 alkyl carboxyclic acid or mannose groups;
D1 and D2 are independently hydrogen or straight or branched chain C1-C12 alkyl,
E1 is straight or branched chain C1-C12 alkyl or -(CH2)a CO2 H wherein a is 1 to 18. and
E2 is straight or branched chain C1-C12 alkyl, and the pharmaceutically acceptable salts. esters. and amides and prodrugs thereof.

2. The compound of claim 1 wherein X is --CH2 CO2 H; R1, R2 and R3 are hydrogen; R4 is --OH; R5 is --SCH2 CO2 H; n and p are 1; b is 0; and the mannopyranoside moiety is attached to the R3 containing phenyl ring in the ortho position.

3. The compound of claim 1 wherein X is --CH2 CO2 H; R1, R2 and R3 are hydrogen; R4 is --OH; R5 is --N3 ; n and p are 1; b is 0; and the mannopyranoside moiety is attached to the R3 containing phenyl ring in the ortho position.

4. The compound of claim 1 wherein X is --CH2 CO2 H; R1, R2 and R3 are hydrogen, R4 and R5 are --OH; n, p and b are 1; and the mannopyranoside moiety is attached to the R3 containing phenyl ring in the meta position.

5. The compound of claim 1 wherein each alkyl substituent is C1-C6 alkyl.

6. The compound of claim 1 wherein X is -CH2 CO2 HCH2CO2H; R1, R2 and R3 are hydrogen, R4 and R5 are -OH.

7. A compound having the formula: 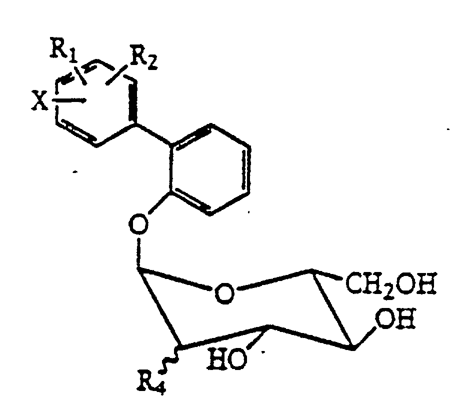 wherein X is --Q, (CH2)n Q, --O(CH2)n Q, -(CH2)nO(CH2)mQ--NH(CH2)m Q; --CONH(CH2)n Q, --(CH2)n O(CH2)m Q, --O(CH2)n O(CH2)m Q, or -CONH(CHR6)Q;
R1 and R2 are independently selected from the group consisting of hydrogen and -(CH2)nQ;
R4 is hydroxyl or hydrogen;
R6 is selected from the group consisting of hydrogen, straight or branched chain C1-C12 alkyl, optionally substituted aryl-(straight or branched chain C1-C12 alkyl) by groups selected from straight or branched chain C1-C12 alkyl, (straight or branched chain C1-C12 alkyl)-oxy, straight or branched chain C1-C12 alkyl carboxyclic acid or mannose groups; hydroxy-(straight or branched chain C1-C12 alkyl), RₓR_{y}N- (straight or branched chain C1-C12 alkyl), (straight or branched chain C1-C12 alkyl) carboxylate and RₓR_{y}NOC- (straight or branched chain C1-C12 alkyl) , Rₓ and R_{y} are independly selected from the group consisting of hydrogen, straight or branched chain C1-C12 alkyl, or aryl which is optionally substituted by groups selected from straight or branched chain C1-C12 alkyl, (straight or branched chain C1-C12 alkyl)-oxy, straight or branched chain C1-C12 alkyl carboxyclic acid or mannose groups;
Q is --CO2 H, n is 0 to 6, and m is 1 to 6, and the pharmaceutically acceptable salts, esters, and amides.

8. The compound of claim 7 wherein X is --CH2 CO2 H and R4 is --OH.

9. The compound of claim 7 wherein X is --CO2 H and R4 is --OH.

10. The compound of claim 7 wherein X is --CH2 OCH2 CO2 H and R4 is --OH.

11. The compound of claim 7 wherein X is --CONHCH2 CO2 H and R4 is --OH.

12. The compound of claim 7 wherein X is --OCH2 CO2 H and R4 is --OH.

13. The compound of claim 7 wherein X is --CONHCH(CO2 H)(CH2 C6 H5) and R4 is --OH.

14. The compound of claim 7 wherein X is --CONHCH(CO2 H)(CH2 CH2 CO2 H) and R4 is --OH.

15. The compound of claim 7 wherein X is --CO2 Li and R4 is --OH.

16. The compound of claim 7 wherein X is --(CH2)2 SO3 H and R4 is --OH.

17. The compound of claim 7 wherein X is --CONHCH2 CH2 CO2 H and R4 is --OH.

18. The compound of formula IV: 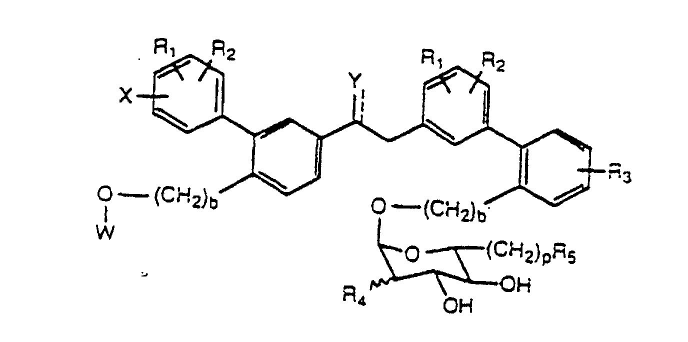 wherein X is selected from the group consisting of -(CH2)nCH3, -CN,--(CH2)n CO2 H, --O(CH2)m CO2 H, -(CH2)nCOZ, (CH2)nZ, -CHCO2H(CH2)mCO2H, --(CH2)n O(CH2)m CO2 H, --CONH(CH2)m CO2H, --CH(OZ)(CO2H), --CH(Z)(CO2H), --(CH2)n SO3H, --(CH2)n PO3 D1 D2, --NH(CH2)m CO2 H, --CONH(CHR6)CO2 H, (1-H-tetrazolyl-5-straight or branched chain C1-C12 alkyl-), and -OH;
wherein W is hydrogen, straight or branched chain C1-C12 alkyl or alpha-D-mannosyl and Y is selected from H/H, oxygen, H/hydrox H/NH2, H/NHE1, H/NE1E2, NH, NE1, oxime and O-. straight or branched chain C1-C12 alkyl oxime, and the pharmaceutically acceptable salts, esters, and amides and prodrugs thereof,
wherein n is 0 to 6, m is 1 to 6, p is 0 to 6, b is 0 to 2,
Z is straight or branched chain C1-C12 alkyl, optionally substituted aryl by groups selected from straight or branched chain C1-C12 alkyl, (straight or branched chain C1-C12 alkyl)-oxy, straight or branched chain C1-C12 alkyl carboxyclic acid or mannose groups; optionally substituted aryl-(straight or branched chain C1-C12 alkyl) by groups selected from straight or branched chain C1-C12 alkyl, (straight or branched chain C1-C12 alkyl)-oxy, straight or branched chain C1-C12 alkyl carboxyclic acid or mannose groups;
D1 and D2 are independently hydrogen or straight or branched chain C1-C12 alkyl,
E1 is straight or branched chain C1-C12 alkyl or --(CH2)8 CO2 H, and
E2 is straight or branched chain C1-C12 alkyl, and the pharmaceutically acceptable salts, esters, and amides thereof,
each R1 and R2 are independently selected from the group consisting of hydrogen, straight or branched chain C1-C12 alkyl, halogen, --OZ, --NO2, --(CH2)n CO2 H, --NH2, -NHZ;
R3 is selected from the group consisting of hydrogen, halogen, straight or branched chain C1-C12 alkyl--OZ and -NHZ;
R4 is selected from the group consisting of hydrogen, halogen, straight or branched chain C1-C12 alkyl, hydroxyl, hydroxyl-O-sulfate and -OZ;
R5 is selected from the group consisting of hydroxyl, --CN, --N3, --NH2, --NHNH2, --NE1 E2, --NHE1, --NHCO(CH2)n CO2 H, --S(CH2)m CO2 H and --NHCHNHNH2;
R6 is selected from the group consisting of hydrogen, straight or branched chain C1-C12 alkyl, optionally substituted aryl-(straight or branched chain C1-C12 alkyl) by groups selected from straight or branched chain C1-C12 alkyl, (straight or branched chain C1-C12 alkyl)-oxy, straight or branched chain C1-C12 alkyl carboxyclic acid or mannose groups; hydroxy-(straight or branched chain C1-C12 alkyl), Rₓ R_{y} N-(straight or branched chain C1-C12 alkyl), (straight or branched chain C1-C12 alkyl) carboxylic acid and RₓR_{y}NOC-(straight or branched chain C1-C12 alkyl)
Rₓ and R_{y} are independently selected from the group consisting of hydrogen, straight or branched chain C1-C12 alkyl, or aryl which is optionally substituted by groups selected from straight or branched chain C1-C12 alkyl, (straight or branched chain C1-C12 alkyl)-oxy, straight or branched chain C1-C12 alkyl carboxyclic acid or mannose groups;

19. The compound of claim 18 wherein X is --CH2 CO2 H; R1, R2 and R3 are hydrogen, R4 and R5 are --OH; each b is 0; and p is 1.

20. The compound of claim 19 wherein W is alpha-D-mannosyl and Y is H/H.

21. The compound of claim 19 wherein W is methyl and Y is oxygen.

22. Use of at least one compound according to claim I for manufacturing a medicament for treating or preventing diseases **characterized by** the binding of E-, P-selectin and/or L-selectin to sialyl-Lewis x or sialyl-Lewis a presented on a cell surface through the inhibition of such binding.

23. Use of at least one compound according to claim 7 for manufacturing a medicament for treating or preventing diseases **characterized by** the binding of E-, P-selectin and/or L-selectin to sialyl-Lewis x or sialyl-Lewis a presented on a cell surface through the inhibition of such binding.

24. Use of at least one compound according to claim 18 for manufacturing a medicament for treating or preventing diseases **characterized by** the binding of E-, P-selectin and/or L-selectin to sialyl-Lewis x or sialyl-Lewis a presented on a cell surface through the inhibition of such binding.

25. Use according to claim 22 for manufacturing a medicament for treating any of the diseases selected from the group consisting of septic shock, chronic inflammatory disease, psoriasis, rheumatoid arthritis, reperfusion injury that occurs following heart attacks, strokes and organ transplants, traumatic shock, multi-organ failure, autoimmune diseases, asthma, inflammatory bowel disease, Chron's disease, ARDA and cancer.

26. Use according to claim 23 for manufacturing a medicament for treating any of the diseases selected from the group consisting of septic shock, chronic inflammatory disease, psoriasis, rheumatoid arthritis, reperfusion injury that occurs following heart attacks, strokes and organ transplants, traumatic shock, multi-organ failure, autoimmune diseases, asthma, inflammatory bowel disease, Chron's disease, ARDA and cancer.

27. Use according to claim 24 for manufacturing a medicament for treating any of the diseases selected from the group consisting of septic shock, chronic inflammatory disease, psoriasis, rheumatoid arthritis, reperfusion injury that occurs following heart attacks, strokes and organ transplants, traumatic shock, multi-organ failure, autoimmune diseases, asthma, inflammatory bowel disease, Chron's disease, ARDA and cancer.

28. A pharmaceutically active composition comprising a compound of claim 1 and a pharmaceutically acceptable carrier.

29. A pharmaceutically active composition comprising a compound of claim 7 and a pharmaceutically acceptable carrier.

30. A pharmaceutically active composition comprising a compound of claim 18 and a pharmaceutically acceptable carrier.

## Patentansprüche

1. Eine Verbindung mit der Formel: 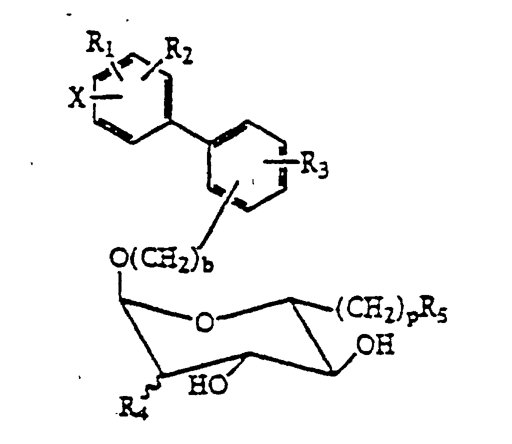 worin X gewählt ist aus der Gruppe bestehend aus -(CH₂)ₙCH₃, -CN, --(CH₂)n CO2 H, --O(CH2)m CO2 H, -(CH2)nCOZ, (CH2)nZ, -CHCO2H(CH2)mCO2H, --(CH2)n O(CH2)m CO2 H, --CONH(CH2)m CO2 H, --CH(OZ)(CO2 H), --CH(Z)(CO2 H), --(CH2)n SO3 H, --(CH2)n P03 D1 D2, --NH(CH2)m CO2 H, --CONH(CHR6)CO2 H, (1-H-Tetrazolyl-5-gerade- oder verzweitkettiges C1-C12 Alkyl-); und --OH;
R1 und R2 sind unabhängig gewählt aus der Gruppe bestehend aus Wasserstoff, gerade- oder verzweigtkettigem C1-C12 Alkyl, Halogen, --(CH2)n CO2 H, --OZ, --NO2, --NH2, -NHZ;
R3 ist gewählt aus der Gruppe bestehend aus Wasserstoff, Halogen, gerade- oder verzweigtkettigem C1-C12 Alkyl--OZ und --NHZ;
R4 ist gewählt aus der Gruppe bestehend aus Wasserstoff, Halogen, gerade- oder verzweigtkettigem C1-C12 Alkyl, Hydroxyl, Hydroxyl-O-sulfat und --OZ;
R5 ist gewählt aus der Gruppe bestehend aus Hydroxyl, --CN, --N3, --NH2, --NHNH2, --NE1 E2, --NHE1, --NHCO(CH2)n CO2 H, --S(CH2)m CO2 H und --NHCHNHNH2; und
R6 ist gewählt aus der Gruppe bestehend aus Wasserstoff, geradeoder verzweigtkettigem C1-C12 Alkyl, wahlweise substituiertem Aryl-(gerade- oder verzweigtkettiges C1-C12 Alkyl) durch Gruppen gewählt aus gerade- oder verzweigtkettigem C1-C12 Alkyl, (gerade- oder verzweigtkettigem C1-C12 Alkyl)-oxy, gerade- oder verzweigtkettigem C1-C12 Alkylcarbonsäure oder Mannosegruppen; Hydroxy-(gerade- oder verzweigtkettigem C1-C12 Alkyl), RxRyN-(gerade- oder verzweigtkettigem C1-C12 Alkyl), geradeoder verzweigtkettiger C1-C12 Alkylcarbonsäure und RxRyNOCgerade- oder verzweigtkettigem C1-C12 Alkyl),
Rx und Ry sind unabhängig gewählt aus der Gruppe bestehend aus Wasserstoff, gerade- oder verzweigtkettigem C1-C12 Alkyl, oder Aryl, das wahlweise substituiert ist durch Gruppen gewählt aus gerade- oder verzweigtkettigem C1-C12 Alkyl, (gerade- oder verzweigtkettigem C1-C12 Alkyl)-oxy, gerade- oder verzweigtkettiger C1-C12 Alkylcarbonsäure oder Mannosegruppen;
worin n 0 bis 6 ist, m ist 1 bis 6, p ist 0 bis 6, b ist 0 bis 2;
Z ist gerade- oder verzweigtkettiges C1-C12 Alkyl, wahlweise substituiertes Aryl durch Gruppen gewählt aus gerade- oder verzweigtkettigem C1-C12 Alkyl, (gerade- oder verzweigtkettigem C1-C12 Alkyl)-oxy, gerade- oder verzweigtkettigter Alkylcarbonsäure oder Mannosegruppen; wahlweise substituiertes Aryl-(gerade- oder verzweigtkettiges C1-C12 Alkyl) durch Gruppen gewählt aus gerade- oder verzweigtkettigem C1-C12 Alkyl, (gerade- oder verzweigtkettigem C1-C12 Alkyl)-oxy, gerade- oder verzweigtkettiger C1-C12 Alkylcarbonsäure oder Mannosegruppen;
D1 und D2 sind unabhängig Wasserstoff oder gerade- oder verzweigtkettiges C1-C12 Alkyl.
E1 ist gerade- oder verzweigtkettiges C1-C12 Alkyl oder --(CH2)a CO2 H worin a 1 bis 18 ist, und
E2 ist gerade- oder verzweigtkettiges C1-C12 Alkyl, und die pharmazeutisch verträglichen Salze, Ester und Amide und Prodrugs davon.

2. Die Verbindung von Anspruch 1, worin X --CH2 CO2 H ist, R1, R2 und R3 sind Wasserstoff; R4 ist --OH; R5 ist --SCH2 CO2 H; n und p sind 1; b ist 0; und der Mannopyranosidteil ist gebunden an den R3 enthaltenden Phenylring in der Orthoposition.

3. Die Verbindung von Anspruch 1, worin X --CH2 CO2 H ist; R1, R2 und R3 sind Wasserstoff, R4 ist --OH; R5 ist --NR; n und p sind 1; b ist 0; und der Mannopyranosidteil ist gebunden an den R3 enthaltenden Phenylring in der Orthoposition.

4. Die Verbindung von Anspruch 1, worin X --CH2 CO2 H ist, R1, R2 und R3 sind Wasserstoff, R4 und R5 sind --OH; n, p und b sind 1; und der Mannopyranosidteil ist gebunden an dem R3 enthaltenden Phenylring in der Metaposition.

5. Die Verbindung von Anspruch 1, worin jeder Alkylsubstituent C1-C6 Alkyl ist.

6. Die Verbindung von Anspruch 1, worin X --CH2 CO2 HCH2CO2H ist; R1, R2 und R3 sind Wasserstoff, R4 und R5 sind -OH.

7. Eine Verbindung mit der Formel: 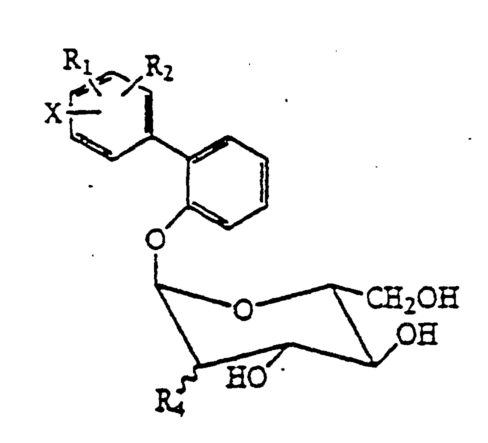 worin X --Q, (CH2)n Q, --O(CH2)n Q, -(CH2)nO(CH2)mQ--NH(CH2)m Q; --CONH(CH2)n Q, --(CH2)n O(CH2)m Q, --O(CH2)n O(CH2)m Q oder -CONH(CHR6)Q ist;
R1 und R2 sind unabhängig gewählt aus der Gruppe bestehend aus Wasserstoff und -(CH2)nQ;
R4 ist Hydroxyl oder Wasserstoff;
R6 ist gewählt aus der Gruppe bestehend aus Wasserstoff, geradeoder verzweigtkettigem C1-C12 Alkyl, wahlweise substituiertem Aryl-(gerade- oder verzweigtkettigtem C1-C12 Alkyl) durch Gruppen gewählt aus gerade- oder verzweigtkettigtem C1-C12 Alkyl, (gerade- oder verzweigtkettigem C1-C12 Alkyl, (geradeoder verzweigtkettigem C1-C12 Alkyl)-oxy, gerade- oder verzweigtkettiger C1-C12 Alkylcarbonsäure oder Mannosegruppen; Hydroxy-(gerade- oder verzweigtkettiges C1-C12 Alkyl), RxRyN (gerade- oder verzweigtkettiges C1-C12 Alkyl), (gerade- oder verzweigtkettiges C1-C12 Alkyl) carboxylat und RxRyNOC-(geradeoder verzweigtkettigem C1-C12 Alkyl), Rx und Ry sind unabhängig gewählt aus der Gruppe bestehend aus Wasserstoff, gerade- oder verzweigtkettigem C1-C12 Alkyl, oder Aryl, das wahlweise substituiert ist durch Gruppen gewählt aus gerade- oder verzweigtkettigem C1-C12 Alkyl, (gerade- oder verzweigtkettigem C1-C12 Alkyl)-oxy, gerade- oder verzweigtkettiger C1-C12 Alkylcarbonsäure oder Mannosegruppen; Q ist --CO2 H, n ist 0 bis 6 und m ist 1 bis 6 und die pharmazeutisch verträglichen Salze, Ester und Amide.

8. Die Verbindung von Anspruch 7, worin X --CH2 CO2 H ist und R4 ist --OH.

9. Die Verbindung von Anspruch 7, worin X --CO2 H ist und R4 ist --OH.

10. Die Verbindung von Anspruch 7, worin X --CH2 OCH2 CO2 H ist und R4 ist --OH.

11. Die Verbindung von Anspruch 7, worin X --CONHCH2 CO2 H ist und R4 ist --OH.

12. Die Verbindung von Anspruch 7, worin X --OCH2 CO2 H ist und R4 ist --OH.

13. Die Verbindung von Anspruch 7, worin X --CONHCH(CO2 H)(CH2 C6 H5) ist und R4 ist --OH.

14. Die Verbindung von Anspruch 7, worin X --CONHCH(CO2 H)(CH2 CH2 CO2 H) ist und R4 ist --OH.

15. Die Verbindung von Anspruch 7, worin X --CO2 Li ist und R4 ist --OH.

16. Die Verbindung von Anspruch 7, worin X --(CH2)2 SO3 H ist und R4 ist --OH.

17. Die Verbindung von Anspruch 7, worin X --CONHCH2 CH2 CO2 H ist und R4 ist --OH.

18. Die Verbindung von Formel IV: 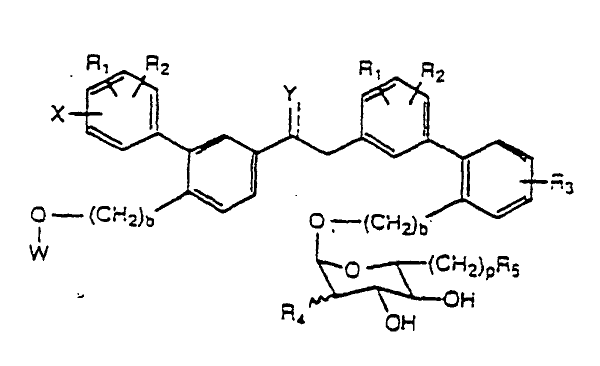 worin X gewählt ist aus der Gruppe bestehend aus -(CH2)nCH3, -CN,--(CH2)n CO2, --O(CH2)m CO2 H, -(CH2)nCOZ, (CH2)nZ, -CHCO2H(CH2)mCO2H, --(CH2)n O(CR2)m CO H, --CONH(CH2)m CO2H, --CH(OZ)(CO2H), --CH(Z)(CO2H), --(CH2)n SO3H, --(CH2)n P03 D1 D2, --NH(CH2)m CO2 H, --CONH(CHR6)CO2 H, (1-H-Tetrazolyl-5-gerade- oder verzweigtkettiges C1-C12 Alkyl-), und -OH; worin W Wasserstoff ist, gerade- oder verzweigtkettiges C1-C12 Alkyl oder alpha-D-Mannosyl und Y ist gewählt aus H/H, Sauerstoff, H/Hydroxyl, H/NH2, H/NHE1, H/NE1E2, NH, NE1, Oxim und O-gerade- oder verzweigtkettiges C1-C12 Alkyloxim, und die pharmazeutisch verträglichen Salze, Ester und Amide und Prodrugs davon,
worin n 0 bis 6 ist, m ist 1 bis 6, p ist 0 bis 6, b ist 0 bis 2,
Z ist gerade- oder verzweigtkettiges C1-C12 Alkyl, wahlweise substituiertes Aryl durch Gruppen gewählt aus gerade- oder verzweigtkettigem C1-C12 Alkyl, (gerade- oder verzweigtkettiges C1-C12 Alkyl)-oxy, gerade- oder verzweigtkettige C1-C12 Alkylcarbonsäure oder Mannosegruppen; wahlweise substituiertes Aryl-(gerade- oder verzweigtkettiges C1-C12 Alkyl) durch Gruppen gewählt aus gerade- oder verzweigtkettigem C1-C12 Alkyl, (gerade- oder verzweigtkettigem C1-C12 Alkyl)-oxy, gerade- oder verzweigtkettiger C1-C12 Alkylcarbonsäure oder Mannosegruppen;
D1 und D2 sind unabhängig Wasserstoff oder gerade- oder verzweigtkettiges C1-C12 Alkyl,
E1 ist gerade- oder verzweigtkettiges C1-C12 Alkyl oder --(CH2)8 CO2 H, und
E2 ist gerade- oder verzweigtkettiges C1-C12 Alkyl, und die pharmazeutisch verträglichen Salze, Ester und Amide davon, jedes R1 und R2 ist unabhängig gewählt aus der Gruppe bestehend aus Wasserstoff, gerade- oder verzweigtkettigtem C1-C12 Alkyl, Wasserstoff, --OZ, --NO2, --(CH2)n CO2 H, --NH2, -NHZ;
R3 ist gewählt aus der Gruppe bestehend aus Wasserstoff, Halogen, gerade- oder verzweigtkettigem C1-C12 Alkyl--OZ und -NHZ;
R4 ist gewählt aus der Gruppe bestehend aus Wasserstoff, Halogen, gerade- oder verzweigtkettigem C1-C12 Alkyl, Hydroxyl, Hydroxyl-O-sulfat und -OZ;
R5 ist gewählt aus der Gruppe bestehend aus Hydroxyl, --CN, --N3, --NH2, --NHNH2, --NE1 E2, --NHE1, --NHCO(CH2)n CO2 H, --S(CH2)m CO2 H und --NHCHNHNH2;
R6 ist gewählt aus der Gruppe bestehend aus Wasserstoff, geradeoder verzweigtkettigem C1-C12 Alkyl, wahlweise substituiertem Aryl-(gerade- oder verzweigtkettigem C1-C12 Alkyl) durch Gruppen gewählt aus gerade- oder verzeigtkettigem C1-C12 Alkyl, (geradeoder verzweigtkettigtem C1-C12 Alkyl)-oxy, gerade- oder verzweigkettigter C1-C12 Alkylcarbonsäure und Mannosegruppen;
Hydroxy-(gerade- oder verzweigtkettigtem C1-C12 Alkyl), RxRyN-(gerade- oder verzweigtkettigtem C1-C12 Alkyl), (geradeoder verzweigtkettigter C1-C12 Alkyl)carbonsäure und RxRyNOCgerade- oder verzweigtkettigtem C1-C12 Alkyl);
Rx und Ry sind unabhängig gewählt aus der Gruppe bestehend aus Wasserstoff, gerade- oder verzweigtkettigem C1-C12 Alkyl oder Aryl, das wahlweise substituiert ist durch Gruppen gewählt aus gerade- oder verzweigtkettigem C1-C12- Alkyl), (gerade- oder verzweigtkettigem C1-C12 Alkyl)-oxy, gerade- oder verzweigtkettiger C1-C12 Alkylcarbonsäure oder Mannosegruppen;

19. Die Verbindung von Anspruch 18, worin X --CH2 CO2 H ist; R1, R2 und R3 sind Wasserstoff, R4 und R5 sind --OH, jedes b ist 0; und p ist 1.

20. Die Verbindung von Anspruch 19, worin W alpha-D-Mannosyl ist und Y ist H/H.

21. Die Verbindung von Anspruch 19, worin W Methyl ist und Y ist Sauerstoff.

22. Verwendung von mindestens einer Verbindung gemäß Anspruch 1 zur Herstellung eines Medikaments zum Behandeln oder Verhindern von Krankheiten, **gekennzeichnet durch** die Bindung von E-, P-Selectin und/oder L-Selectin an Sialyl-Lewis x oder Sialyl-Lewis a, die auf einer Zelloberfläche präsentiert werden, **durch** die Inhibition einer solchen Bindung.

23. Verwendung von mindestens einer Verbindung gemäß Anspruch 7 zur Herstellung eines Medikaments zum Behandeln oder Verhindern von Krankheiten, **gekennzeichnet durch** die Bindung von E-, P-Selectin und/oder L-Selectin an Sialyl-Lewis x oder-Sialyl-Lewis a, die auf einer Zelloberfläche präsentiert werden, **durch** die Inhibition einer solchen Bindung.

24. Verwendung von mindestens einer Verbindung gemäß Anspruch 18 zur Herstellung eines Medikaments zum Behandeln oder Verhindern von Krankheiten, **gekennzeichnet durch** die Bindung von E-, P-Selectin und/oder L-Selectin an Sialyl-Lewis x oder Sialyl-Lewis a, die auf einer Zelloberfläche präsentiert werden, **durch** die Inhibition einer solchen Bindung.

25. Verwendung gemäß Anspruch 22 zur Herstellung eines Medikaments zur Behandlung irgendeiner der Krankheiten, die gewählt sind aus der Gruppe bestehend aus septischem Schock, chronischer Entzündungserkrankung, Schuppenflechte, Rheumatoider Arthritis, Reperfusionsverletzung, die als Folge von Herzattacken vorkommt, Stroke und Organtransplantationen, traumatischem Schock, multi-Organstörung, Autoimmunkrankheiten, Asthma, entzündlicher Darmerkrankung, Morbus Crohn, ARDA und Krebs.

26. Verwendung gemäß Anspruch 23 zur Herstellung eines Medikaments zur Behandlung irgendeiner der Krankheiten gewählt aus der Gruppe bestehend aus septischem Schock, chronischer Entzündungserkrankung, Schuppenflechte, Rheumatoider Arthritis, Reperfusionsverletzung, die als Folge von Herzattacken vorkommt, Stroke und Organtransplantationen, traumatischem Schock, multi-Organstörung, Autoimmunkrankheiten, Asthma, entzündlicher Darmerkrankung, Morbus Crohn, ARDA und Krebs.

27. Verwendung gemäß Anspruch 24 zur Herstellung eines Medikaments zur Behandlung irgendeiner der Krankheiten gewählt aus der Gruppe bestehend aus septischem Schock, chronischer Entzündungserkrankung, Schuppenflechte, Rheumatoider Arthritis, Reperfusionsverletzung, die als Folge von Herzattacken vorkommt, Stroke und Organtransplantationen, traumatischem Schock, multi-Organstörung, Autoimmunkrankheiten, Asthma, entzündlicher Darmerkrankung, Morbus Crohn, ARDA und Krebs.

28. Eine pharmazeutisch wirksame Zusammensetzung, die eine Verbindung von Anspruch 1 und einen pharmazeutisch verträglichen Träger umfaßt.

29. Eine pharmazeutisch wirksame Zusammensetzung, die eine Verbindung von Anspruch 7 und einen pharmazeutisch verträglichen Träger umfaßt.

30. Eine pharmazeutisch wirksame Zusammensetzung, die eine Verbindung von Anspruch 18 und einen pharmazeutisch verträglichen Träger umfaßt.

## Revendications

1. Composé ayant la formule 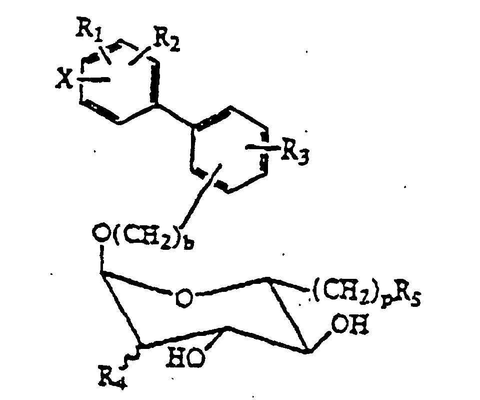 dans laquelle X est choisi dans l'ensemble comprenant -(CH)ₙCH₃, -CN, -(CH₂)ₙCO₂H, -O(CH₂)ₘCO₂H, -(CH₂)ₙCOZ, (CH₂)ₙZ, -CHCO₂H(CH₂)ₘCO₂H, -(CH₂)ₙO(CH₂)ₘCO₂H, -CONH(CH₂)ₘCO₂H, -CH(OZ)(CO₂H), -CH(Z)(CO₂H), -(CH₂)ₙSO₃H, -(CH₂)ₙPO₃D₁D₂, -NH(CH₂)ₘCO₂H, -CONH(CHR₆)CO₂H, (1-H-tétrazolyl-5-(groupe alkyle en C₁ à C₁₂ à chaîne droite ou ramifiée), et -OH ;
R₁ et R₂ sont chacun indépendamment de l'autre choisis dans l'ensemble comprenant l'hydrogène, les groupes alkyle en C₁ à C₁₂ à chaîne droite ou ramifiée, les groupes halogéno, -(CH₂)ₙCO₂H, -OZ, -NO₂, -NH₂, -NHZ ;
R₃ est choisi dans l'ensemble comprenant l'hydrogène, les groupes hatogéno, les groupes (alkyle en C₁ à C₁₂ à chaîne droite ou ramifée)-OZ, et -NHZ ;
R₄ est choisi dans l'ensemble comprenant l'hydrogène, les groupes halogéno, alkyle en C₁ à C₁₂ à chaîne droite ou ramifiée, hydroxyle, hydroxyl-O-sulfate et -OZ ;
R₅ est choisi dans l'ensemble comprenant les groupes hydroxyle, -CN, -N₃, -NH₂, -NHNH₂, -NE₁E₂, -NHE₁ -NHCO(CH₂)ₙCO₂H,S(CH₂)ₘCO₂H et -NHCHNHNH₂ ; et
R₆ est choisi dans l'ensemble comprenant l'hydrogène, les groupes alkyle en C₁ à C₁₂ à chaîne droite ou ramifiée, les groupes aryl(alkyle en C₁ à C₁₂ à chaîne droite ou ramifiée) éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique, ou mannose ; hydroxy(alkyle en C₁ à C₁₂ à chaîne droite ou ramifiée), RₓR_{y}N-(alkyle en C₁ à C₁₂ à chaîne droite ou ramifiée), acide(alkyle en C₁ à C₁₂ à chaîne droite ou ramifiée)carboxylique et RₓR_{y}NOC-(alkyle en C₁ à C₁₂ à chaîne droite ou ramifiée),
Rₓ et R_{Y} sont choisis indépendamment l'un de l'autre dans l'ensemble comprenant l'hydrogène, les groupes alkyle en C₁ à C₁₂ à chaîne droite ou ramifiée ou aryle, qui sont éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique ou mannose ;
où n vaut de 0 à 6, m vaut de 1 à 6, p vaut de 0 à 6, b vaut de 0 à 2,
Z est un groupe alkyle en C₁ à C₁₂ à chaîne droite ou ramifiée, aryle éventuellement substitué par des groupes choisis parmi les groupes alkyle en C₁ à C₁₂ à chaîne droite ou ramifiée (alkyle en C₁ à C₁₂ à chaîne droite ou ramifiée)oxy, acide (alkyle en C₁ à C₁₂ à chaîne droite ou ramifiée)carboxylique ou mannose, aryl(alkyle en C₁ à C₁₂ à chaîne droite ou ramifiée) éventuellement substitué par des groupes choisis parmi les groupes alkyle en C₁ à C₁₂ à chaîne droite ou ramifiée, (alkyle en C₁ à C₁₂ à chaîne droite ou ramifiée)oxy, acide (alkyle en C₁ à C₁₂ à chaîne droite ou ramifiée)carboxylique ou mannose ;
D₁ et D₂ sont chacun indépendamment de l'autre un atome d'hydrogène ou un groupe alkyle en C₁ à C₁₂ à chaîne droite ou ramifiée,
E₁ est un groupe alkyle en C₁ à C₁₂ à chaîne droite ou ramifiée, ou -(CH₂)ₐCO₂H, où a vaut de 1 à 18, et
E₂ est un groupe alkyle en C₁ à C₁₂ à chaîne droite ou ramifiée,
et ses sels, esters, amides et prodrogues acceptables d'un point de vue pharmaceutique.

2. Composé selon la revendication 1, dans lequel X est -CH₂CO₂H ; R₁, R₂ et R₃ sont des atomes d'hydrogène ; R₄ est -OH ; R₅ est -SCH₂CO₂H ; n et p valent 1 ; b vaut 0 ; et le fragment mannopyrannoside est fixé en position ortho au noyau phényle contenant R₃.

3. Composé selon la revendication 1, dans lequel X est -CH₂CO₂H ; R₁, R₂ et R₃ sont des atomes d'hydrogène ; R₄ est -OH ; R₅ est -N₃ ; n et p valent 1 ; b vaut 0 ; et le fragment mannopyrannoside est fixé en position ortho au noyau phényle contenant R₃.

4. Composé selon la revendication 1, dans lequel X est -CH₂CO₂H ; R₁, R₂ et R₃ sont des atomes d'hydrogène ; R₄ et R₅ sont -OH ; n, p et b valent 1 ; et le fragment mannopyrannoside est fixé en position méta au noyau phényle contenant R₃.

5. Composé selon la revendication 1, dans lequel chaque substituant alkyle est un groupe alkyle en C₁ à C₆.

6. Composé selon la revendication 1, dans lequel X est -CH₂CO₂HCH₂-CO₂H ; R₁, R₂ et R₃ sont des atomes d'hydrogène, R₄ et R₅ sont -OH.

7. Composé ayant la formule : 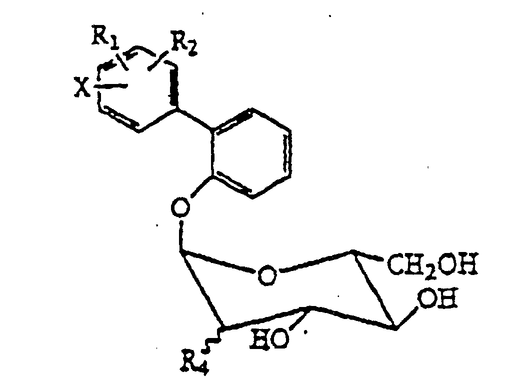 dans laquelle X est -Q, (CH₂)ₙQ, -O(CH₂)ₙQ, -(CH₂)ₙO(CH₂)ₘQ, -NH(CH₂)ₘQ, -CONH(CH₂)ₙQ, -(CH₂)ₙO(CH₂)ₘQ, -O(CH₂)ₙO(CH₂)ₘQ ou -CONH(CHR₆)Q ;
R₁ et R₂ sont chacun indépendamment de l'autre choisis dans l'ensemble comprenant l'atome d'hydrogène et -(CH₂)ₙQ ;
R₄ est un groupe hydroxyle ou un atome d'hydrogène ;
R₆ est choisi dans l'ensemble comprenant l'hydrogène, les groupes alkyle en C₁ à C₁₂ à chaîne droite ou ramifiée, les groupes aryl(alkyle en C₁ à C₁₂ à chaîne droite ou ramifiée) éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique, ou mannose ; hydroxy(alkyle en C₁ à C₁₂ à chaîne droite ou ramifiée), RₓR_{y}N-(alkyle en C₁ à C₁₂ à chaîne droite ou ramifiée), acide(alkyle en C₁ à C₁₂ à chaîne droite ou ramifiée)carboxylique et RₓR_{y}NOC-(alkyle en C₁ à C₁₂ à chaîne droite ou ramifiée),
R_{X} et R_{Y} sont choisis indépendamment l'un de l'autre dans l'ensemble comprenant l'hydrogène, les groupes alkyle en C₁ à C₁₂ à chaîne droite ou ramifiée ou aryle, qui sont éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique ou mannose ;
Q est -CO₂H, n vaut de 0 à 6 et m vaut de 1 à 6, et ses sels, esters et amides acceptables d'un point de vue pharmaceutique.

8. Composé selon la revendication 7, dans lequel X est -CH₂CO₂H et R₄ est -OH.

9. Composé selon la revendication 7, dans lequel X est -CO₂H et R₄ est -OH.

10. Composé selon la revendication 7, dans lequel X est -CH₂OCH₂CO₂H et R₄ est -OH.

11. Composé selon la revendicarian 7, dans lequel X est -CONHCH₂-CO₂H et R₄ est -OH.

12. Composé selon la revendication 7, dans lequel X est -OCH₂CO₂H et R₄ est -OH.

13. Composé selon la revendication 7, dans lequel X est -CONHCH-(CO₂H)(CH₂C₆H₅) et R₄ est -OH.

14. Composé selon la revendication 7, dans lequel X est -CONHCH-(CO₂H)(CH₂CH₂CO₂H) et R₄ est -OH.

15. Composé selon la revendicarion 7, dans lequel X est -CO₂Li et R₄ est -OH.

16. Composé selon la revendication 7, dans lequel X est -(CH₂)₂SO₃H et R₄ est -OH.

17. Composé selon la revendication 7, dans lequel X est -CONHCH₂-CH₂CO₂H et R₄ est -OH.

18. Composé de formule IV: 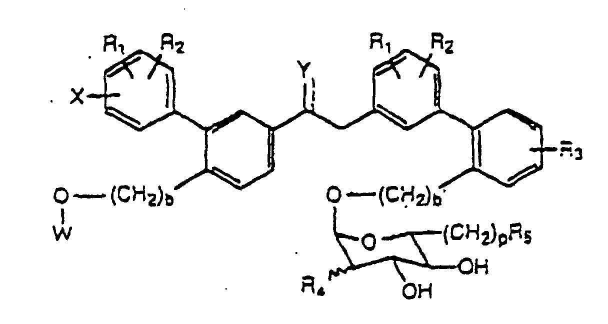 dans laquelle X est choisi dans l'ensemble comprenant -(CH)ₙCH₃, -CN, -(CH₂)ₙCO₂H, -O(CH₂)ₘCO₂H, -(CH₂)ₙCOZ, (CH₂)ₙZ, -CHCO₂H(CH₂)ₘCO₂H, -(CH₂)ₙO(CH₂)ₘCO₂H, -CONH(CH₂)ₘCO₂H, -CH(OZ)(CO₂H), -CH(Z)(CO₂H), -(CH₂)ₙSO₃H, -(CH₂)ₙPO₃D₁D₂, -NH(CH₂)ₘCO₂H, -CONH(CHR₆)CO₂H, (1-H-tétrazolyl-5-(groupe alkyle en C₁ à C₁₂ à chaîne droite ou ramifiée), et -OH ;
où W est un atome d'hydrogène, un groupe alkyle en C₁ à C₁₂ à chaîne droite ou ramifiée ou alpha-D-mannosyle, et Y est choisi parmi H/H, l'oxygène, H/hydroxyle, NlNH₂, H/NHE₁, H/NE₁E₂, NH, NE₁, les oximes et les O-(alkyle en C₁ à C₁₂ à chaîne droite ou ramifiée)oximes, et ses sels, esters et amides et prodrogues acceptables d'un point de vue pharmaceutique,
où n vaut de 0 à 6, m vaut de 1 à 6, p vaut de 0 à 6, b vaut de 0 à 2,
Z est un groupe allyle en C₁ à C₁₂ à chaîne droite ou ramifiée, aryle éventuellement substitué par des groupes choisis parmi les groupes alkyle en C₁ à C₁₂ à chaîne droite ou ramifiée (alkyle en C₁ à C₁₂ à chaîne droite ou ramifiée)oxy, acide (alkyle en C₁ à C₁₂ à chaîne droite ou ramifiée)carboxylique ou mannose, aryl(alkyle en C₁ à C₁₂ à chaîne droite ou ramifiée) éventuellement substitué par des groupes choisis parmi les groupes alkyle en C₁ à C₁₂ à chaîne droite ou ramifiée, (alkyle en C₁ à C₁₂ à chaîne droite ou ramifiée)oxy, acide (alkyle en C₁ à C₁₂ à chaîne droite ou ramifiée)carboxylique ou mannose ;
D₁ et D₂ sont chacun indépendamment de l'autre un atome d'hydrogène ou un groupe alkyle en C₁ à C₁₂ à chaîne droite ou ramifiée,
E₁ est un groupe alkyle en C₁ à C₁₂ à chaîne droite ou ramifiée ou -(CH₂)₈CO₂H, et
E₂ est un groupe alkyle en C₁ à C₁₂ à chaîne droite ou ramifiée, et ses sels, esters et amides acceptables d'un point de vue pharmaceutique,
chacun des radicaux R₁ et R₂ est choisi indépendamment de l'autre dans l'ensemble comprenant l'hydrogène, les groupes alkyle en C₁ à C₁₂ à chaîne droite ou ramifiée, halogéno, -OZ, -NO₂, -(CH₂)ₙCO₂H, -NH₂, -NHZ ;
R₃ est choisi dans l'ensemble comprenant l'hydrogène, les groupes halogéno, les groupes (allyle en C₁ à C₁₂ à chaîne droite ou ramifiée)-OZ, et -NHZ ;
R₄ est choisi dans l'ensemble comprenant l'hydrogène, les groupes halogéno, alkylc en C₁ à C₁₂ à chaîne droite ou ramifiée, hydroxyle, hydroxyl-O-sulfate et -OZ ;
R₅ est choisi dans l'ensemble comprenant les groupes hydroxyle, -CN, -N₃, -NH₂, -NHNH₂, -NE₁E₂, -NHE₁, -NHCO(CH₂)ₙCO₂H, -S(CH₂)ₘCO₂H et -NHCHNHNH₂ ;
R₆ est choisi dans l'ensemble comprenant l'hydrogène, les groupes alkyle en C₁ à C₁₂ à chaîne droite ou ramifiée, les groupes aryl(alkyle en C₁ à C₁₂ à chaîne droite ou ramifiée) éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique, ou mannose ; hydroxy(alkyle en C₁ à C₁₂ à chaîne droite ou ramifiée), RₓR_{y}N-(alkyle en C₁ à C₁₂ à chaîne droite ou ramifiée), acide(alkyle en C₁ à C₁₂ à chaîne droite ou ramifiée)carboxylique et RₓR_{y}NOC-(alkyle en C₁ à C₁₂ à chaîne droite ou ramifiée),
Rₓ et R_{Y} sont choisis indépendamment l'un de l'autre dans l'ensemble comprenant l'hydrogène, les groupes alkyle en C₁ à C₁₂ à chaîne droite ou ramifiée ou aryle, qui sont éventuellement substitués par des groupes choisis parmi les groupes alkyle en C₁ à C₁₂ à chaîne droite ou ramifiée, (alkyle en C₁ à C₁₂ à chaîne droite ou ramifiée)-oxy, acide (alkyle en C₁ à C₁₂ à chaîne droite ou ramifiée)carboxylique ou mannose.

19. Composé selon la revendication 18, dans lequel X est -CH₂CO₂H ; R₁, R₂ et R₃ sont des atomes d'hydrogène, R₄ et R₅ sont -OH ; chaque b vaut 0 ; et p vaut 1.

20. Composé selon la revendication 19, dans lequel W est le groupe alpha-D-mannosyle, et Y est H/H.

21. Composé selon la revendication 19, dans Lequel W est le méthyle et Y est l'oxygène.

22. Utilisation d'au moins un composé selon la revendication 1 pour fabriquer un médicament destiné au traitement ou à la prévention de maladies **caractérisées par** la liaison de la sélectine-E, P et/ou L au sialyl-Lewis x ou au sialyl-Lewis a présenté sur une surface cellulaire, par l'intermédiaire de l'inhibition de cette liaison.

23. Utilisation d'au moins un composé selon la revendication 7 pour fabriquer un médicament destiné au traitement ou à la prévention de maladies **caractérisées par** la liaison de la sélective-E, P et/ou L au sialyl-Lewis x ou au sialyl-Lewis a présenté sur une surface cellulaire, par l'intermédiaire de l'inhibition de cette liaison.

24. Utilisation d'au moins un composé selon la revendication 18 pour fabriquer un médicament destiné au traitement ou à la prévention de maladies **caractérisées par** la liaison de la sélective-E, P et/ou L au sialyl-Lewis x ou au sialyl-Lewis a présenté sur une surface cellulaire, par l'intermédiaire de l'inhibition de cette liaison.

25. Utilisation selon la revendication 22 pour fabriquer un médicament destiné au traitement de l'une quelconque des maladies choisies dans l'ensemble comprenant le choc septique, la maladie inflammatoire chronique, le psoriasis, la polyarthrite rhumatoïde, la lésion par reperfusion qui apparaît après des attaques cardiaques, des accidents vasculaires cérébraux et des transplantations d'organes, le choc traumatique, l'insuffisance multiple, les maladies auto-immunes, l'asthme, les entérites, la maladie de Crohn, le SDRA et le cancer.

26. Utilisation selon la revendication 23 pour fabriquer un médicament destiné au traitement de l'une quelconque des maladies choisies dans l'ensemble comprenant le choc septique, la maladie inflammatoire chronique, le psoriasis, la polyarthrite rhumatoïde, la lésion par reperfusion qui apparaît après des attaques cardiaques, des accidents vasculaires cérébraux et des transplantations d'organes, le choc traumatique, l'insuffisance multiple, les maladies auto-immunes, l'asthme, les entérites, la maladie de Crohn, le SDRA et le cancer.

27. Utilisation selon la revendication 24 pour fabriquer un médicament destiné au traitement de l'une quelconque des maladies choisies dans l'ensemble comprenant le choc septique, la maladie inflammatoire chronique, le psoriasis, la polyarthrite rhumatoïde, la lésion par reperfusion qui apparaît après des attaques cardiaques, des accidents vasculaires cérébraux et des transplantations d'organes, le choc traumatique, l'insuffisance multiple, les maladies auto-immunes, l'asthme, les entérites, la maladie de Crohn, le SDRA et le cancer.

28. Composition pharmaceutiquement active comprenant un composé selon la revendication 1 et un excipient acceptable d'un point de vue pharmaceutique.

29. Composition pharmaceutiquement active comprenant un composé selon la revendication 7 et un excipient acceptable d'un point de vue pharmaceutique.

30. Composition pharmaceutiquement active comprenant un composé selon la revendication 18 et un excipient acceptable d'un point de vue pharmaceutique.
